# EUROPEAN PATENT SPECIFICATION

(11) **EP 2 310 364 B1**
(45) Date of publication and mention of the grant of the patent: **02.01.2013**
(21) Application number: 09761692.4
(22) Date of filing: 08.06.2009
(51) Int. Cl.: C07D 211/58, C07D 295/10, C07D 295/18, C07D 401/06, C07D 401/14, C07D 471/08, C07D 471/10, A61K 31/495, A61P 35/00

(54) **HETEROCYCLIC DERIVATIVES AS HDAC INHIBITORS**
HETEROCYCLISCHE DERIVATE ALS HDAC-INHIBITOREN
DÉRIVÉS HÉTÉROCYCLIQUES COMME INHIBITEURS DES HDAC

(30) Priority: 09.06.2008 EP 08157857
(43) Date of publication of application: 20.04.2011
(73) Proprietor: DAC S.r.l., 20121 Milano (MI) (IT)
(72) Inventor: THALER, Florian, I-21040 Gerenzano (IT); VARASI, Mario, I-20142 Milano (IT); GAGLIARDI, Stefania, I-20059 Vimercate (IT); COLOMBO, Andrea, I-20015 Parabiago (IT); MINUCCI, Saverio, I-20090 Noverasco Di Opera (IT); MERCURIO, Ciro, I-20025 Legnano (IT)
(74) Representative: Palladino, Saverio Massimo
(86) International application number: PCT/EP2009/057042
(87) International publication number: WO 2009/150129

(56) References cited:
- WO-A-2006/037761
- WO-A-2007/113249
- FINN P W ET AL: "Novel Sulfonamide Derivatives as Histone Deacetylase" HELVETICA CHIMICA ACTA, VERLAG HELVETICA CHIMICA ACTA. BASEL, CH, vol. 88, 1 July 2005 (2005-07-01), pages 1630-1657, XP002367316 ISSN: 0018-019X

## Description

### Field of the invention

The present invention relates to inhibitors of histone deacetylases (HDACs), to a process for their preparation, pharmaceutical compositions comprising them, and to their use as therapeutic agents, in particular for the treatment of cancer.

### Background of the invention

The reversible acetylation of the ε-amino groups of several lysine residues in the *N-*terminal histone tails mediates important conformational modifications in nucleosomes. These modifications influence the access of transcription factor to DNA and regulate gene expression (Davie, J.R. Curr. Opin. Genet. Dev. 1998, 8, 173-178). Two enzyme classes are involved in the process of acetylation and deacetylation of histones: histone acetyltransferases (HAT), which catalyse histone acetylation by acting as transcriptional co-activators, and histone deacetylases (HDAC).

After their recruitment to the promoter regions induced by transcription repressors and co-repressors such as Sin3, SMRT and N-CoR, histone deacetylases induce the formation of hypoacetylated histones and ultimately lead to transcriptional silencing (Wu, J. et al. Trends Biochem. Sci. 2000, 25, 619-623). The aberrant recruitment of histone deacetylases by oncogene proteins, or the disruption of the equilibrium between the activities of histone acetyltransferases and histone deacetylases are implicated in a series of pathologies, such as cancer, diseases of the central and peripheral nervous system, infections, immune diseases, cardiovascular diseases, muscular disorders, fibrosis or psoriasis.

The following (non exhaustive) selection of references demonstrate the involvement of HDACs in different diseases and the potential therapeutic benefit, which can be achieved by inhibiting them: Timmermann S. et al. Cell Mol Life Sci. 2001 58, 728-736; Huang, L. J. Cell. Physiol. 2006, 209, 611-616; Minucci, S. et al. Nature Reviews Cancer, 2006, 6, 38-51; Sharma, P. et al. Schizophr. Res. 2006, 88, 227-231. Glozak M.A. et al. Oncogene. 2007, 26, 5420-5432; Elaut G. et al. Curr Pharm Des. 2007, 13, 2584-2620; Balakin K.V. et al. Anticancer Agents Med Chem. 2007 7, 576-92; Lee H.B. et al. Kidney Int. Suppl. 2007, 106, S61-66; Morrison B.E. et al. Cell Mol Life Sci. 2007, 64, 2258-2269.

In recent years there has been a considerable effort to develop inhibitors of histone deacetylases and several classes of compounds have been found to have potent and specific activities in preclinical studies. Their clinical benefits, however, are limited by toxicity problems, poor pharmacokinetic properties, poor potency and lack of selectivity (Elaut G. et al. Curr Pharm Des. 2007, 13, 2584-2620; Vigushin, D. et al. Anti-Cancer Drugs 2002, 13, 1-13).

PCT application WO2006/037761 discloses HDAC inhibitors with the general formula 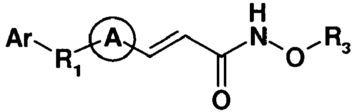 wherein **R₁** is a linear or branched chain, containing at least two conjugated double bonds, **R₃** is hydrogen or alkoxyalkyl; **Ar** is an optionally substituted aryl or heteroaryl group and **A** is a phenyl or pyridyl group, substituted by hydrogen, alkyl, cycloalkyl, aryl, arylalkyl, heterocyclyl, heterocyclylalkyl, halogen, haloalkyl, hydroxy, hydroxyalkyl, alkoxy, haloalkoxy, amino, aminoalkyl, alkylamino, (thio)carbonylamino, (thio)aminocarbonyl, sulphonylamino, aminosulphonyl, (thio)acyl, (thio)acyloxy, (thio)alkoxycarbonyl, nitro or nitryl.

PCT applications WO1993/07148 and WO1995/31977 disclose hydroxamic acid derivatives useful for selectively inducing terminal differentiation, cell growth arrest or apoptosis of neoplastic cells. The applications refer among others to structures with the general formula 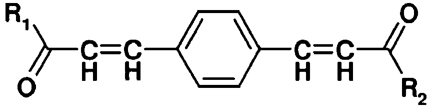 wherein each of **R₁** and **R₂** are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group; no HDAC inhibiting activity is disclosed for these compounds.

We have found now that certain substituted heterocyclic derivatives are highly potent inhibitors of the HDAC enzyme.

### Summary of the invention

According to the present invention there are provided compounds, endowed with a potent HDAC inhibitory activity, of general formula **(I)** 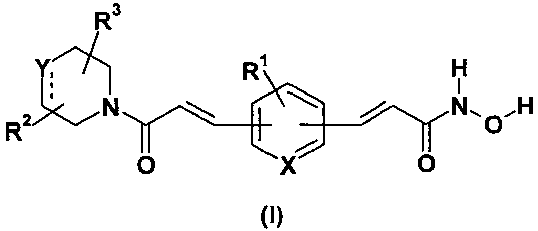 wherein:
- R¹: the dotted line is an optional additional bond; is hydrogen;
- **R², R³**: are, independently, hydrogen; C₁-C₆ alkyl; aryl, optionally substituted by halogen, C₁-C₆ alkyl, C₁-C₆ alkoxy, or C₁-C₆ haloalkyl; heteroaryl, optionally substituted by aryl, which may be optionally substituted by halogen; or taken together with the carbon atoms to which they are bound form a bridged bicyclic ring or a fused heterocycle;
- **X**: is CH or nitrogen;
- **Y**: is a bond, oxygen, (CH₂)ₘC**R⁴R⁵**(CH₂)ₙ, or **NR⁶**;
- **m, n**: are, independently, zero or 1;
- **R⁴, R⁵**: are, independently, hydrogen; CN; C₁-C₆ alkyl, optionally substituted by aryl; (CO)-aryl; aryl, optionally substituted by one or more substituents selected from C₁-C₆ alkyl; heterocyclyl or heteroaryl; or taken together with the carbon atom to which they are bound form a spirocycle; or **R⁴** taken together with the carbon atom to which it is bound and **R²** together with the carbon atom to which it is bound can form a fused heterocycle;
- **R⁶**: is hydrogen; C₁-C₆ alkyl, optionally substituted by aryl; aryl, optionally substituted by one or more substituents selected from halogen, CN, C₁-C₆ alkyl, C₁-C₆ alkoxy, C₁-C₆ haloalkyl, methanesulfonyl, or benzyloxy; heterocyclyl or heteroaryl; (CO)**R⁷**;
- **R⁷**: is hydrogen; aryl; C₁-C₆ alkyl;
and the pharmaceutically acceptable salts thereof, provided that, when the dotted line is an additional bond, then **Y** is **CR⁴R⁵,** wherein **R⁴** is as defined above and **R⁵ is** absent.

### Detailed description of the invention

According to the description and claims, "aryl" represents a mono or bicyclic aromatic ring system of, respectively, 6, 9 or 10 atoms, such as benzene, indene and naphthalene and includes also indan and tetrahydronaphthalene.

According to the description and claims, "heteroaryl" represents a mono or bicyclic heteroaromatic ring system of, respectively, 5 to 10 members, which contains one, two or three heteroatoms selected from nitrogen, oxygen and sulphur. Examples of said heteroaryls include, but are not limited to: pyrrolyl, imidazolyl, oxazole, oxadiazole, pyridyl, pyrimidinyl, pyridazinyl, furyl, thienyl, indolyl, isoindolyl, benzimidazolyl, benzoxazole, purinyl, quinolyl, isoquinolyl, quinazolinyl, quinoxalinyl, benzofuranyl, and benzopyranyl.

According to the description and claims, "heterocyclyl", or "heterocycle" represents a mono, bi- or tricyclic saturated or partially saturated non-aromatic ring system of, respectively, 4 to 16 members, which contains one, two, three or four heteroatoms selected from nitrogen, oxygen and sulphur. Examples of such heterocycles include, but are not limited to: pyrrolidinyl, piperidinyl, piperazinyl, tetrahydrofuranyl, tetrahydropyranyl, morpholinyl, thiomorpholinyl, tetrahydroquinolinyl, tetrahydroisoquinolinyl, tetrahydroquinoxalinyl, tetrahydro-1H-beta-carboline, tetrahydro-1H-pyrido[4,3-b]indolyl.

According to the description and claims, "fused heterocycle" represents a bi- or tricyclic saturated or partially saturated non-aromatic ring system of, respectively, 8 to 16 members, which contains one nitrogen atom and may optionally contain one, two, or three heteroatoms selected from nitrogen, oxygen and sulphur. Examples of such heterocycles include, but are not limited to: tetrahydroquinolinyl, tetrahydroisoquinolinyl, 4,5,6,7-tetrahydro-thiazolo[4,5-b]pyridine, 4,5,6,7-tetrahydro-thiazolo[4,5-c]pyridine, 4,5,6,7-tetrahydro-thiazolo[5,4-b]pyridine, 4,5,6,7-tetrahydro-thiazolo[5,4-c]pyridine, tetrahydro-1H-beta-carboline, tetrahydro-1H-pyrido[4,3-b]indolyl.

According to the description and claims, the term "bridged bicyclic ring" refers to a bicyclic heterocyclicaliphatic ring system in which the rings are bridged. Examples of bridged bicyclic ring systems include, but are not limited to, 2-aza-bicyclo[2.2.1]heptane or 2,5-diaza-bicyclo[2.2.1]hept-2-yl.

According to the description and claims, the term "spirocycle" represents a C₃-C₇ alkylene, a 3-7 membered heteroalkylene, a C₃-C₇ alkenylene, or a 3-7 membered heteroalkenylene group, in which both ends of the alkylene, heteralkylene, alkenylene or heteroalkenylene group are attached to the same carbon to form a bicyclic ring. The aryl may be optionally substituted with one or more substituents selected from halogen, CN, C₁-C₆ alkyl, C₁-C₆ haloalkyl, C₁-C₆ alkoxy, C₁-C₆ haloalkoxy, C₁-C₆ acylamino or -S(O)₂-C₁-C₆.

The heteroaryl may be optionally substituted with one or more substituents selected from halogen, CN, C₁-C₆ alkyl, C₁-C₆ haloalkyl, C₁-C₆ alkoxy, C₁-C₆ haloalkoxy or aryl.

The "heterocycle", "heterocyclyl" group or "fused heterocycle" may be optionally substituted with one or more substituents selected from halogen, CN, C₁-C₆ alkyl, C₁-C₆ haloalkyl, C₁-C₆ alkoxy, or C₁-C₆ haloalkoxy, aryl or oxo.

The spirocycle may be optionally substituted with one or more substituents selected from halogen, CN, C₁-C₆ alkyl, C₁-C₆ haloalkyl, C₁-C₆ alkoxy, C₁-C₆ haloalkoxy or aryl or oxo. In certain preferred embodiments, the spirocycle is fused to an aryl or heteroaryl ring.

According to the description and claims, the term "C₁-C₆ alkyl" refers to a straight or branched hydrocarbon chain radical, consisting solely of carbon and hydrogen atoms, having from one to six carbon atoms. The "C₁-C₆ alkyl" group is preferably a linear or branched C₁-C₄ alkyl group, more preferably a C₁-C₃ alkyl group.

According to the description and claims, the term "C₁-C₆ alkoxy" refers to a straight or branched O-C₁-C₆ alkyl, with alkyl as defined herein. The "C₁-C₆ alkoxy" group is preferably a linear or branched C₁-C₄ alkoxy group, more preferably a C₁-C₃ alkoxy group.

According to the description and claims, the term "C₁-C₆ haloalkyl" refers to a straight or branched hydrocarbon chain radical, which is substituted by one or more halogen atoms and having from one to six carbon atoms. The "C₁-C₆ haloalkyl" group is preferably a linear or branched C₁-C₄ haloalkyl group, more preferably a C₁-C₃ haloalkyl group, being in particular CF₃.

According to the description and claims, the term "C₁-C₆ haloalkoxy" refers to a straight or branched O-C₁-C₆ haloalkyl, where haloalkyl is defined herein. The "C₁-C₆ haloalkoxy" group is preferably a linear or branched C₁-C₄ haloalkoxy group, more preferably a C₁-C₃ haloalkoxy group, being in particular OCF₃, OCHF₂ or OCH₂F. According to the description and claims, the term "C₁-C₆ acylamino" refers to a straight or branched -NH-(CO)-C₁-C₆ alkyl, with C₁-C₆ alkyl as defined herein. According to the description and claims, the term "C₁-C₆ acyl" refers to a straight or branched -(CO)-C₁-C₆ alkyl, with C₁-C₆ alkyl as defined herein.

According to the description and claims, the term "C₃-C₇ alkylene" refers to a straight divalent hydrocarbon chain consisting solely of carbon and hydrogen atoms, having from three to seven carbon atoms.

According to the description and claims, the term "C₃-C₇ alkenylene" refers to a divalent hydrocarbon chain consisting solely of carbon and hydrogen atoms, containing at least one double bond and having from three to seven carbon atoms. According to the description and claims, the term "3-7 membered heteroalkylene" refers to a straight divalent chain, having from three to seven atoms in the chain and consisting of carbon and hydrogen atoms, wherein at least one carbon atom is replaced by nitrogen, oxygen or sulphur.

According to the description and claims, the term "3-7 membered heteroalkenylene" refers to a straight divalent chain, containing at least one double bond, having from three to seven atoms in the chain, and consisting of carbon and hydrogen atoms, wherein at least one carbon atom is replaced by nitrogen, oxygen or sulphur.

"Halogens" are preferably fluorine, chlorine or bromine, being in particular fluorine or chlorine.

"Acceptable pharmaceutical salts" comprise conventional non-toxic salts obtained by salification with inorganic acids (e.g. hydrochloric, hydrobromide, sulphuric or phosphoric acids), or with organic acids (e.g. acetic, propionic, succinic, benzoic, cinnamic, mandelic, salicylic, glycolic, lactic, oxalic, malic, maleic, malonic, fumaric, tartaric, citric, p-toluenesulfonic or methanesulfonic acids).

In addition, the compounds of the present invention can exist in unsolvated as well as in solvated forms with pharmaceutically acceptable solvents such as water, ethanol and the like.

The compounds of the invention and their pharmaceutical acceptable salts can exist as single stereoisomers, racemates, and as mixtures of diastereoisomers. The compounds can exist also as geometric isomers. All such salts, solvates, geometric isomers, single stereoisomers, racemates and mixtures thereof, are intended to be within the scope of the invention.

The present invention comprises metabolic precursors of compounds of formula **(I)**. The term "metabolic precursors" means compounds having a different structure from that of the relevant formula **(I)**, which after administration to the patient are directly or indirectly transformed into a compound of said formula **(I)**. Methods for selecting metabolic precursors and their relative preparation are described for example in the book by Bundgaard (Bundgaard, H. ed., "Design of Prodrugs", Elsevier, 1985). Preferably in formula **(I)**:
the dotted line is an optional additional bond;
   - **R¹**: is hydrogen;
   - **R², R³**: are, independently, hydrogen; C₁-C₃ alkyl, phenyl, naphthyl, or taken together with the carbon atoms to which they are bound form a bridged bicyclic ring or a fused heterocycle;
   - **X**: is CH or nitrogen;
   - **Y**: is a bond, (CH₂)ₘC**R⁴R⁵**(CH₂)ₙ, or **NR⁶**;
   - **m, n**: are, independently, zero or 1;
   - **R⁴**, **R⁵**: are, independently, hydrogen; CN; C₁-C₃ alkyl, optionally substituted by phenyl; (CO)-phenyl; phenyl or naphthyl; 6-membered heterocyclyl or heteroaryl, containing one or two heteroatoms selected from nitrogen or oxygen, optionally fused with one or more phenyl rings; or taken together with the carbon atom to which they are bound form a spirocycle; or **R⁴** taken together with the carbon atom to which it is bound and **R²** together with the carbon atom to which it is bound can form a fused heterocycle;
   - **R⁶**: is hydrogen; C₁-C₃ alkyl, optionally substituted by phenyl; phenyl; 6-membered heterocyclyl or heteroaryl, containing one or two nitrogen heteroatoms, optionally fused with one or more phenyl rings; (CO)-C₁-C₃ alkyl; (CO)-phenyl;
and the pharmaceutically acceptable salts thereof, provided that, when the dotted line is an additional bond, then **Y** is C**R⁴R⁵**, wherein **R⁴** is as defined above and **R⁵** is absent.

Examples of specific compounds belonging to formula **(I)** are the following:
(E)-N-Hydroxy-3-{4-[(E)-3-(4-methyl-piperazin-1-yl)-3-oxo-propenyl]-phenyl}-acrylamide;
(E)-N-Hydroxy-3-{3-[(E)-3-(4-methyl-piperazin-1-yl)-3-oxo-propenyl]-phenyl}-acrylamide;
(E)-N-Hydroxy-3-{3-[(E)-3-oxo-3-(4-phenyl-piperazin-1-yl)-propenyl]-phenyl}-acrylamide;
(E)-3-[3-((E)-3-[1,4']Bipiperidinyl-1'-yl-3-oxo-propenyl)-phenyl]-N-hydroxy-acrylamide;
(E)-N-Hydroxy-3-{3-[(E)-3-oxo-3-(*cis*-3,4,5-trimethyl-piperazi\n-1-yl)-propenyl]-phenyl}-acrylamide;
(E)-3-{3-[(E)-3-((1S,4S)-5-methyl-2,5-diaza-bicyclo[2.2.1]hept-2-yl)-3-oxo-propenyl]-phenyl}-N-hydroxy-acrylamide;
(E)-N-hydroxy-3-{4-[(E)-3-oxo-3-(4-phenyl-piperazin-1-yl)-propenyl]-phenyl}-acrylamide;
(E)-3-[4-((E)-3-[1,4']bipiperidinyl-1'-yl-3-oxo-propenyl)-phenyl]-N-hydroxy-acrylamide;
(E)-N-hydroxy-3-{4-[(E)-3-oxo-3-(*cis*-3,4,5-trimethyl-piperazin-1-yl)-propenyl]-phenyl}-acrylamide;
(E)-N-hydroxy-3-{4-[(E)-3-oxo-3-((1S,4S)-5-methyl-2,5-diaza-bicyclo[2.2.1]hept-2-yl)-propenyl]-phenyl}-acrylamide;
(E)-N-hydroxy-3-{5-[(E)-3-oxo-3-(4-phenyl-piperazin-1-yl)-propenyl]-pyridin-2-yl}-acrylamide;
(E)-N-hydroxy-3-{5-[(E)-3-(4-methyl-piperazin-1-yl)-3-oxo-propenyl]-pyridin-2-yl}-acrylamide;
(E)-N-hydroxy-3-{6-[(E)-3-oxo-3-(4-phenyl-piperazin-1-yl)-propenyl]-pyridin-2-yl}-acrylamide;
(E)-N-hydroxy-3-{6-[(E)-3-(4-methyl-piperazin-1-yl)-3-oxo-propenyl]-pyridin-2-yl}-acrylamide;
(E)-3-(6-{(E)-3-[4-(3-chloro-phenyl)-piperazin-1-yl]-3-oxo-propenyl}-pyridin-2-yl)-N-hydroxy-acrylamide;
(E)-3-{6-[(E)-3-(4-benzoyl-piperazin-1-yl)-3-oxo-propenyl]-pyridin-2-yl}-N-hydroxy-acrylamide hydrochloride;
(E)-3-(6-{(E)-3-[4-(2-chloro-phenyl)-piperazin-1-yl]-3-oxo-propenyl}-pyridin-2-yl)-N-hydroxy-acrylamide hydrochloride;
(E)-N-hydroxy-3-{6-[(E)-3-oxo-3-(4-phenyl-piperidin-1-yl)-propenyl]-pyridin-2-yl}-acrylamide hydrochloride;
(E)-N-hydroxy-3-{6-[(E)-3-oxo-3-(4-pyrimidin-2-yl-piperazin-1-yl)-propenyl]-pyridin-2-yl}-acrylamide hydrochloride;
(E)-3-(6-{(E)-3-[4-(4-chloro-phenyl)-piperazin-1-yl]-3-oxo-propenyl}-pyridin-2-yl)-N-hydroxy-acrylamide hydrochloride;
(E)-3-{6-[(E)-3-(4-benzyl-piperazin-1-yl)-3-oxo-propenyl]-pyridin-2-yl}-N-hydroxy-acrylamide hydrochloride;
(E)-N-hydroxy-3-{6-[(E)-3-oxo-3-(4-phenethyl-piperazin-1-yl)-propenyl]-pyridin-2-yl}-acrylamide hydrochloride;
(E)-3-{6-[(E)-3-(4-benzoyl-piperidin-1-yl)-3-oxo-propenyl]-pyridin-2-yl}-N-hydroxy-acrylamide hydrochloride;
(±)-(E)-N-hydroxy-3-{6-[(E)-3-oxo-3-(3-phenyl-piperidin-1-yl)-propenyl]-pyridin-2-yl}-acrylamide hydrochloride;
(E)-N-hydroxy-3-{6-[(E)-3-oxo-3-(4-phenyl-3,6-dihydro-2H-pyridin-1-yl)-propenyl]-pyridin-2-yl}-acrylamide hydrochloride;
(E)-3-(6-{(E)-3-[4-(2,6-dimethyl-phenyl)-piperidin-1-yl]-3-oxo-propenyl}-pyridin-2-yl)-N-hydroxy-acrylamide hydrochloride;
(E)-3-{6-[(E)-3-(4-cyano-4-phenyl-piperidin-1-yl)-3-oxo-propenyl]-pyridin-2-yl}-N-hydroxy-acrylamide hydrochloride;
(E)-N-hydroxy-3-{6-[(E)-3-oxo-3-(4-pyridin-2-yl-piperazin-1-yl)-propenyl]-pyridin-2-yl}-acrylamide hydrochloride;
(E)-N-hydroxy-3-(6-{(E)-3-oxo-3-[4-(2-oxo-2,3-dihydro-benzoimidazol-1-yl)-piperidin-1-yl]-propenyl}-pyridin-2-yl)-acrylamide hydrochloride;
(E)-3-(6-{(E)-3-[4-(2,6-dimethyl-phenyl)-piperazin-1-yl]-3-oxo-propenyl}-pyridin-2-yl)-N-hydroxy-acrylamide hydrochloride;
(E)-N-hydroxy-3-{6-[(E)-3-oxo-3-(4-oxo-1-phenyl-1,3,8-triaza-spiro[4.5]dec-8-yl)-propenyl]-pyridin-2-yl}-acrylamide hydrochloride;
(±)-(E)-N-hydroxy-3-{6-[(E)-3-oxo-3-(3-phenyl-piperazin-1-yl)-propenyl]-pyridin-2-yl}-acrylamide hydrochloride;
(E)-N-hydroxy-3-(6-{(E)-3-[4-(4-methoxy-phenyl)-piperazin-1-yl]-3-oxo-propenyl}-pyridin-2-yl)-acrylamide hydrochloride;
(E)-N-hydroxy-3-(6-{(E)-3-oxo-3-[4-(4-trifluoromethyl-phenyl)-piperazin-1-yl]-propenyl}-pyridin-2-yl)-acrylamide hydrochloride;
(E)-3-(6-{(E)-3-[4-(4-cyano-phenyl)-piperazin-1-yl]-3-oxo-propenyl}-pyridin-2-yl)-N-hydroxy-acrylamide hydrochloride;
(E)-3-(6-{(E)-3-[4-(4-fluoro-phenyl)-piperazin-1-yl]-3-oxo-propenyl}-pyridin-2-yl)-N-hydroxy-acrylamide hydrochloride;
(E)-3-(6-{(E)-3-[4-(4-bromo-phenyl)-piperazin-1-yl]-3-oxo-propenyl}-pyridin-2-yl)-N-hydroxy-acrylamide hydrochloride;
(E)-3-(6-{(E)-3-[4-(4-benzyloxy-phenyl)-piperazin-1-yl]-3-oxo-propenyl}-pyridin-2-yl)-N-hydroxy-acrylamide hydrochloride;
(E)-N-hydroxy-3-{6-[(E)-3-oxo-3-(4-pyridin-4-yl-piperazin-1-yl)-propenyl]-pyridin-2-yl}-acrylamide hydrochloride;
(E)-3-(6-{(E)-3-[5-(4-chloro-phenyl)-(1S,4S)-2,5-diaza-bicyclo[2.2.1]hept-2-yl]-3-oxo-propenyl}-pyridin-2-yl)-N-hydroxy-acrylamide trifluoroacetate;
(±)-(E)-N-hydroxy-3-{6-[(E)-3-oxo-3-(3-o-tolyl-piperidin-1-yl)-propenyl]-pyridin-2-yl}-acrylamide hydrochloride;
(±)-(E)-N-hydroxy-3-{6-[(E)-3-oxo-3-(3-m-tolyl-piperidin-1-yl)-propenyl]-pyridin-2-yl}-acrylamide hydrochloride;
(±)-(E)-N-hydroxy-3-{6-[(E)-3-(3-naphthalen-1-yl-piperidin-1-yl)-3-oxo-propenyl]-pyridin-2-yl}-acrylamide hydrochloride;
(E)-N-hydroxy-3-{6-[(E)-3-oxo-3-(1,3,4,5-tetrahydro-pyrido[4,3-b]indol-2-yl)-propenyl]-pyridin-2-yl}-acrylamide hydrochloride;
(±)-(E)-N-hydroxy-3-{6-[(E)-3-oxo-3-(3-p-tolyl-piperidin-1-yl)-propenyl]-pyridin-2-yl}-acrylamide hydrochloride;
(±)-(E)-N-hydroxy-3-{6-[(E)-3-(3-naphthalen-2-yl-piperidin-1-yl)-3-oxo-propenyl]-pyridin-2-yl}-acrylamide hydrochloride;
(±)-(E)-3-(6-{(E)-3-[3-(4-fluoro-phenyl)-piperidin-1-yl]-3-oxo-propenyl}-pyridin-2-yl)-N-hydroxy-acrylamide hydrochloride;
(E)-N-hydroxy-3-(6-{(E)-3-[4-(4-isopropyl-phenyl)-piperazin-1-yl]-3-oxo-propenyl}-pyridin-2-yl)-acrylamide hydrochloride;
(E)-3-(6-{(E)-3-[4-(4-tert-butyl-phenyl)-piperazin-1-yl]-3-oxo-propenyl}-pyridin-2-yl)-N-hydroxy-acrylamide hydrochloride;
(E)-N-hydroxy-3-(6-{(E)-3-[4-(4-methanesulfonyl-phenyl)-piperazin-1-yl]-3-oxo-propenyl}-pyridin-2-yl)-acrylamide hydrochloride;
(±)-(E)-N-hydroxy-3-{6-[(E)-3-oxo-3-(3-phenyl-pyrrolidin-1-yl)-propenyl]-pyridin-2-yl}-acrylamide hydrochloride;
(±)-(E)-N-hydroxy-3-{6-[(E)-3-oxo-3-(2-phenyl-piperidin-1-yl)-propenyl]-pyridin-2-yl}-acrylamide hydrochloride;
(±)-(E)-N-hydroxy-3-{6-[(E)-3-oxo-3-(4-phenyl-azepan-1-yl)-propenyl]-pyridin-2-yl}-acrylamide hydrochloride
(E)-3-{6-[(E)-3-(3,4-dihydro-2H-quinolin-1-yl)-3-oxo-propenyl]-pyridin-2-yl}-N-hydroxy-acrylamide trifluoroacetate;
(E)-N-hydroxy-3-{6-[(E)-3-oxo-3-(1,3,4,9-tetrahydro-beta-carbolin-2-yl)-propenyl]-pyridin-2-yl}-acrylamide hydrochloride;
(E)-3-{6-[(E)-3-(4-benzooxazol-2-yl-piperidin-1-yl)-3-oxo-propenyl]-pyridin-2-yl}-N-hydroxy-acrylamide hydrochloride;
(E)-3-{6-[(E)-3-(3,4-dihydro-1H-isoquinolin-2-yl)-3-oxo-propenyl]-pyridin-2-yl}-N-hydroxy-acrylamide hydrochloride;
(E)-3-(6-{(E)-3-[4-(1H-benzoimidazol-2-yl)-piperidin-1-yl]-3-oxo-propenyl}-pyridin-2-yl)-N-hydroxy-acrylamide hydrochloride;
(E)-3-(6-{(E)-3-[spiro[indene-1,4'-piperidine-1'-yl]-3-oxo-propenyl}-pyridin-2-yl)-N-hydroxy-acrylamide hydrochloride;
(E)-3-(6-{(E)-3-[spiro[2-benzofuran-1,4'-piperidine-1'-yl]-3-oxo-propenyl}-pyridin-2-yl)-N-hydroxy-acrylamide hydrochloride;
(E)-N-hydroxy-3-(6-{(E)-3-oxo-3-[4-(2-phenyl-benzoimidazol-1-yl)-piperidin-1-yl]-propenyl}-pyridin-2-yl)-acrylamide hydrochloride;
(±)-(E)-N-hydroxy-3-{6-[(E)-3-(4-methyl-3-phenyl-piperazin-1-yl)-3-oxo-propenyl]-pyridin-2-yl}-acrylamide hydrochloride
(±)-(E)-3-{6-[(E)-3-(4-ethyl-3-phenyl-piperazin-1-yl)-3-oxo-propenyl]-pyridin-2-yl}-N-hydroxy-acrylamide hydrochloride;
(±)-(E)-3-{6-[(E)-3-(4-benzyl-3-phenyl-piperazin-1-yl)-3-oxo-propenyl]-pyridin-2-yl}-N-hydroxy-acrylamide hydrochloride;
(±)-(E)-3-{6-[(E)-3-(4-acetyl-3-phenyl-piperazin-1-yl)-3-oxo-propenyl]-pyridin-2-yl}-N-hydroxy-acrylamide hydrochloride;
(±)-(E)-N-hydroxy-3-(6-{(E)-3-[3-(2-methoxy-phenyl)-piperidin-1-yl]-3-oxo-propenyl}-pyridin-2-yl)-acrylamide hydrochloride;
(±)-(E)-N-hydroxy-3-(6-{(E)-3-[3-(3-methoxy-phenyl)-piperidin-1-yl]-3-oxo-propenyl}-pyridin-2-yl)-acrylamide hydrochloride;
(±)-(E)-N-hydroxy-3-(6-{(E)-3-[3-(4-methoxy-phenyl)-piperidin-1-yl]-3-oxo-propenyl}-pyridin-2-yl)-acrylamide hydrochloride;
(±)-(E)-N-hydroxy-3-(6-{(E)-3-oxo-3-[3-(2-trifluoromethyl-phenyl)-piperidin-1-yl]-propenyl}-pyridin-2-yl)-acrylamide hydrochloride;
(±)-(E)-N-hydroxy-3-(6-{(E)-3-oxo-3-[3-(3-trifluoromethyl-phenyl)-piperidin-1-yl]-propenyl}-pyridin-2-yl)-acrylamide hydrochloride;
(±)-(E)-N-hydroxy-3-(6-{(E)-3-oxo-3-[3-(4-trifluoromethyl-phenyl)-piperidin-1-yl]-propenyl}-pyridin-2-yl)-acrylamide hydrochloride;
(E)-N-hydroxy-3-{6-[(E)-3-oxo-3-((S)-3-phenyl-piperidin-1-yl)-propenyl]-pyridin-2-yl}-acrylamide hydrochloride;
(E)-N-hydroxy-3-{6-[(E)-3-oxo-3-((R)-3-phenyl-piperidin-1-yl)-propenyl]-pyridin-2-yl}-acrylamide hydrochloride;
(E)-N-hydroxy-3-{3-[(E)-3-oxo-3-(4-phenyl-3,6-dihydro-2H-pyridin-1-yl)-propenyl]-phenyl}-acrylamide hydrochloride;
(E)-3-(3-{(E)-3-[4-(4-chloro-phenyl)-piperazin-1-yl]-3-oxo-propenyl}-phenyl)-N-hydroxy-acrylamide hydrochloride;
(E)-3-{3-[(E)-3-(4-benzoyl-piperidin-1-yl)-3-oxo-propenyl]-phenyl}-N-hydroxy-acrylamide;
(±)-(E)-N-hydroxy-3-{3-[(E)-3-oxo-3-(3-m-tolyl-piperidin-1-yl)-propenyl]-phenyl}-acrylamide;
(±)-(E)-N-hydroxy-3-{3-[(E)-3-oxo-3-(3-o-tolyl-piperidin-1-yl)-propenyl]-phenyl}-acrylamide;
(±)-(E)-N-hydroxy-3-{3-[(E)-3-oxo-3-(3-p-tolyl-piperidin-1-yl)-propenyl]-phenyl}-acrylamide;
(E)-N-hydroxy-3-{3-[(E)-3-oxo-3-((R)-3-phenyl-piperidin-1-yl)-propenyl]-phenyl}-acrylamide;
(E)-N-hydroxy-3-{3-[(E)-3-oxo-3-((S)-3-phenyl-piperidin-1-yl)-propenyl]-phenyl}-acrylamide;
(±)-(E)-N-hydroxy-3-{3-[(E)-3-oxo-3-(3-phenyl-piperidin-1-yl)-propenyl]-phenyl}-acrylamide;
(±)-(E)-3-(3-{(E)-3-[3-(4-fluoro-phenyl)-piperidin-1-yl]-3-oxo-propenyl}-phenyl)-N-hydroxy-acrylamide;
(E)-3-(3-{(E)-3-[4-(3-chloro-phenyl)-piperazin-1-yl]-3-oxo-propenyl}-phenyl)-N-hydroxy-acrylamide hydrochloride;
(E)-3-{3-[(E)-3-(4-benzyl-piperidin-1-yl)-3-oxo-propenyl]-phenyl}-N-hydroxy-acrylamide;
(E)-3-(3-{(E)-3-[4-(2-chloro-phenyl)-piperazin-1-yl]-3-oxo-propenyl}-phenyl)-N-hydroxy-acrylamide hydrochloride;
(E)-3-(3-{(E)-3-[4-(4-cyano-phenyl)-piperazin-1-yl]-3-oxo-propenyl}-phenyl)-N-hydroxy-acrylamide;
(E)-N-hydroxy-3-{3-[(E)-3-oxo-3-(5-phenyl-3,6-dihydro-2H-pyridin-1-yl)-propenyl]-phenyl}-acrylamide;
(E)-N-hydroxy-3-(3-((E)-3-oxo-3-(3-phenylpiperazin-1-yl)prop-1-enyl)phenyl)acrylamide;
(E)-N-hydroxy-3-(3-{(E)-3-oxo-3-[4-(4-trifluoromethyl-phenyl)-piperazin-1-yl]-propenyl}-phenyl)-acrylamide;
(E)-3-(6-{(E)-3-[3-(2-fluoro-phenyl)-piperidin-1-yl]-3-oxo-propenyl}-pyridin-2-yl)-N-hydroxy-acrylamide hydrochloride;
(E)-3-(6-{(E)-3-[3-(3-fluoro-phenyl)-piperidin-1-yl]-3-oxo-propenyl}-pyridin-2-yl)-N-hydroxy-acrylamide hydrochloride;
(E)-3-[6-((E)-3-{3-[3-(4-fluoro-phenyl)-1,2,4-oxadiazol-5-yl]-piperidin-1-yl}-3-oxo-propenyl)-pyridin-2-yl]-N-hydroxy-acrylamide hydrochloride;
(E)-N-hydroxy-3-{6-[(E)-3-oxo-3-(5-phenyl-3,6-dihydro-2H-pyridin-1-yl)-propenyl]-pyridin-2-yl}-acrylamide hydrochloride;
(E)-N-hydroxy-3-{6-[(E)-3-oxo-3-(6-phenyl-3,4-dihydro-1H-isoquinolin-2-yl)-propenyl]-pyridin-2-yl}-acrylamide trifluoro-acetate;
(E)-N-hydroxy-3-{6-[(E)-3-oxo-3-(2-phenyl-6,7-dihydro-4H-thiazolo[5,4-c]pyridin-5-yl)-propenyl]-pyridin-2-yl}-acrylamide hydrochloride;
(E)-N-hydroxy-3-{6-[(E)-3-oxo-3-(2-phenyl-6,7-dihydro-5H-thiazolo[5,4-b]pyridin-4-yl)-propenyl]-pyridin-2-yl}-acrylamide trifluoro-acetate.

The present invention also comprises a process for preparing compounds of formula **(I)**. The compounds according to the present invention can be prepared, for example, as shown in the reaction schemes below and according to the reaction steps specified as follows, or, particularly, in a manner as described by way of example in the following examples, or analogously or similarly thereto using preparation procedures and synthesis strategies known to the person skilled in the art.

In a preferred embodiment, the compounds of formula **(I)** are prepared from a compound of formula **A1** 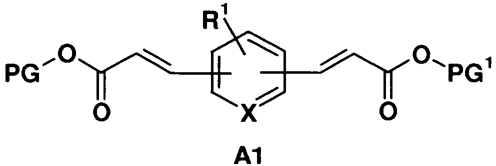 wherein **R¹**, **X** are as defined above and **PG**, **PG¹** are protecting groups chosen among those known in the art, for example methyl, *tert*-butyl, etc. The compounds of formula **(I)** can be obtained by removing the PG and **PG¹** groups; the deprotected sites are treated with suitable precursors of the moieties: 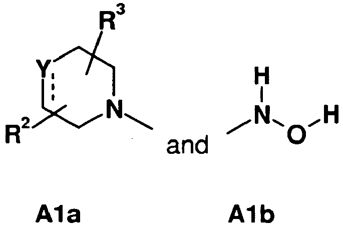 wherein the dotted line, **R², R³** and **Y** are as defined above. Independent deprotection reactions are preferably conducted; the order of deprotection is indifferent; however once a first protecting group (**PG** or **PG¹**) has been removed, the resulting structure is treated first with the relevant precursor; then the second protecting group is removed and the resulting structure is then treated with the other precursor.

The resulting synthetic route is represented according to **Scheme A:** 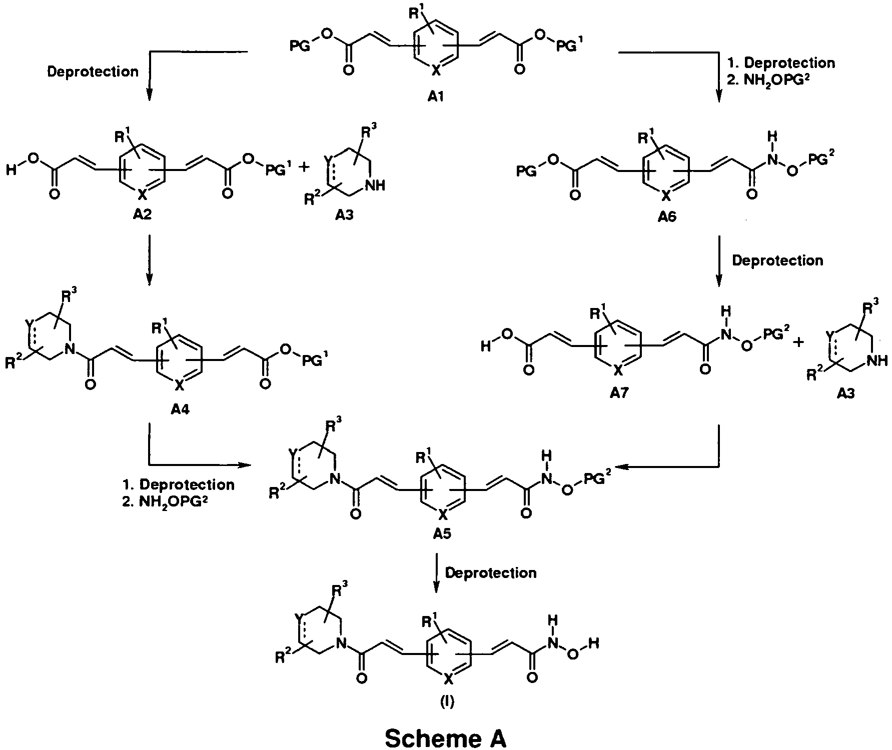 wherein the dotted line, **R¹, R², R³, X** and **Y** are as defined above and **PG**, **PG¹** and **PG²** are protecting groups among those known in the art, for example methyl, *tert-*butyl, etc. for PG and **PG¹**, and O-(tetrahydro-2H-pyran-2-yl), etc. for **PG²**.

A compound of formula **A1** can be deprotected into a compound of formula **A2** according to known methods, e.g. by treatment of a methylester with LiOH or NaOH in a suitable solvent, for example methanol or a methanol/water mixture at a temperature ranging from 0 °C to the boiling point of the solvent.

The reaction of a compound of formula **A2** with a compound of formula **A3** can be carried out with coupling agents such as EDC (1-(3-dimethylaminopropyl)-3-ethylcarbodiimide), in the presence of a suitable base (e.g. triethylamine or diisopropylethylamine) in a suitable solvent (e.g. tetrahydrofuran, dichloromethane or DMF). Generally, an activator of the condensation reaction, such as HOBT (1-hydroxybenzotriazole) or HOAT (1-hydroxy-7-aza-benzotriazole), can be added to the reaction mixture. The reaction can be carried out at room temperature for a period lasting between about 2 and 12 h.

A compound of formula **A4** can be converted into a compound of formula **A5** by removing the protecting group **PG¹** according to known methods, e.g. by treatment of a *tert*-butyl ester derivative with TFA (trifluoroacetic acid) in a suitable solvent such as dichloromethane at a temperature ranging from 0 °C to room temperature, and the reaction of the formed product with the protected hydroxylamine NH₂O**PG²**, which can be carried out under the same conditions like the reaction between a compound of formula **A2** with a compound of formula **A3.**

Deprotection of the hydroxylamine to give a compound of formula **(I)** can be achieved by known methods, for example in the case of tetrahydropyranyl, using HCl in aprotic solvents (such as THF, diethylether or dioxane).

Alternatively, a compound of formula **A1** can be converted into a compound of formula **A6** according to the same conditions described above for the conversion of a compound of formula **A4** into a compound of formula **A5.** The protecting group PG of a compound of formula **A6** can be removed according to known methods, e.g. by treatment of a methylester with LiOH or NaOH in a suitable solvent, for example methanol or a methanol/water mixture at a temperature ranging from 0 °C to the boiling point of the solvent.

Reaction between a compound of formula **A7** and a compound of formula A3 can be carried out under the same conditions described above for the reaction between a compound of formula **A2** and a compound of formula.

Compounds of general formula **A1** can be prepared according to **Scheme B:**

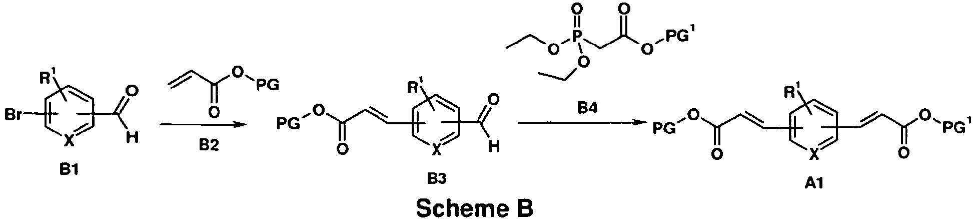

Compounds of formula **B1** are known compounds or can be prepared by known methods. Reaction of a compound of formula **B1** with the protected acroylester **B2** can be carried out according to the Heck reaction. The reaction conditions are described for example in the book by Larhed and Hallberg (Larhed, M.; Hallberg, A. "Handbook of Organopalladium Chemistry for Organic Synthesis", Negishi, E., Ed.; Wiley-Interscience, 2002). The reaction can be carried out in a suitable organic solvent (e.g. DMF) in the presence of palladium salts (e.g. palladium acetate), organic or inorganic bases (e.g. triethylamine, 1,4-diazabicyclo[2,2,2]-octane, sodium or potassium carbonate) and phosphine ligand derivatives, such as triphenylphosphine, at a temperature between room temperature and the boiling point of the solvent. Reaction of a compound of formula **B3** with a protected diethyl-phosphonoacetate **B4** can be carried out according to the Horner-Emmons reaction. The reaction can be carried out in the presence of an inorganic base, e.g. NaH, in an aprotic solvent, such as tetrahydrofuran, at a temperature between about 0°C and room temperature.

Alternatively, compounds of general formula **A1** can be prepared according to Scheme **C:**

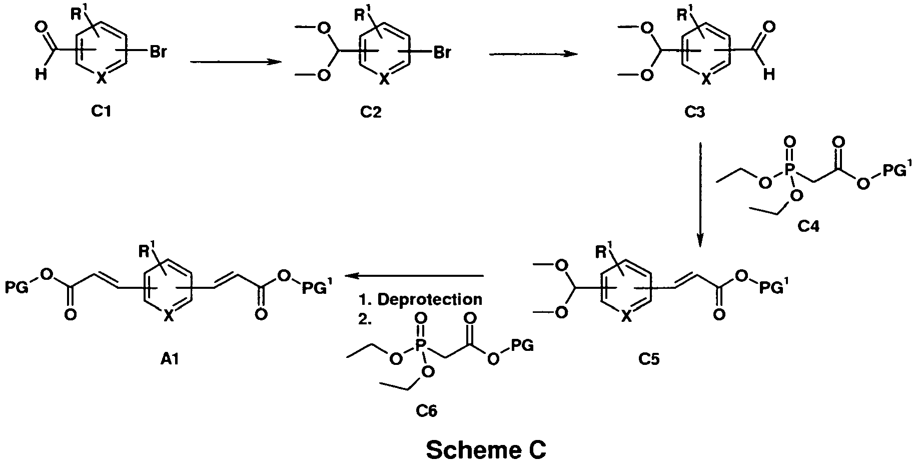

Compounds of formula **C1** are known compounds or can be prepared by known methods. The formyl moiety can be protected according to known methods, e.g. by treatment with trimethyl orthoformate and p-toluensulphonic acid in a suitable solvent, for example methanol at a temperature between 0 °C and the boiling point of the solvent. Compounds of formula **C3** can be obtained by treating a compound of formula **C2** firstly with alkyl lithium, e.g. n-butyl-lithium, then with DMF in an aprotic solvent (e.g. THF) at a temperature between about -78°C and room temperature. The Horner-Emmons reaction between a compound of formula **C3** and a protected diethyl-phosphonoacetate **C4** can be carried out under the same conditions of the reaction between a compound of formula **B3** and the protected diethyl-phosphonoacetate **B4** as outlined in **Scheme B**. A compound of formula **C5** can be deprotected according to known methods, e.g. by treatment with hydrochloric acid in an appropriate solvent, for example tetrahydrofuran at a temperature ranging from 0 °C to the boiling point of the solvent. Subsequent reaction with a protected diethyl-phosphonoacetate **C6** can be carried out under the same conditions like the reaction between a compound of formula **B3** and the protected diethyl-phosphonoacetate **B4** as outlined in **Scheme B**.

The invention also comprises a method for preventing and/or treating diseases linked to the disregulation of histone deacetylase activity consisting into the administration to a patient a pharmacologically useful quantity of one or more compounds of formula **(I)**, as previously defined. The invention includes the same compounds for use in the prevention or treatment of the aforesaid diseases. Further provided by the invention is the use of the same compounds for the manufacture of a medicament for the prevention or treatment of the aforesaid diseases.

In view of the above described mechanisms of action, the compounds of the present invention are useful in the prevention or treatment of tumor type diseases, including but not limited to: acute and chronic myeloid leukaemia, acute and chronic lymphoblastic leukaemia, myelodysplastic syndromes, multiple myeloma, Hodgkin's disease, non-Hodgkin's lymphomas, cutaneous and peripheral T-cell lymphoma; mammary tumors; pulmonary tumors and pleural mesotheliomas, adenocarcinoma, non-small lung cancer, small-cell lung cancer; skin tumors including basal cell carcinomas (basaliomas), melanomas, squamous cell carcinoma, Kaposi's sarcoma, keratocanthomas; osteosarcomas, fibrosarcomas, rhabdomyosarcomas, neuroblastomas, glioblastomas, cerebral tumors, testicular and ovarian tumors, endometrial and prostate tumors (for example advanced prostate cancer), thyroid carcinomas (for example tyroid follicular cancer), colon cancers (for example colon adenocarcinoma, colon adenoma), gastric tumors and gastrointestinal adenocarcinomas, hepatic carcinomas, pancreatic carcinomas (for example exocrine pancreatic carcinoma), renal tumors, teratocarcinomas and embryonic carcinomas. The compounds of the invention are also useful in the prevention or treatment of neurological conditions, including, but not limited to, epilepsy, cerebral ischemia, spinal and bulbar muscular atrophy, Friedreich's ataxia, Huntington's disease, Alzheimer's disease, Parkinson's disease, amyotrophic lateral sclerosis, diseases caused by protein aggregates, Kennedy's disease, and multiple sclerosis.

The compounds of the invention are also useful in the prevention or treatment of mental retardation, including, but not limited to, fragile X syndrome and Rubinstein-Taybi syndrome.

The compounds of the invention are also useful in the prevention or treatment of psychiatric disorders, including, but not limited to, bipolar disorders and schizophrenia.

The compounds of the invention are also useful in the prevention or treatment of inflammatory diseases, including, but not limited to, inflammatory responses of the nervous system, intestinal and colitic diseases and arthritis.

The compounds of the invention are also useful in the prevention or treatment of immune disorders, including, but not limited to, autoimmune diseases, chronic immune reactions against the host, psoriasis, atopic dermatitis and systemic lupus erythematosus.

The compounds of the invention are also useful in the prevention or treatment of infections, including, but not limited to, HIV infections, malaria, leishmaniasis, infections by protozoa, fungi, phytotoxic agents, viruses and parasites.

The compounds of the invention are also useful in the prevention or treatment of cardiovascular disorders, including, but not limited to, hypertrophy and cardiac decompensation, and cardiac ischemia.

The compounds of the invention are also useful in the prevention or treatment of other diseases such as diabetes, fibrotic diseases of the skin, fibrosis, renal diseases, beta thalassemia and respiratory diseases, including, but not limited to, chronic obstructive pulmonary disorders and asthma.

The compounds of formula **(I)** can also be used in combination with additional agents, in particular anti tumor and differentiating agents, either by separate administrations, or by including the two active principles in the same pharmaceutical formulation. Non-exhaustive examples of suitable additional agents include:
a) other histone deacetylase inhibitors (for example SAHA, PXD101, JNJ-16241199, JNJ-26481585, SB939, ITF-2357, LBH589, PCI-24781, valproic acid, butyric acid, MS-275, MGCD0103 or FK-228);
b) retinoid receptor modulators such as 13-cis-retinoic acid, 9-cis-retinoic acid, bexarotene, alitretinoin, or tretinoin; vitamin D;
c) antiproliferative/antineoplastic drugs and combinations thereof, as used in medical oncology, such as alkylating agents (for example platin derivatives like cis-platin, carboplatin, oxaliplatin, lobaplatin, satraplatin, nedaplatin, heptaplatin; nitrogen mustard such as chlorambucil, chlormethine, cyclophosphamide, ifosfamide, melphalan; uramustine, busulphan, temozolomide or nitrosoureas); antimetabolites (for example antifolates such as aminopterin, methotrexate, pemetrexed, raltitrexed); purines such as cladribine, clofarabine, fludarabine, mercaptopurine, pentostatin, thioguanine; pyrimidines like capecitabine, cytarabine, fluorouracil, floxuridine, gemcitabine; cytosine arabinoside or hydroxyurea); antitumour antibiotics (for example anthracyclines like doxorubicin, daunomycin, epirubicin, idarabicin, mitoxantrone, valrubicin; or antibiotics from streptomyces like actinomycin, bleomycin, mitomycin, or plicamycin); antimitotic agents (for example vinca alkaloids like vincristine, vinblastine, vindesine or vinorelbine; taxoids like docetaxel or paclitaxel; epothilones like ixabepilone) and topoisomerase inhibitors (for example epipodophyllotoxins like etoposide and teniposide; amsacrine, hycaptamine, topotecan, irinotecan, rubitecan and camptothecin);
d) cytostatic agents such as antioestrogens (for example tamoxifen, toremifene, raloxifene, droloxifene and idoxifene), oestrogen receptor down regulators (for example fulvestrant), antiandrogens (for example bicalutamide, flutamide, nilutamide, liarozole or cyproterone acetate), LHRH antagonists or LHRH agonists (for example goserelin, leuprorelin or buserelin), progestogens (for example megestrol acetate), aromatase inhibitors (for example as anastrozole, letrozole, vorazole and exemestane) and inhibitors of 5-alpha-reductase such as finasteride;
e) agents that inhibit cancer cell invasion (for example metalloproteinase inhibitors and inhibitors of urokinase plasminogen activator receptor function);
f) inhibitors of growth factor function, for example growth factor antibodies, growth factor receptor antibodies (for example the anti-erbb2 antibody trastuzumab, the anti-erbbl antibody cetuximab and panitumumab), farnesyl transferase inhibitors, MEK inhibitors, tyrosine kinase inhibitors and serine/threonine kinase inhibitors, for example dasatinib, erlotinib, gefitinib, imatinib, lapatinib, nilotinib, sorafenib, sunitinib, everolimus, sirolimus temsirolimus;
g) antiangiogenic agents such as those that inhibit the effects of vascular endothelial growth factor, (for example the anti-vascular endothelial cell growth factor antibody bevacizumab [Avastin™]);
h) cell cycle inhibitors including for example CDK inhibitors (for example flavopiridol, roscovitine) and other inhibitors of cell cycle checkpoints; inhibitors of aurora kinase and other kinases involved in mitosis and cytokinesis regulation;
i) proteasome inhibitors (for example lactacystin, bortezomib, epoxomicin);
j) HSP90 inhibitors (for example 17-AAG, KOS-953, KOS-1022, CNF-1010, CNF-2024, IPI-504 or SNX 5422).

The invention also comprises pharmaceutical compositions characterized by containing one or more active principles of formula **(I)**, in association with pharmaceutically acceptable carrier, excipients and diluents.

The compounds of this invention can be administered via any of the accepted modes of administration or agents for serving similar utilities. Thus, administration can be, for example, oral, nasal, parental (intravenous, subcutaneous, intramuscular), including buccal, sublingual, rectal, topical, transdermal, intravesical, or using any other route of administration.

The compounds of formula **(I)** can be pharmaceutically formulated according to known methods. The pharmaceutical compositions can be chosen on the basis of the treatment requirements. Such compositions are prepared by blending and are suitably adapted to oral or parenteral administration, and as such can be administered in the form of tablets, capsules, oral preparations, powders, granules, pills, injectable or infusible liquid solutions, suspensions or suppositories.

Tablets and capsules for oral administration are normally presented in unit dose form and contain conventional excipients such as binders, fillers, diluents, tableting agents, lubricants, detergents, disintegrants, coloring agents, flavoring agents and wetting agents. The tablets can be coated using methods well known in the art.

Suitable fillers include cellulose, mannitol, lactose and other similar agents. Suitable disintegrants include polyvinylpyrrolidone and starch derivatives such as sodium glycolate starch. Suitable lubricants include, for example, magnesium stearate. Suitable wetting agents include sodium lauryl sulfate.

These oral solid compositions can be prepared by conventional methods of blending, filling or tableting. The blending operation can be repeated to distribute the active principle throughout compositions containing large quantities of fillers. Such operations are conventional.

Oral liquid preparations can be in the form of, for example, aqueous or oily suspensions, solutions, emulsions, syrups or elixirs, or can be presented as a dry product for reconstitution with water or with a suitable vehicle before use. Such liquid preparations can contain conventional additives such as suspending agents, for example sorbitol, syrup, methyl cellulose, gelatin, hydroxyethyl cellulose, carboxymethyl cellulose, aluminium stearate gel, or hydrogenated edible fats; emulsifying agents, such as lecithin, sorbitan monooleate, or acacia; non-aqueous vehicles (which can include edible oils), such as almond oil, fractionated coconut oil, oily esters such as esters of glycerine, propylene glycol, or ethyl alcohol; preservatives, such as methyl or propyl p-hydroxybenzoate or sorbic acid, and if desired, conventional flavoring or coloring agents.

Oral formulations also include conventional slow-release formulations such as enterically coated tablets or granules.

For parenteral administration, fluid unit dosages can be prepared, containing the compound and a sterile vehicle. The compound can be either suspended or dissolved, depending on the vehicle and concentration. The parenteral solutions are normally prepared by dissolving the compound in a vehicle, sterilising by filtration, filling suitable vials and sealing. Advantageously, adjuvants such as local anaesthetics, preservatives and buffering agents can also be dissolved in the vehicle. To increase stability, the composition can be frozen after having filled the vials and removed the water under vacuum. Parenteral suspensions are prepared in substantially the same manner, except that the compound can be suspended in the vehicle instead of being dissolved, and sterilized by exposure to ethylene oxide before suspending in the sterile vehicle. Advantageously, a surfactant or wetting agent can be included in the composition to facilitate uniform distribution of the compound of the invention.

Another means of administering the compounds of the invention regards topical treatment. Topical formulations can contain for example ointments, creams, lotions, gels, solutions, pastes and/or can contain liposomes, micelles and/or microspheres. Examples of ointments include oleaginous ointments such as vegetable oils, animal fats, semisolid hydrocarbons, emulsifiable ointments such as hydroxystearin sulfate, anhydrous lanolin, hydrophilic petrolatum, cetyl alcohol, glycerol monostearate, stearic acid, water soluble ointments containing polyethylene glycols of various molecular weights. A reference for the formulations is the book by Remington ("Remington: The Science and Practice of Pharmacy", Lippincott Williams & Wilkins, 2000). Creams, as known to formulation experts, are viscous liquids or semisolid emulsions, and contain an oil phase, an emulsifier and an aqueous phase. The oil phase generally contains petrolatum and an alcohol such as cetyl or stearic alcohol. The emulsifier in a cream formulation is chosen from non-ionic, anionic, cationic or amphoteric surface-active agents. The monophasic gels contain the organic molecules uniformly distributed in the liquid, which is generally aqueous, but they also preferably contain an alcohol and optionally an oil. Preferred gelling agents are crosslinked acrylic acid polymers (e.g. carbomer-type polymers, such as carboxypolyalkylenes, which are commercially available under the Carbopol™ trademark). Hydrophilic polymers are also preferred, such as polyoxyethylene, polyoxyethylene-polyoxypropylene copolymers and polyvinyl alcohol; cellulose polymers such as hydroxypropyl cellulose, hydroxyethyl cellulose, hydroxypropyl methylcellulose, hydroxypropyl methylcellulose phthalate and methylcellulose; gums, such as xanthan gum and tragacanth gum; sodium alginate; and gelatin. Dispersing agents such as alcohol or glycerin can be added for gel preparation. The gelling agent can be dispersed by finely chopping and/or mixing.

A further method of administering the compounds of the invention regards transdermal delivery. Typical transdermal formulations comprise conventional aqueous and non-aqueous vectors, such as creams, oils, lotions or pastes or can be in the form of membranes or medicated patches. One formulation provides that a compound of the invention is dispersed within a pressure sensitive patch which adheres to the skin. This formulation enables the compound to diffuse from the patch to the patient through the skin. For a constant release of the drug through the skin, natural rubber and silicon can be used as pressure sensitive adhesives.

The above mentioned uses and methods also include the possibility of coadministration of additional therapeutic agents, simultaneously or delayed with respect to the administration of the compound of formula **(I)**.

In the previously mentioned uses and methods, the dosage of the compounds of formula **(I)**, can vary depending upon a variety of factors including the patient type and condition, the degree of disease severity, mode and time of administration, diet and drug combinations. As an indication, they can be administered within a dose range of between 0.001 and 1000 mg/kg/day. The determination of optimum dosages for a particular patient is well known to one skilled in the art.

As is common practice, the compositions are normally accompanied by written or printed instructions for use in the treatment in question.

The following examples serve to provide further appreciation of the invention, but are not meant in any way to restrict the scope of the invention.

### EXPERIMENTAL PART

### 1. CHEMICAL SYNTHESIS

### Methods

Unless otherwise indicated, all the starting reagents were found to be commercially available and were used without any prior purification. Specifically, the following abbreviations may have been used in the descriptions of the experimental methods.

| | |
|---|---|
| NMR (Nuclear Magnetic Resonance) | 1H (proton) |
| MHz (Megahertz) | Hz (Hertz) |
| HPLC (High Performance Liquid Chromatography) | LC-MS (Liquid Chromatography Mass Spectrum) |
| s (seconds) | min (minutes) |
| h (hours) | mg (milligrams) |
| g (grams) | µl (microlitres) |
| ml (millilitres) | mmol (millimoles) |
| M (molarity) | rt (retention time in minutes) |
| RT (room temperature) | MW (microwave) |
| BOC (*tert*-butoxycarbonyl) | BOC₂O (di-tert-butyldicarbonate) |
| Bu₄NBr (tetrabutylammonium bromide) | CH₃CN (acetonitrile) |
| DCM (dichloromethane) | DMF (dimethylformamide) |
| DMSO (dimethyl sulfoxide) | DMSO-d₆ (deuterated dimethyl sulfoxide) |
| EDC (1-3(dimethylaminopropyl)-3-ethylcarbodiimide hydrochloride) | Et₂O (diethyl ether) |
| EtOAc (ethyl acetate) | EtOH (ethanol) |
| HCl (hydrochloric acid) | HOBT (1-hydroxybenzotriazole) |
| *i*-PrMgCl (isopropylmagnesium chloride) | *i*-PrOH (isopropyl alcohol) |
| K₂CO₃ (potassium carbonate) | KOH (potassium hydroxide) |
| LiOH (lithium hydroxide) | MeOH (methanol) |
| MeOD (deuterated methanol) | Na₂CO₃ (sodium carbonate) |
| Na₂SO₄ (sodium sulphate) | NaH (sodium hydride) |
| NaHCO₃ (sodium hydrogen carbonate) | NaOH (sodium hydroxide) |
| NH₂OTHP (O-(tetrahydro-2H-pyran-2-yl)hydroxylamine) | NH₄Cl (ammonium chloride) |
| NH₄OH (ammonium hydroxide) | Pd(OAc)₂ (palladium acetate) |
| PPh₃ (triphenylphosphine) | TEA (triethylamine) |
| TFA (trifluoroacetic acid) | THF (tetrahydrofuran) |

Except where indicated otherwise, all temperatures are expressed in °C (degrees centigrade) or K (Kelvin).

The ¹H-NMR spectra were acquired with a Bruker 300 MHz. The chemical shifts are expressed in parts per million (ppm, δ units). The coupling constants are expressed in Hertz (Hz) and the splitting patterns are described as s (singlet), bs (broad singlet), d (doublet), t (triplet), q (quartet), quint (quintet), m (multiplet).

The LC-MS experiments were performed according to the following methods.

### METHOD A: Waters Acquity UPLC, Micromass ZQ 2000 Single quadrupole (Waters). Flow rate: 0.6 ml/min splitting ratio MS: waste/1:4;

Mobile phase: A phase= water/CH₃CN 95/5 + 0.1% TFA; B phase= water/CH₃CN 5/95 + 0.1% TFA.

Gradient: 0-0.50 min (A: 98%, B: 2%), 0.50-6.00 min (A: 0%, B: 100%), 6.00-7.00 min (A: 0%, B: 100%), 7.00-7.10 min (A: 98%, B: 2%); 7.10-8.50 min (A: 98%, B: 2%) UV detection wavelength 254 nm or BPI; injection volume: 2µl

### METHOD B: Waters Acquity UPLC, Micromass ZQ 2000 Single quadrupole (Waters). Flow rate: 0.6 ml/min splitting ratio MS: waste/1:4;

Mobile phase: A phase= water/CH₃CN 95/5 + 0.1 % TFA; B phase= water/CH₃CN 5/95 + 0.1% TFA.

Gradient: 0-0.25 min (A: 98%, B: 2%), 0.25-3.30 min (A: 0%, B: 100%), 3.30-4.00 min (A: 0%, B: 100%), 4.00-4.10 min (A: 98%, B: 2%); 4.10-5.00 min (A: 98%, B: 2%) UV detection wavelength 254 nm or BPI; injection volume: 2µl

### METHOD C: Waters Acquity UPLC, Micromass ZQ 2000 Single quadrupole (Waters). Flow rate: 0.6 ml/min splitting ratio MS: waste/1:4;

Mobile phase: A phase= water/CH₃CN 95/5 + 0.1 % TFA; B phase= water/CH₃CN 5/95 + 0.1% TFA.

Gradient: 0-0.25 min (A: 95%, B: 5%), 0.25-3.30 min (A: 0%, B: 100%), 3.30-4.00 min (A: 0%, B: 100%), 4.00-4.10 min (A: 95%, B: 5%); 4.10-5.00 min (A: 95%, B: 5%) UV detection wavelength 254 nm or BPI; injection volume: 2µl

### METHOD D: Waters Acquity UPLC, Micromass ZQ 2000 Single quadrupole (Waters). Flow rate: 0.6 ml/min splitting ratio MS: waste/1:4;

Mobile phase: A phase= water/CH₃CN 95/5 + 0.1% TFA; B phase= water/CH₃CN 5/95 + 0.1% TFA.

Gradient: 0-0.25 min (A: 100%, B: 0%), 0.25-3.30 min (A: 0%, B: 100%), 3.30-4.00 min (A: 0%, B: 100%), 4.00-4.10 min (A: 100%, B: 0%); 4.10-5.00 min (A: 100%, B: 0%) UV detection wavelength 254 nm or BPI; injection volume: 2µl

### METHOD E: Waters 2777 (Sample Manager), Waters 1525 µ (Pump), Waters 2996 (PDA), Micromass ZQ 2000 Single quadrupole (Waters).

Flow rate: 0.7 ml/min splitting ratio MS: waste/1:4;

Mobile phase: A phase= water/CH₃CN 95/5 + 0.1% TFA; B phase= water/CH₃CN 5/95 + 0.1% TFA.

Gradient: 0-0.25 min (A: 95%, B: 5%), 0.25-3.5 min (A: 0%, B: 100%), 3.35-4.50 min (A: 0%, B: 100%), 4.50-4.60 min (A: 95%, B: 5%); 4.60-6.00 min (A: 95%, B: 5%) UV detection wavelength 254 nm or BPI; injection volume: 2µl

### METHOD F: Waters Acquity UPLC, Micromass ZQ Single quadrupole (Waters). Flow rate: 0.6 ml/min splitting ratio MS: waste/1:4;

Mobile phase: A phase= water/CH₃CN 95/5 + 0.1% TFA; B phase= water/CH₃CN 5/95 + 0.1% TFA.

Gradient: 0-0.50 min (A: 95%, B: 5%), 0.50-6.00 min (A: 0%, B: 100%), 6.00-7.00 min (A: 0%, B: 100%), 7.00-7.10 min (A: 95%, B: 5%); 7.10-8.50 min (A: 95%, B: 5%) UV detection wavelength 254 nm or BPI; injection volume: 2µl

### Preparation 1: (E)-3-{4-[(E)-2-(Tetrahydro-pyran-2-yloxycarbamoyl)-vinyl]-phenyl}-acrylic acid

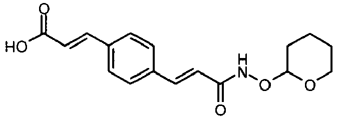

### STEP A: Ethyl (E)-3-[4-((E)-2-tert-butoxycarbonyl-vinyl)-phenyl]-acrylate

(E)-tert-Butyl-3-(4-formyl-phenyl)-acrylate (850 mg, 3.66 mmol) was dissolved in dry THF (5 ml) and added dropwise to a stirred mixture of triethyl phosphonoacetate (0.878 ml, 4.4 mmol) and NaH (60% oil dispersion, 228 mg, 5.7 mmol) in dry THF (5 ml).

The resulting solution was stirred at RT for 7 h and then additional NaH (60% oil dispersion, 100 mg, 2.5 mmol) was added. After stirring overnight at RT the reaction was quenched with water and the mixture was extracted with Et₂O. The organic phase was dried over Na₂SO₄ and evaporated *in vacuo.* The crude product (1.1 g) was used in the next step without any further purification.

### STEP B: (E)-3-[4-((E)-2-Ethoxycarbonyl-vinyl)-phenyl]-acrylic acid

A solution of ethyl (E)-3-[4-((E)-2-tert-butoxycarbonyl-vinyl)-phenyl]-acrylate (2.75 g, 9.1 mmol) and TFA (7 ml) in DCM (20 ml) was stirred at RT for 1 h. The solvent was removed *in vacuo* to give the title compound (2.13 g) as a white solid.
Y= 95%

### STEP C: Ethyl (E)-3-{4-[(E)-2-(tetrahydro-pyran-2-yloxycarbamoyl)-vinyl]-phenyl}-acrylate

(E)-3-[4-((E)-2-Ethoxycarbonyl-vinyl)-phenyl]-acrylic acid (1.5 g, 6.1 mmol) was dissolved in DCM (80 ml) and TEA (1.7 ml, 12.2 mmol). EDC (2.33 g, 12.2 mmol) and HOBT (1.65 g, 12.2 mmol) were added to the resulting mixture. After 15 min NH₂OTHP (856 mg, 7.32 mmol) was added and the resulting mixture was stirred at RT for 5 h. The solution was partitioned between 5% NaHCO₃ and Et₂O. The organic phase was dried over Na₂SO₄ and evaporated *in vacuo.* The crude mixture was purified by column chromatography (eluent: petroleum ether/EtOAc 1:1) to give the title compound (1.94 g).
Y=92%
¹H NMR (DMSO-d₆) δ (ppm): 11.24 (bs, 1 H), 7.77 (d, 2 H), 7.66 (d, 1 H), 7.57-7.65 (m, 2 H), 7.50 (d, 1 H), 6.68 (d, 1 H), 6.58 (d, 1 H), 4.92 (bs, 1 H), 4.20 (q, 2 H), 3.96 (bs, 1 H), 3.43-3.66 (m, 1 H), 1.38-1.96 (m, 6 H), 1.27 (t, 3 H).

### STEP D: (E)-3-{4-[(E)-2-(Tetrahydro-pyran-2-yloxycarbamoyl)-vinyl]-phenyl}-acrylic acid

1 M LiOH (3 ml) was added to a stirred solution of ethyl-(E)-3-{4-[(E)-2-(tetrahydropyran-2-yloxycarbamoyl)-vinyl]-phenyl}-acrylate (520 mg, 1.5 mmol) in THF (10 ml) and the resulting mixture was stirred at RT overnight. Further 1 M LiOH (1.5 ml) was added and, after stirring at RT overnight, the solution was partitioned between water and EtOAc. The aqueous phase was brought to acidic pH by adding citric acid (5% aqueous solution) at 0 °C and extracted twice with EtOAc. The organic layers were washed with brine, dried over Na₂SO₄ and evaporated *in vacuo* to give the title compound (445 mg) as a white powder.
Y=94%
¹H NMR (DMSO-d₆) δ (ppm): 12.35 (bs, 1 H), 11.23 (bs, 1 H), 7.69-7.84 (m, 2 H), 7.58-7.67 (m, 2 H), 7.59 (d, 1 H), 7.51 (d, 1 H), 6.57 (d, 2 H), 4.92 (bs, 1 H), 3.97 (bs, 1 H), 3.43-3.71 (m, 1 H), 1.31-1.95 (m, 6 H).

### Preparation 2: (E)-3-{3-[(E)-2-(Tetrahydro-pyran-2-yloxycarbamoyl)-vinyl]-phenyl}-acrylic acid

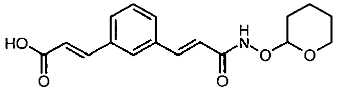

### STEP A: Ethyl (E)-3-(3-formyl-phenyl)-acrylate

A mixture of 3-bromobenzaldehyde (500 mg, 2.7 mmol), TEA (1.62 ml, 11.6 mmol), ethyl acrylate (270 mg, 2.7 mmol), NaHCO₃ (454 mg, 5.4 mmol) and PPh₃ (35 mg, 0.13 mmol) in DMF (13 ml), was degassed with N₂ and then Pd(OAc)₂ (12 mg, 0.054 mmol) was added. The resulting slurry was heated to 100 °C under N₂ for 6 h and then partitioned between water and Et₂O. The organic phase was washed with water, dried over Na₂SO₄ end evaporated to dryness. The crude reaction mixture was purified by column chromatography (eluent: petroleum ether/EtOAc 95:5) to give the title compound (206 mg).
Y=37%
¹H NMR (DMSO-d₆) δ (ppm): 10.04 (s, 1 H), 8.26 (t, 1 H), 8.06 (dt, 1 H), 7.94 (dt, 1 H), 7.75 (d, 1 H), 7.65 (t, 1 H), 6.76 (d, 1 H), 4.22 (q, 2 H), 1.28 (t, 3 H).

### STEP B: tert-Butyl (E)-3-[3-((E)-2-ethoxycarbonyl-vinyl)-phenyl]-acrylate

A solution of ethyl (E)-3-(3-formyl-phenyl)-acrylate (206 mg, 1 mmol) in dry THF (5 ml) was added dropwise under N₂ to a stirred mixture of *tert*-butyl-diethyl-phosphonoacetate (277.5 mg, 1.1 mmol) and NaH (60% oil dispersion, 52 mg, 1.3 mmol) in dry THF (10 ml), cooled down to 0 °C. The resulting solution was stirred at RT for 45 min, then diluted with acetone and stirred for 5 min. The solvent was removed *in vacuo* and the crude mixture was purified by column chromatograpy (eluent: petroleum ether/EtOAc 95:5) to give the title compound (260 mg).
Y=86%
¹H NMR (DMSO-d₆) δ (ppm): 8.13 (s, 1 H), 7.69-7.77 (m, 2 H), 7.66 (d, 1 H), 7.56 (d, 1 H), 7.45 (t, 1 H), 6.78 (d, 1 H), 6.67 (d, 1 H), 4.21 (q, 2 H), 1.50 (s, 9 H), 1.27 (t, 3 H).

### STEP C: (E)-3-[3-((E)-2-ethoxycarbonyl-vinyl)-phenyl]-acrylic acid

A mixture of *tert*-butyl (E)-3-[3-((E)-2-ethoxycarbonyl-vinyl)-phenyl]-acrylate (2.72 g, 9 mmol) and TFA (13.9 ml) in DCM (28 ml) was stirred at RT for 1 h. The solvent was removed *in vacuo* to give the title compound (2.13 g).
Y=96%
¹H NMR (DMSO-d₆) δ (ppm): 12.41 (bs, 1 H), 8.11 (s, 1 H), 7.67-7.78 (m, 2 H), 7.67 (d, 1 H), 7.60 (d, 1 H), 7.46 (t, 1 H), 6.77 (d, 1 H), 6.67 (d, 1 H), 4.21 (q, 2 H), 1.27 (t, 3 H).

### STEP D: Ethyl (E)-3-{3-[(E)-2-(tetrahydro-pyran-2-yloxycarbamoyl)-vinyl]-phenyl}-acrylate

(E)-3-[3-((E)-2-Ethoxycarbonyl-vinyl)-phenyl]-acrylic acid (2.13 g, 8.66 mmol) was dissolved in a mixture of DCM (80 ml) and TEA (2.4 ml, 17.3 mmol). EDC (3.31 g, 17.3 mmol) and HOBT (2.34 g, 17.3 mmol) were then added to the resulting solution. After 15 min NH₂OTHP (2.03 g, 17.3 mmol) was added and the resulting mixture was stirred at RT overnight. The solvent was evaporated and the residue was partitioned between 5% NaHCO₃ and Et₂O. The organic phase was dried over Na₂SO₄ and evaporated *in vacuo.* The crude mixture was purified by column chromatography (eluent: petroleum ether/EtOAc 1:1) to give the title compound (2.62 g).
Y=87%
¹H NMR (DMSO-d₆) δ (ppm): 11.22 (bs, 1 H), 7.95 (s, 1 H), 7.66 (d, 1 H), 7.29-7.82 (m, 4 H), 6.70 (d, 1 H), 6.60 (d, 1 H), 4.93 (bs, 1 H), 4.20 (q, 2 H), 3.98 (d, 1 H), 3.54 (d, 1 H), 1.35-1.89 (m, 6 H), 1.27 (t, 3 H).

### STEP E: (E)-3-{3-[(E)-2-(Tetrahydro-pyran-2-yloxycarbamoyl)-vinyl]-phenyl}-acrylic acid

1 M LiOH (15.2 ml) was added to a stirred solution of ethyl (E)-3-{3-[(E)-2-(tetrahydropyran-2-yloxycarbamoyl)-vinyl]-phenyl}-acrylate (2.62 g, 7.59 mmol) in THF (50 ml) and the mixture was stirred at RT overnight. Further 1 M LiOH (10 ml) was added and the reaction was carried out overnight. The solution was then partitioned between water and EtOAc. The aqueous phase was brought to acidic pH by adding citric acid (5% aqueous solution) at 0 °C and extracted twice with EtOAc. The organic layer was washed with brine, dried over Na₂SO₄ and evaporated *in vacuo* to give the title compound (2.01 g) as a white powder.
Y= 83%
¹H NMR (DMSO-d6) δ (ppm): 11.22 (bs, 1 H), 7.90 (s, 1 H), 7.65-7.76 (m, 1 H), 7.60 (d, 1 H), 7.32-7.65 (m, 3 H), 6.60 (d, 2 H), 4.92 (bs, 1 H), 3.80-4.15 (m, 1 H), 3.46-3.69 (m, 1 H), 1.31-1.95 (m, 6 H).

### Preparation 3: tert-butyl (E)-3-(5-formyl-pyridin-2-yl)-acrylate

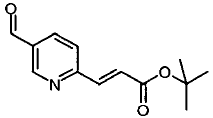

### STEP A: 2-Bromo-5-dimethoxymethyl-pyridine

A mixture of 6-bromo-pyridine-3-carbaldehyde (8.01 g, 43.1 mmol), paratoluenesulfonic acid (819 mg, 4.31 mmol) and trimethyl orthoformate (9.13 g, 86.1 mmol) in MeOH (80 ml) was stirred at RT for 3 h and then poured into 5% K₂CO₃ aqueous solution and extracted with Et₂O. The collected organic phase was dried over Na₂SO₄ and evaporated *in vacuo.* The crude mixture was purified by column chromatography (petroleum ether/EtOAc from 9:1 to 85:15) to give the title compound (7.18 g).
Y=72 %
¹H NMR (CDCl₃) δ (ppm): 8.40 (d, 1 H), 7.59 (dd, 1 H), 7.45 (d, 1 H), 5.39 (s, 1 H), 3.29 (s, 6 H).

### STEP B: 5-Dimethoxymethyl-pyridine-2-carbaldehyde

A solution of 2-bromo-5-dimethoxymethyl-pyridine (7.16 g, 30.86 mmol) in dry THF (20 ml) was added dropwise to a stirred solution of *i*-PrMgCl (2 M in Et₂O, 20.06 ml) in dry THF (20 ml) under N₂, keeping the temperature below 25 °C. The resulting mixture was stirred at RT for 4 h and then DMF (3.09 ml, 40.12 mmol) was added dropwise maintaining the temperature below 25 °C. The reaction was stirred for 2 h at RT and then quenched with water. The mixture was then partitioned between aqueous NH₄Cl and EtOAc and the organic phase was dried over Na₂SO₄ and evaporated *in vacuo.* The crude product was purified by column chromatography (petroleum ether/EtOAc 9:1) to give the title compound (3.27 g).
Y=60%
¹H NMR (DMSO-d₆) 8 (ppm): 10.00 (d, 1 H), 8.80 (ddd, 1 H), 8.01 (dddd, 1 H), 7.95 (dd, 1 H), 5.59 (s, 1 H), 3.31 (s, 6 H).

### STEP C: tert-Butyl (E)-3-(5-dimethoxymethyl-pyridin-2-yl)-acrylate

*tert*-Butyl diethylphosphonoacetate (5 g, 19.8 mmol) was dissolved in dry THF (20 ml) and added dropwise to a stirred suspension of NaH (60% oil dispersion, 936 mg, 23.4 mmol) in dry THF (20 ml). After 10 min at RT, a solution of 5-dimethoxymethyl-pyridine-2-carbaldehyde (3.26 g, 18.01 mmol) in dry THF (30 ml) was added dropwise under N₂, keeping the temperature below 25 °C. The resulting mixture was stirred at RT for 1 h and then partitioned between water and EtOAc. The organic phase was dried over Na₂SO₄ and evaporated to dryness. The crude mixture was purified by column chromatography (petroleum ether/EtOAc from 95:5 to 9:1) to give the title compound (3.86 g).
Y= 76%
¹H NMR (DMSO-d₆) δ (ppm): 8.61 (d, 1 H), 7.77-7.84 (m, 1 H), 7.69-7.78 (m, 1 H), 7.56 (d, 1 H), 6.81 (d, 1 H), 5.50 (s, 1 H), 3.29 (s, 6 H), 1.49 (s, 9 H).

### STEP D: tert-Butyl (E)-3-(5-formyl-pyridin-2-yl)-acrylate

1 M HCl (27.7 ml) was added to a stirred solution of *tert*-butyl (E)-3-(5-dimethoxymethyl-pyridin-2-yl)-acrylate (3.86 g, 13.84 mmol) in THF (27 ml). The mixture was stirred at RT for 4 h and then poured into an aqueous solution containing 10% K₂CO₃ and extracted three times with EtOAc. The collected organic phases were dried over Na₂SO₄ and evaporated *in vacuo* to give the title compound as white solid (2.97 g).
Y=92%
¹H NMR (CDCl₃) δ (ppm): 10.13 (s, 1 H), 9.08 (d, 1 H), 8.18 (dd, 1 H), 7.63 (d, 1 H), 7.57 (d, 1 H), 6.99 (d, 1 H), 1.56 (s, 9 H).

### Preparation 4: Ethyl (E)-3-{6-[(E)-2-(Tetrahydro-pyran-2-yloxycarbamoyl)-vinyl]-pyridin-3-yl}-acrylate

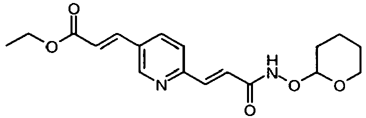

### STEP A: Ethyl (E)-3-[6-((E)-2-tert-butoxycarbonyl-vinyl)-pyridin-3-yl]-acrylate

A solution of triethyl phosphonoacetate (373 mg, 1.66 mmol) and NaH (60% oil dispersion, 71.7 mg, 1.79 mmol) in dry THF (5 ml) was added dropwise to a stirred solution of *tert*-butyl (E)-3-(5-formyl-pyridin-2-yl)-acrylate (prepared as described in Preparation 3, 323 mg, 1.38 mmol) in dry THF (5 ml) under N₂. The resulting mixture was stirred at RT for 4 h and then partitioned between water and Et₂O. The aqueous layer was washed with Et₂O and the collected organic phases were dried over Na₂SO₄ and evaporated *in vacuo* to give the title compound (420 mg). The crude product was used in the next step without any further purification.
Y= quantitative
¹H NMR (CDCl₃) δ (ppm): 8.77 (d, 1 H), 7.86 (dd, 1 H), 7.68 (d, 1 H), 7.60 (d, 1 H), 7.45 (d, 1 H), 6.89 (d, 1 H), 6.54 (d, 1 H), 4.31 (q, 2 H), 1.56 (s, 9 H), 1.37 (t, 3 H).

### STEP B: Ethyl (E)-3-{6-[(E)-2-(Tetrahydro-pyran-2-yloxycarbamoyl)-vinyl]-pyridin-3-yl}-acrylate

A mixture of ethyl (E)-3-[6-((E)-2-tert-butoxycarbonyl-vinyl)-pyridin-3-yl]-acrylate (300 mg, 0.99 mmol) and TFA (4 ml) in DCM (10 ml) was stirred at RT for 3 h. The solvent was removed to dryness and the residue (380 mg) was dissolved in DCM (20 ml) and TEA (1.17 ml, 8.4 mmol). EDC (401 mg, 2.1 mmol), HOBT (283 mg, 2.1 mmol) and NH₂OTHP (184 mg, 1.57 mmol) were added and the mixture was stirred at RT for 4 h. Further NH₂OTHP (40 mg, 0.34 mmol) was added and the mixture was stirred overnight. The solvent was then evaporated and the residue was partitioned between water and EtOAc. The organic layer was dried over Na₂SO₄ and evaporated to dryness. The crude mixture was purified by column chromatography (petroleum ether/EtOAc 4:6) to give the title compound as yellow powder (237 mg).
¹H NMR (DMSO-d₆) δ (ppm): 11.39 (bs, 1 H), 8.92 (d, 1 H), 8.22 (dd, 1 H), 7.27-7.88 (m, 3 H), 7.02 (d, 1 H), 6.82 (d, 1 H), 4.94 (bs, 1 H), 4.21 (q, 2 H), 3.84-4.09 (m, 1 H), 3.46-3.63 (m, 1 H), 1.41-1.86 (m, 6 H), 1.27 (t, 3 H).

### Preparation 5: (E)-3-{6-[(E)-2-(Tetrahydro-pyran-2-yloxycarbamoyl)-vinyl]-pyridin-2-yl}-acrylic acid

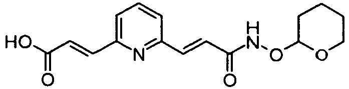

### STEP A: tert-Butyl (E)-3-(6-formyl-pyridin-2-yl)-acrylate

Four batches of 6-bromo-pyridine-2-carbaldehyde (1 g, 5.38 mmol), *tert*-butyl acrylate (2.06 g, 16.1 mmol), 1,4-diaza-bicyclo[2.2.2]octane (25 mg, 0.22 mmol), K₂CO₃ (734 mg, 5.31 mmol), Bu₄NBr (1.73 g, 5.38 mmol) and Pd(OAc)₂ (25 mg, 0.108 mmol) in DMF (10 ml) were heated by MW irradiation in different vials at 120 °C for 5 h. The resulting black coloured mixtures were collected and partitioned between water and Et₂O. The organic phase was washed with water, dried over Na₂SO₄ and evaporated to dryness. The crude mixture was purified by column chromatography (eluent: petroleum ether/EtOAc 95:5) to give the title compound (3.16 g).
Y= 64%

### STEP B: Ethyl (E)-3-[6-((E)-2-tert-butoxycarbonyl-vinyl)-pyridin-2-yl]-acrylate

A solution of triethyl phosphonoacetate (3.34 g, 14.9 mmol) in dry THF (10 ml) was added dropwise under N₂ atmosphere to a stirred suspension of NaH (60% oil dispersion, 705 mg, 17.63 mmol) in dry THF (5 ml). The resulting mixture was stirred at RT for 15 min and then added dropwise -using a syringe equipped with a filtering septum- to a stirred solution of *tert*-butyl (E)-3-(6-formyl-pyridin-2-yl)-acrylate (3.16 g, 13.56 mmol) in dry THF (40 ml) keeping the temperature below 25 °C. The mixture was stirred at RT for 1.5 h, then diluted with EtOAc and washed with a saturated NH₄Cl solution. The aqueous phase was washed twice with EtOAc, the collected organic layers were dried over Na₂SO₄ and then evaporated to dryness. The crude product was purified by column chromatography (eluent:petroleum ether/EtOAc from 95:5 to 9:1) to give the title compound (3.92 g).
Y=95%
¹H NMR (DMSO-d₆) δ (ppm): 7.93 (t, 1 H), 7.76 (d, 1 H), 7.75 (d, 1 H), 7.67 (d, 1 H), 7.58 (d, 1 H), 6.98 (d, 1 H), 6.89 (d, 1 H), 4.23 (q, 2 H), 1.50 (s, 9 H), 1.28 (t, 3 H).

### STEP C: (E)-3-[6-((E)-2-Ethoxycarbonyl-vinyl)-pyridin-2-yl]-acrylic acid

A mixture of ethyl (E)-3-[6-((E)-2-*tert*-butoxycarbonyl-vinyl)-pyridin-2-yl]-acrylate (3.92 g, 12.94 mmol) and TFA (3.99 ml) in DCM (50 ml) was stirred at RT for 24 h and then further TFA (2 ml) was added. After stirring for additional 5 h at 30 °C, the solvent was removed *in vacuo* and the residue was triturated with Et₂O and decanted to give the title compound as its trifluoroacetate salt (4.6 g). Y= 98%
1 H NMR (DMSO-d₆) δ (ppm): 12.58 (bs, 1 H), 7.93 (t, 1 H), 7.76 (d, 1 H), 7.74 (d, 1 H), 7.68 (d, 1 H), 7.61 (d, 1 H), 6.98 (d, 1 H), 6.92 (d, 1 H), 4.23 (q, 2 H), 1.28 (t, 3 H).

### STEP D: Ethyl (E)-3-{6-[(E)-2-(tetrahydro-pyran-2-yloxycarbamoyl)-vinyl]-pyridin-2-yl}-acrylate

(E)-3-[6-((E)-2-Ethoxycarbonyl-vinyl)-pyridin-2-yl]-acrylic acid trifluoroacetate salt (8.49 g, 23.5 mmol) was dissolved in DCM (150 ml) and TEA (3.27 ml, 23.52 mmol). EDC (4.49 g, 23.52 mmol), HOBT (3.18 g, 23.5 mmol) and NH₂OTHP (2.75 g, 23.5 mmol) were added and the resulting mixture was stirred at RT for 5 h. Further EDC (1.35 g, 7.0 mmol), HOBT (952 mg, 7.0 mmol) and NH₂OTHP (819 mg, 7.0 mmol) were added. The solution was stirred at RT overnight and then washed with water and brine. The organic phase was dried over Na₂SO₄ and evaporated to dryness. The crude product was purified by column chromatography (eluent: petroleum ether/EtOAc from 65:35 to 45:55) to give the title compound (6.58 g) as a white powder.
Y= 81%
¹H NMR (DMSO-d₆) δ (ppm): 11.39 (bs, 1 H), 7.91 (t, 1 H), 7.71 (d, 1 H), 7.69 (d, 1 H), 7.61 (d, 1 H), 7.52 (d, 1 H), 7.10 (d, 1 H), 7.00 (d, 1 H), 4.94 (bs, 1 H), 4.23 (q, 2 H), 3.80-4.04 (m, 1 H), 3.41-3.68 (m, 1 H), 1.42-1.87 (m, 6 H), 1.28 (t, 3 H).

### STEP E: (E)-3-{6-[(E)-2-(Tetrahydro-pyran-2-yloxycarbamoyl)-vinyl]-pyridin-2-yl}-acrylic acid

4 M NaOH (11.38 ml) was added dropwise to a stirred solution of ethyl (E)-3-{6-[(E)-2-(tetrahydro-pyran-2-yloxycarbamoyl)-vinyl]-pyridin-2-yl}-acrylate (6.58 g, 19.0 mmol) in THF (200 ml). The mixture was stirred at RT for 2 h and then brought to a pH value of 5 with citric acid (20% aqueous solution). The volume of THF was reduced in *vacuo* and the resulting slurry was acidified to pH=4 with citric acid and extracted with EtOAc and DCM several times. The collected organic layers were dried over Na₂SO₄ and evaporated to dryness. The crude mixture was triturated in EtOAc, THF and petroleum ether to give the title compound (5.7 g) as white powder.
Y= 95 %
¹H NMR (DMSO-d₆) δ (ppm): 12.30 (bs, 1 H), 11.41 (bs, 1 H), 7.90 (t, 1 H), 7.67 (d, 1 H), 7.60 (d, 1 H), 7.61 (d, 1 H), 7.54 (d, 1 H), 7.09 (d, 1 H), 6.93 (d, 1 H), 4.95 (bs, 1 H), 3.84-4.11 (m, 1 H), 3.36-3.74 (m, 1 H), 1.28-2.01 (m, 6 H).

### Preparation 6: (±)-1-Ethyl-2-phenyl-piperazine

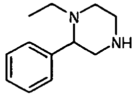

### STEP A: (±)-1-BOC-3-phenyl-piperazine

A mixture of (±)-3-phenyl piperazine (200 mg, 1.23 mmol), BOC₂O (269 mg, 1.23 mmol) and TEA (0.189 ml, 1.35 mmol) in DCM (5 ml) was stirred at RT for 1 h and then partitioned between water and DCM. The organic layer was washed with water, dried over Na₂SO₄ and evaporated *in vacuo.* The crude product was purified by column chromatography (eluent: DCM/MeOH/NH₄OH from 99:1:0.2 to 9:1:0.2) to give the title compound (290 mg).
Y= 90%
¹H NMR (CDCl₃) δ (ppm): 7.39-7.47 (m, 2H), 7.32-7.39 (m, 2H), 7.29-7.34 (m, 1 H), 4.07 (bs, 2H), 3.72 (dd, 1 H), 3.10 (dd, 1 H), 2.91-3.03 (m, 1 H), 2.84-2.95 (m, 1 H), 2.76 (bs, 1 H), 1.49 (s, 9H).

### STEP B: (±)-1-BOC-4-ethyl-3-phenyl-piperazine

NaBH(OAc)₃ (607 mg, 2.86 mmol) was added to a stirred solution of (±)-1-BOC-3-phenyl-piperazine (500 mg, 1.91 mmol) and acetaldehyde (109 mg, 2.48 mmol) in DCM (20 ml). The resulting mixture was stirred at RT for 4 h and then washed with 1 M K₂CO₃. The organic layer was dried over Na₂SO₄ and evaporated *in vacuo.* The crude mixture was purified by column chromatography (eluent: petroleum ether/AcOEt from 9:1 to 8:2) to give the title compound (454 mg).
Y= 82%
¹H NMR (CDCl₃) δ (ppm): 6.75-7.65 (m, 5H), 4.10 (d, 1 H), 3.94 (d, 1 H), 3.15 (dd, 1 H), 2.93-3.11 (m, 2H), 2.70-2.89 (m, 1H), 2.57 (dq, 1 H), 2.20 (td, 1 H), 2.02 (dq, 1 H), 1.47 (s, 9H), 0.94 (t, 3H).

### STEP C: (±)-1-Ethyl-2-phenyl-piperazine

(±)-1-BOC-4-ethyl-3-phenyl-piperazine (454 mg, 1.56 mmol) was dissolved in DCM (10 ml) and treated with Et₂O/HCl at RT for 4 h. 4 M HCl in dioxane (5 ml) was added and the reaction was carried out overnight. The resulting precipitate was filtered off and washed with Et₂O to give the title compound (231 mg) as its hydrochloride salt.
Y= 65 %
¹H NMR (DMSO-d₆ +Na₂CO₃) δ (ppm): 5.88-7.94 (m, 5H), 3.61-4.49 (m, 2H), 2.99-3.16 (m, 1 H), 2.93 (d, 1H), 2.76 (t, 1 H), 2.23-2.48 (m, 2H), 2.02 (t, 1 H), 1.64-1.96 (m, 1H), 0.85 (t, 3H).

### Preparation 7: (±)-1-Methyl-2-phenyl-piperazine

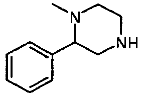

The title compound was prepared as described in Preparation 6 and was obtained as its hydrochloride salt.
¹H NMR (DMSO-d₆ +Na₂CO₃) δ (ppm): 6.46-7.93 (m, 5H), 3.58-4.44 (m, 2H), 2.60-3.08 (m, 3H), 2.33-2.48 (m, 1 H), 1.96-2.17 (m, 1 H), 1.90 (s, 3H).

### Preparation 8: (±)-1-Benzyl-2-phenyl-piperazine

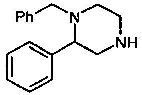

The title compound was prepared as described in Preparation 6 and was obtained as its hydrochloride salt.
¹H NMR (DMSO-d₆) δ (ppm): 7.42-7.56 (m, 2H), 7.08-7.43 (m, 8H), 3.98 (bs, 1H), 3.62 (d, 1H), 2.98-3.26 (m, 1H), 2.84 (d, 1H), 2.52-2.90 (m, 4H), 1.97 (bs, 1H).

### Preparation 7: (±)-1-Acetyl-2-phenyl-piperazine

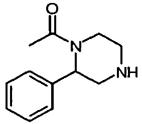

### STEP A: (±)-1-BOC-4-acetyl-3-phenyl-piperazine

Acetyl chloride (0.163 ml, 2.29 mmol) was added to a stirred solution of (±)-1-BOC-3-phenyl-piperazine (prepared as described in Preparation 6 STEP A, 500 mg, 1.91 mmol) and TEA (0.531 ml, 3.82 mmol) in DCM (20 ml). The resulting mixture was stirred for 5 h at RT and then washed with 5% NaHCO₃. The organic phase was dried over Na₂SO₄ and evaporated *in vacuo.* The crude product was purified by column chromatography (eluent: DCM/MeOH/NH₄OH 98:2:0.5) to give the title compound (501 mg).
Y=86%
¹H NMR (DMSO-d₆ 353K) δ (ppm): 7.19-7.46 (m, 5H), 5.40 (bs, 1H), 4.33 (ddd, 1H), 3.97 (d, 1H), 3.61-3.83 (m, 1H), 3.39 (dd, 1H), 3.15 (ddd, 1H), 3.05 (ddd, 1H), 2.07 (s, 3H), 1.36 (s, 9H).

### STEP B: (±)-1-Acetyl-2-phenyl-piperazine

(±)-1-BOC-4-acetyl-3-phenyl-piperazine (490 mg, 1.61 mmol) was dissolved in DCM (10 ml) and 4 M HCl in dioxane (5 ml) was added. The mixture was stirred at RT overnight and the resulting precipitate was filtered off and washed with Et₂O to give the title compound as hydrochloride salt (285 mg).
Y=74%
¹H NMR (DMSO-d₆ +Na₂CO₃ 353K) δ (ppm): 6.49-7.80 (m, 5H), 5.28 (bs, 1H), 3.88 (d, 1H), 3.19-3.54 (m, 1H), 2.54-2.95 (m, 4H), 2.05 (s, 3H).

### Example 1: (E)-N-Hydroxy-3-{4-[(E)-3-(4-methyl-piperazin-1-yl)-3-oxo-propenyl]-phenyl}-acrylamide

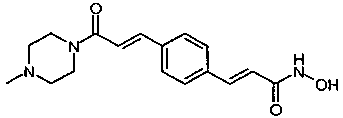

### STEP A: (E)-3-(4-Bromo-phenyl)-1-(4-methyl-piperazin-1-yl-propenone

A mixture of (E)-3-(4-bromo-phenyl)-acrylic acid (500 mg, 2.20 mmol), N-methyl piperazine (330 mg, 3.30 mmol), EDC (840 mg, 4.4 mmol), HOBT (598 mg, 4.4 mmol) and TEA (0.612 ml, 4.4 mmol) in DCM (10 ml) was stirred at RT overnight and then partitioned between water and DCM. The organic extract was washed with 10% Na₂CO₃, dried over Na₂SO₄ and evaporated *in vacuo.* The crude mixture was purified by SCX cartridge (eluent: MeOH and then 3% NH₄OH in MeOH) to give the title compound (630 mg).
Y= 92%

### STEP B: tert-Butyl (E)-3-{4-[(E)-3-(4-methyl-piperazin-1-yl)-3-oxo-propenyl]-phenyl}-acrylate

(E)-3-(4-Bromo-phenyl)-1-(4-methyl-piperazin-1-yl)-propenone (630 mg, 2.04 mmol) was dissolved in DMF (10 ml) and TEA (1.3 ml). NaHCO₃ (440 mg, 5.23 mmol), PPh₃ (26.7 mg, 0.102 mmol), Pd(OAc)₂ (8.96 mg, 0.04 mmol) and *tert*-butyl acrylate (261 mg, 2.04 mmol) were added under N₂ atmosphere. The resulting slurry was stirred at 80 °C under N₂ and further Pd(OAc)₂ (20 mg, 0.09 mmol) was added over 8 h. After stirring at RT overnight, additional *tert*-butyl acrylate (52 mg, 0.40 mmol) and Pd(OAc)₂ (15 mg, 0.067) were added. The mixture was heated to 80 °C for 4 h, then diluted with water and extracted twice with EtOAc. The collected organic phases were washed with water, dried over Na₂SO₄ and evaporated to dryness. The crude product was purified by column chromatography to give the title compound (550 mg).
Y= 77%

### STEP C: (E)-3-{4-[(E)-3-(4-Methyl-piperazin-1-yl)-3-oxo-propenyl]-phenyl}-acrylic acid

A mixture of *tert*-butyl (E)-3-{4-[(E)-3-(4-methyl-piperazin-1-yl)-3-oxo-propenyl]-phenyl}-acrylate (550 mg, 1.54 mmol) and TFA (3 ml) in DCM (20 ml) was stirred at RT for 4 h. The solvent was removed *in vacuo* and the crude mixture was triturated in EtOH and filtered to give the title compound as its trifluoroacetate salt (424 mg).
Y= 66%

### STEP D: (E)-N-Hydroxy-3-{4-[(E)-3-(4-methyl-piperazin-1-yl)-3-oxo-propenyl-phenyl}-acrylamide

(E)-3-{4-[(E)-3-(4-methyl-piperazin-1-yl)-3-oxo-propenyl]-phenyl}-acrylic acid trifluoroacetate (250 mg, 0.60 mmol) was dissolved in DMF (5 ml), THF (5 ml), and TEA (0.167 ml, 1.2 mmol). EDC (229 mg, 1.2 mmol), HOBT (162 mg, 1.2 mmol), and NH₂OTHP (84 mg, 0.72 mmol) were added and the mixture was stirred at RT overnight and then partitioned between water and EtOAc. The organic extract was dried over Na₂SO₄ and evaporated *in vacuo.* The crude product was purified by column chromatography (eluent: DCM/MeOH/NH₄OH 95:5:0.2) and the resulting oil was diluted with DCM and treated with HCl/Et₂O for 1 h. The precipitate was filtered off and washed with DCM to give the title compound as its hydrochloride salt (35 mg).
Y= 18%
LC-MS: Method A, column Atlantis dC18 100x2.1x3µm, rt=2.31
(ES+) MH⁺: 316.1
¹H NMR (DMSO-d₆) δ (ppm): 10.83 (bs, 1 H), 7.77 (d, 2 H), 7.60 (d, 2 H), 7.55 (d, 1 H), 7.46 (d, 1 H), 7.32 (d, 1 H), 6.54 (d, 1 H), 4.53 (d, 2 H), 3.23-3.56 (m, 4 H), 2.88-3.13(m,2H),2.78(d,3H).

### Example 2: (E)-N-Hydroxy-3-{3-[(E)-3-(4-methyl-piperazin-1-yl)-3-oxo-propenyl]-phenyl}-acrylamide

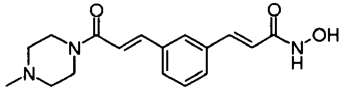

The product was obtained starting from (E)-3-(3-bromo-phenyl)-acrylic acid following the experimental procedure similar to Example 1.

The title compound was freeze dried and obtained as its hydrochloride salt.
LC-MS: Method B, column Acquity UPLC-BEH C18 50x2.1 mmx1.7µm, rt=0.95;
(ES+) MH⁺: 316.33
¹H NMR (DMSO-d₆ +TFA) δ (ppm): 10.30 (bs, 1 H), 7.94 (s, 1 H), 7.72 (d, 1 H), 7.41-7.63 (m, 4 H), 7.34 (d, 1 H), 6.54 (d, 1 H), 4.55 (d, 2 H), 3.48 (d, 3 H), 2.94-3.22 (m, 3 H), 2.82 (s, 3 H).

### Example 3: (E)-N-Hydroxy-3-{3-[(E)-3-oxo-3-(4-phenyl-piperazin-1-yl)-propenyl]-phenyl}-acrylamide

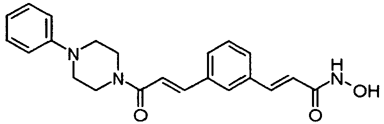

(E)-3-{3-[(E)-2-(Tetrahydro-pyran-2-yloxycarbamoyl)-vinyl]-phenyl}-acrylic acid (obtained as described in Preparation 2, 250 mg, 0.79 mmol) was dissolved in DCM (10 ml) and TEA (0.110 ml, 0.789 mmol). EDC (226 mg, 1.18 mmol), HOBT (159 mg, 1.18 mmol), and N-phenylpiperazine (153 mg, 0.947 mmol) were added and the mixture was stirred overnight at RT. The resulting solution was partitioned between brine and DCM. The aqueous phase was extracted with DCM and the collected organic layers were dried over Na₂SO₄ and evaporated to dryness. The crude product was purified by column chromatography (eluent: DCM/MeOH/NH₄OH from 99:1:0.1 to 98:2:0.2) and the resulting product was dissolved in DCM (5 ml) and treated with HCl/Et₂O for 2 h. The precipitate was filtered off and washed with DCM to give the title compound (238 mg) as its hydrochloride salt.
Y=73%
LC-MS: Method C, column Acquity UPLC-BEH C18 50x2.1mmx1.7µm, rt=1.60;
(ES+) MH⁺: 378.09
¹H NMR (DMSO-d₆ + Na₂CO₃) δ (ppm): 9.81 (bs, 1 H), 7.80-8.04 (m, 1 H), 7.67 (d, 1 H), 7.48-7.60 (m, 1 H), 7.53 (d, 1 H), 7.38 (d, 1 H), 7.31-7.48 (m, 2 H), 7.09-7.30 (m, 2 H), 6.98 (m, 2 H), 6.75-6.86 (m, 1 H), 6.58 (d, 1 H), 3.60-4.11 (m, 4 H), 3.01-3.25 (m, 4 H).

### Example 4: (E)-3-[3-((E)-3-[1,4']Bipiperidinyl-1'-yl-3-oxo-propenyl)-phenyl]-N-hydroxy-acrylamide

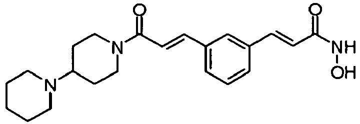

The product was prepared starting from (E)-3-{3-[(E)-2-(tetrahydro-pyran-2-yloxycarbamoyl)-vinyl]-phenyl}-acrylic acid (obtained as described in Preparation 2) and 4-(piperidin-1-yl)-piperidine following the synthetic procedure similar to Example 3. The title compound was purified by preparative LC-MS and was obtained as its trifluoroacetate salt.
LC-MS: Method B, column Acquity UPLC-BEH C18 50x2.1mmx1.7µm, rt=1.16;
(ES+) MH⁺: 384.23
¹H NMR (DMSO-d₆ + TFA) δ (ppm): 8.94 (bs, 1 H), 7.81-7.90 (m, 1 H), 7.66 (dt, 1 H), 7.54-7.60 (m, 1 H), 7.47-7.54 (m, 2 H), 7.44 (dd, 1 H), 7.24 (d, 1 H), 6.54-6.67 (m, 1 H), 4.47-4.65 (m, 2 H), 3.33-3.53 (m, 3 H), 2.84-3.10 (m, 4 H), 2.04-2.24 (m, 2 H), 1.81-1.97 (m, 2 H), 1.54-1.81 (m, 5 H), 1.35-1.54 (m, 1 H).

### Example 5: (E)-N-Hydroxy-3-{3-[(E)-3-oxo-3-(cis-3,4,5-trimethyl-piperazin-1-yl)-propenyl]-phenyl}-acrylamide

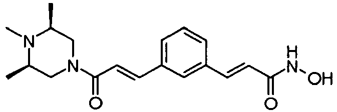

A mixture of (E)-3-{3-[(E)-2-(tetrahydro-pyran-2-yloxycarbamoyl)-vinyl]-phenyl}-acrylic acid (obtained as described in Preparation 2, 200 mg, 0.63 mmol), EDC (240 mg, 1.26 mmol), HOBT (170 mg, 1.26 mmol), TEA (0.354 ml, 2.52 mmol) and cis-3,4,5-trimethyl-piperazine bis hydrochloride (230 mg, 1.13 mmol) in DCM (6 ml) and DMF (1 ml) was stirred overnight at RT. The solvent was removed *in vacuo* and the residue was partitioned between water and EtOAc. The aqueous phase was extracted with DCM and the collected organic layers were dried over Na₂SO₄ and evaporated to dryness. The crude mixture was purified by column chromatography (eluent: DCM/MeOH/NH₄OH 95:5:0.1). The resulting product was dissolved in DCM (10 ml) and treated with HCl/Et₂O for 3 h. The precipitate was filtered off and crystallized from *i*-PrOH to give the title compound (62 mg) as its hydrochloride salt.
Y=26%
LC-MS: Method B, column Acquity UPLC-BEH C18 50x2.1mmx1.7µm, rt=1.09;
(ES+) MH⁺: 344.17
¹H NMR (DMSO-d₆ 353 +TFA) δ (ppm): 7.86 (s, 1 H), 7.69 (d, 1 H), 7.38-7.64 (m, 4 H), 7.26 (d, 1 H), 6.64 (d, 1 H), 4.35-4.62 (m, 2 H), 3.17-3.49 (m, 4 H), 2.80 (bs, 3 H), 1.43 (d, 6 H).

### Example 6: (E)-3-{3-[(E)-3-((1S,4S)-5-Methyl-2,5-diaza-bicyclo[2.2.1]hept-2-yl)-3-oxo-propenyl]-phenyl}-N-hydroxy-acrylamide

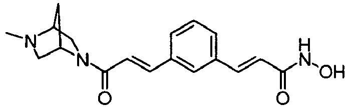

The product was obtained starting from (E)-3-{3-[(E)-2-(tetrahydro-pyran-2-yloxycarbamoyl)-vinyl]-phenyl}-acrylic acid (obtained as described in Preparation 2) and (1S,4S)-5-methyl-2,5-diaza-bicyclo[2.2.1]heptane bis hydrobromide, following the synthetic procedure similar to Example 5. No DMF was used as solvent. The title compound was purified by preparative LC-MS and was obtained as its trifluoroacetate salt.
LC-MS: Method C, column Acquity UPLC-BEH C18 50x2.1mmx1.7µm, rt=0.91;
(ES+) MH⁺: 328.24
1 H NMR (DMSO-d₆ 353K +TFA) δ (ppm): 7.78-7.91 (m, 1 H), 7.68 (d, 1 H), 7.59 (d, 1 H), 7.56 (d, 1 H), 7.50 (d, 1 H), 7.46 (t, 1 H), 7.00 (bs, 1 H), 6.61 (d, 1 H), 4.42 (bs, 1 H), 3.15-4.16 (m, 5 H), 2.93 (s, 3 H), 2.21-2.40 (m, 1 H), 1.97-2.21 (m, 1 H).

### Example 7: (E)-N-Hydroxy-3-{4-[(E)-3-oxo-3-(4-phenyl-piperazin-1-yl)-propenyl]-phenyl}-acrylamide

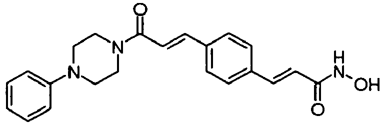

The product was obtained starting from (E)-3-{4-[(E)-2-(tetrahydro-pyran-2-yloxycarbamoyl)-vinyl]-phenyl}-acrylic acid (obtained as described in Preparation 1) and N-phenylpiperazine, following the synthetic procedure similar to Example 5. The title compound was obtained as its hydrochloride salt.
LC-MS: Method B, column Acquity UPLC-BEH C18 50x2.1mmx1.7µm, rt=1.56;
(ES+) MH⁺: 378.24
¹H NMR (DMSO-d₆) δ (ppm): 7.78 (m, 2 H), 7.60 (m, 2 H), 7.50-7.57 (m, 1 H), 7.46 (d, 1 H), 7.37 (d, 1 H), 7.27-7.34 (m, 2 H), 7.10-7.23 (m, 2 H), 6.87-7.05 (m, 1 H), 6.57 (d, 1 H), 3.85 (bs, 4 H), 3.27 (bs, 4 H).

### Example 8: (E)-3-[4-((E)-3-[1,4']Bipiperidinyl-1'-yl-3-oxo-propenyl)-phenyl]-N-hydroxy-acrylamide

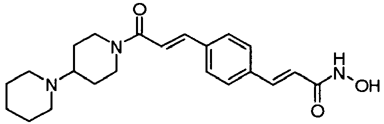

The product was obtained starting from (E)-3-{4-[(E)-2-(tetrahydro-pyran-2-yloxycarbamoyl)-vinyl]-phenyl}-acrylic acid (obtained as described in Preparation 1) and 4-(piperidin-1-yl)-piperidine, following a synthetic procedure similar to Example 5. The title compound was crystallized from *i*-PrOH and was obtained as its hydrochloride salt.
LC-MS: Method B, column Acquity UPLC-BEH C18 50x2.1mmx1.7µm, rt=1.03;
(ES+) MH⁺: 384.30
¹H NMR (DMSO-d₆) δ (ppm): 10.78 (s, 1 H), 10.23 (bs, 1 H), 7.77 (m, 2 H), 7.59 (m, 2 H), 7.50 (d, 1 H), 7.47 (d, 1 H), 7.24-7.40 (m, 1 H), 6.54 (d, 1 H), 4.38-4.70 (m, 2 H), 3.27-3.46 (m, 4 H), 3.00-3.22 (m, 1 H), 2.80-3.00 (m, 2 H), 2.56-2.80 (m, 1 H), 2.05-2.26 (m, 2 H), 1.76-1.88 (m, 3 H), 1.54-1.74 (m, 3 H), 1.35-1.48 (m, 1 H).

### Example 9: (E)-N-Hydroxy-3-{4-[(E)-3-oxo-3-(cis-3,4,5-trimethyl-piperazin-1-yl)-propenyl]-phenyl}-acrylamide

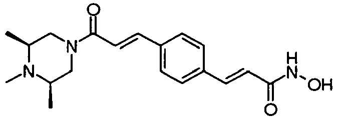

The product was obtained starting from (E)-3-{4-[(E)-2-(tetrahydro-pyran-2-yloxycarbamoyl)-vinyl]-phenyl}-acrylic acid (obtained as described in Preparation 1) and *cis*-3,4,5-trimethyl-piperazine, following a synthetic procedure similar to Example 5. The title compound was obtained as its hydrochloride salt.
LC-MS: Method B, column Acquity UPLC-BEH C18 50x2.1mmx1.7µm, rt=0.94;
(ES+) MH⁺: 344.29
¹H NMR (DMSO-d₆ + TFA 373 K) δ (ppm): 7.71 (m, 2 H), 7.58 (m, 2 H), 7.55 (d, 1 H), 7.48 (d, 1 H), 7.21 (d, 1 H), 6.61 (d, 1 H), 4.34-4.54 (m, 2 H), 3.12-3.47 (m, 4 H), 2.79 (s, 3 H), 1.43 (d, 6 H).

### Example 10: (E)-N-Hydroxy-3-{4-[(E)-3-oxo-3-((1S,4S)-5-methyl-2,5-diaza-bicyclo[2.2.1]hept-2-yl)-propenyl]-phenyl}-acrylamide

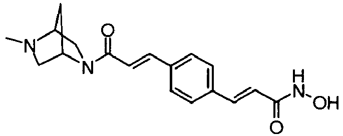

The product was obtained starting from (E)-3-{4-[(E)-2-(tetrahydro-pyran-2-yloxycarbamoyl)-vinyl]-phenyl}-acrylic acid (obtained as described in Preparation 1) and (1S,4S)-5-methyl-2,5-diaza-bicyclo[2.2.1]heptane bis hydrobromide, following a synthetic procedure similar to Example 5. The title compound was purified by preparative LC-MS and was obtained as its trifluoroacetate salt.
LC-MS: Method A, column Acquity UPLC-BEH C18 50x2.1mmx1.7µm, rt=0.75;
(ES+) MH⁺: 328.13
¹H NMR (DMSO-d₆ 373 K + TFA) δ (ppm): 7.71 (m, 2 H), 7.59 (m, 2 H), 7.55 (d, 1 H), 7.48 (d, 1 H), 6.82-7.04 (m, 1 H), 6.60 (d, 1 H), 4.89-5.22 (m, 1 H), 4.42 (s, 1 H), 3.63-3.94 (m, 2 H), 3.22-3.63 (m, 2 H), 2.93 (s, 3 H), 2.22-2.37 (m, 1 H), 2.07-2.21 (m, 1 H).

### Example 11: (E)-N-Hydroxy-3-{5-[(E)-3-oxo-3-(4-phenyl-piperazin-1-yl)-propenyl]-pyridin-2-yl}-acrylamide

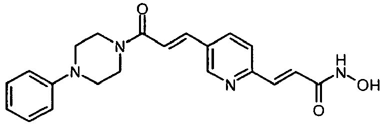

1 M LiOH (1.68 ml) was added to a stirred solution of ethyl (E)-3-{6-[(E)-2-(tetrahydropyran-2-yloxycarbamoyl)-vinyl]-pyridin-3-yl}-acrylate (prepared as described in Preparation 4, 292 mg, 0.84 mmol) in THF (8 ml) and the resulting mixture was stirred at RT. Further 1 M LiOH (1.26 ml) was added over 20 h. The solution was then brought to pH=6 by slow addition of a 4 M HCl solution and then brought to basic pH value by adding NH₄OH. The solvent was concentrated and the residue was freeze-dried. The crude (E)-3-{6-[(E)-2-(tetrahydro-pyran-2-yloxycarbamoyl)-vinyl]-pyridin-3-yl}-acrylic acid was dissolved in DCM (8 ml), DMF (8 ml) and TEA (0.234 ml, 1.68 mmol). EDC (322 mg, 1.68 mmol), HOBT (252 mg, 1.68 mmol) and 1-phenyl-piperazine (164 mg, 1.01 mmol) were added and the resulting slurry was stirred at RT overnight. Further 1-phenyl-piperazine (136 mg, 0.84 mmol) was added and the mixture was heated until dissolution of the slurry. After stirring for additional 6 h at RT the solution was partitioned between water and EtOAc and the resulting precipitate was filtered off and washed with EtOAc and DCM. The obtained solid was suspended in DCM and treated with HCl/Et₂O for 4 h. The resulting precipitate was filtered off and washed with DCM to give the title compound as its hydrochloride salt (102 mg).
Y=30%
LC-MS: Method E, column Synergi 20x2.0mmx2.5µm, rt=1.54;
(ES+) MH⁺: 379.49
¹H NMR (DMSO-d₆) δ (ppm): 8.96 (d, 1 H), 8.34 (dd, 1 H), 7.73 (d, 1 H), 7.44-7.67 (m, 3 H), 7.27-7.41 (m, 2 H), 7.10-7.25 (m, 2 H), 7.01 (d, 1 H), 6.92-7.02 (m, 1 H), 3.67-4.15 (m, 4 H), 3.30 (bs, 4 H).

### Example 12: (E)-N-Hydroxy-3-{5-[(E)-3-(4-methyl-piperazin-1-yl)-3-oxo-propenyl]-pyridin-2-yl}-acrylamide

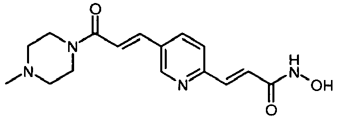

The product was obtained starting from ethyl (E)-3-{6-[(E)-2-(tetrahydro-pyran-2-yloxycarbamoyl)-vinyl]-pyridin-3-yl}-acrylate (prepared as described in Preparation 4) following a synthetic procedure similar to Example 11. The title compound was triturated in MeOH and obtained as its hydrochloride salt.
LC-MS: Method D, column Acquity UPLC-BEH C18 50x2.1mmx1.7µm, rt=0.55; (ES+)
MH⁺: 317.20
¹H NMR (DMSO-d₆) δ (ppm): 10.77 (s, 1 H), 8.93 (d, 1 H), 8.26 (dd, 1 H), 7.68 (d, 1 H), 7.60 (d, 1 H), 7.39-7.54 (m, 2 H), 6.99 (d, 1 H), 4.42-4.63 (m, 2 H), 3.37-3.62 (m, 3 H), 2.94-3.20 (m, 3 H), 2.79 (d, 3 H).

### Example 13: (E)-N-Hydroxy-3-{6-[(E)-3-oxo-3-(4-phenyl-piperazin-1-yl)-propenyl]-pyridin-2-yl}-acrylamide

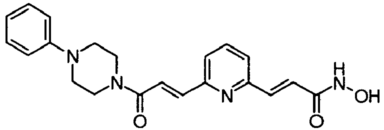

### STEP A: Ethyl (E)-3-{6-[(E)-3-oxo-3-(4-phenyl-piperazin-1-yl)-propenyl]-pyridin-2-yl}-acrylate

A mixture of ethyl (E)-3-[6-((E)-2-tert-butoxycarbonyl-vinyl)-pyridin-2-yl]-acrylate (prepared as described in Preparation 5 STEP A-B, 240 mg, 0.79 mmol) and TFA (0.6 ml) in DCM (2 ml) was stirred at RT for 3 h. The solvent was evaporated *in vacuo* and the resulting oil (190 mg) was dissolved in DMF (2 ml). EDC (177 mg, 0.92 mmol), HOBT (125 mg, 0.92 mmol) and 1-phenylpiperazine (149 mg, 0.92 mmol) were added and the resulting mixture was stirred at RT overnight and then partitioned between an aqueous 5 % NaHCO₃ solution and DCM. The organic phase was dried over Na₂SO₄ and evaporated to dryness. The residue was triturated with petroleum ether and filtered off to give the title compound (390 mg). The crude product was used in the next step without any further purification.

### STEP B: (E)-3-{6-[(E)-3-oxo-3-(4-phenyl-piperazin-1-yl)-propenyl]-pyridin-2-yl}-acrylic acid

Ethyl (E)-3-{6-[(E)-3-oxo-3-(4-phenyl-piperazin-1-yl)-propenyl]-pyridin-2-yl}-acrylate (obtained as in step A, 379 mg) was dissolved in EtOH (5 ml) and the resulting solution was added to a stirred mixture of KOH (163 mg, 2.9 mmol) in EtOH (5 ml). After stirring the mixture at RT for 2 h, the solvent was concentrated *in vacuo* and the residue was partitioned between water and EtOAc. The aqueous phase was acidified with concentrated HCl and extracted with EtOAc. The organic layer was dried over Na₂SO₄ and evaporated to dryness. The crude compound was triturated with EtOAc to give the title compound (168 mg).

### STEP C: (E)-N-Hydroxy-3-{6-[(E)-3-oxo-3-(4-phenyl-piperazin-1-yl)-propenyl]-pyridin-2-yl}-acrylamide

A mixture of (E)-3-{6-[(E)-3-oxo-3-(4-phenyl-piperazin-1-yl)-propenyl]-pyridin-2-yl}-acrylic acid (160 mg, 0.44 mmol), EDC (102 mg, 0.53 mmol), HOBT (72 mg, 0.53 mmol) and NH₂OTHP (62 mg, 0.53 mmol) in DMF (2 ml) was stirred at RT overnight. The solvent was removed *in vacuo* and the crude mixture was purified by column chromatography (petroleum ether/EtOAc/TEA 1:1:0.1). The collected fractions were evaporated and the resulting compound was dissolved in DCM (4 ml) and treated with HCl/Et₂O for 3 h at RT. The resulting precipitate was filtered off and rinsed with DCM to give the title compound as its hydrochloride salt (89 mg).
Y=49%
LC-MS: Method E, column Synergi 20x2.0mmx2.5µm, rt=1.61;
(ES+) MH⁺: 379.46
¹H NMR (DMSO-d₆) δ (ppm): 7.93 (t, 1 H), 7.80 (d, 1 H), 7.66 (d, 1 H), 7.60 (d, 1 H), 7.55 (d, 1 H), 7.51 (d, 1 H), 7.32 (t, 2 H), 7.18 (d, 2 H), 7.04 (d, 1 H), 6.93-7.01 (m, 1 H), 3.67-4.06 (m, 4 H), 3.31 (bs, 4 H).

### Example 14: (E)-N-Hydroxy-3-{6-[(E)-3-(4-methyl-piperazin-1-yl)-3-oxo-propenyl]-pyridin-2-yl}-acrylamide

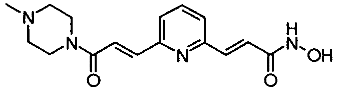

### STEP A: (E)-3-{6-[(E)-3-(4-methyl-piperazin-1-yl)-3-oxo-propenyl]-pyridin-2-yl}-acrylic acid

Ethyl (E)-3-{6-[(E)-3-(4-methyl-piperazin-1-yl)-3-oxo-propenyl]-pyridin-2-yl}-acrylate (prepared following the synthetic procedure similar to Example 13, STEP A, 244 mg, 0.74 mmol) was dissolved in EtOH (2 ml) and the resulting solution was added to a stirred mixture of KOH (125 mg, 2.22 mmol) in EtOH (1 ml). After 2 h at RT, the solvent was concentrated *in vacuo* and the residue was partitioned between water and EtOAc. The aqueous phase was acidified with saturated citric acid solution, washed with EtOAc and freeze-dried. The residue was charged on SCX cartridge and after washing with MeOH the title compound (200 mg) was eluted with 3% NH₄OH in MeOH.
Y=90%

### STEP B: (E)-N-Hydroxy-3-{6-[(E)-3-(4-methyl-piperazin-1-yl)-3-oxo-propenyl]-pyridin-2-yl}-acrylamide

A mixture of (E)-3-{6-[(E)-3-(4-methyl-piperazin-1-yl)-3-oxo-propenyl]-pyridin-2-yl}-acrylic acid (193 mg, 0.64 mmol), EDC (148 mg, 0.77 mmol), HOBT (104 mg, 0.77 mmol), TEA (0.089 ml, 0.64 mmol) and NH₂OTHP (90 mg, 0.77 mmol) in DCM (2 ml) was stirred at RT for 5 h and then partitioned between a saturated NaHCO₃ solution and DCM. The organic phase was dried over Na₂SO₄ and evaporated *in vacuo.* The crude mixture was purified by column chromatography (DCM/MeOH/NH₄OH 98:2:0.2) and the resulting compound was dissolved in DCM (5 ml) and treated with HCl/Et₂O for 1.5 h at RT. The resulting precipitate was filtered off and purified by preparative LC-MS to give the title compound (60 mg) as its bis trifluoroacetate salt.
Y= 17%
LC-MS: Method F, column Acquity UPLC-BEH C18 50x2.1mmx1.7µm, rt=0.81;
(ES+) MH⁺:317.22
¹H NMR (DMSO-d₆ +TFA) δ (ppm): 10.68 (bs, 1 H), 7.91 (t, 1 H), 7.77 (d, 1 H), 7.60 (d, 1 H), 7.57-7.61 (m, 1 H), 7.54 (d, 1 H), 7.50 (d, 1 H), 7.03 (d, 1 H), 4.32-4.69 (m, 2 H), 3.38-3.72 (m, 3 H), 2.94-3.27 (m, 3 H), 2.81 (s, 3 H).

### Example 15: (E)-3-(6-{(E)-3-[4-(3-Chloro-phenyl)-piperazin-1-yl]-3-oxo-propenyl}-pyridin-2-yl)-N-hydroxy-acrylamide

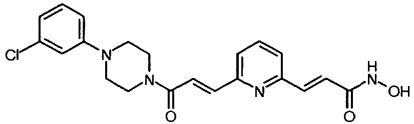

1-(3-Chloro-phenyl)-piperazine (51.7 mg, 0.264 mmol) was added to a stirred solution of (E)-3-{6-[(E)-2-(tetrahydro-pyran-2-yloxycarbamoyl)-vinyl]-pyridin-2-yl}-acrylic acid (obtained as described in preparation 5, 70 mg, 0.22 mmol), TEA (0.061 ml, 0.44 mmol), EDC (63 mg, 0.33 mmol) and HOBT (44.5 mg, 0.33 mmol) in DCM (3 ml). The mixture was stirred at RT for 8 h and then washed with 1 M K₂CO₃. The layers were separated by a phase separator cartridge and the organic layer was shaken overnight in presence of PS-isocyanate (240 mg, loading: 1.58 mmol/g). The resin was filtered off and the solvent was evaporated *in vacuo.* The crude product was purified using a SiO₂ cartridge (eluent: DCM/MeOH/NH₄OH to 98:2:0.1 to 95:5:0.2) and the resulting product was dissolved in DCM and treated with HCl/Et₂O for 5 h. The precipitate was filtered off to give the title compound as its hydrochloride salt (60 mg).
Y=60%
LC-MS: Method C, column Acquity UPLC-BEH C18 50x2.1mmx1.7µm, rt=1.86;
(ES+) MH⁺: 413.18
¹H NMR (DMSO-d₆) δ (ppm): 7.94 (t, 1 H), 7.82 (d, 1 H), 7.66 (d, 1 H), 7.61 (d, 1 H), 7.55 (d, 1 H), 7.52 (d, 1 H), 7.25 (t, 1 H), 6.93-7.12 (m, 3 H), 6.84 (dt, 1 H), 3.61-3.99 (m, 4 H), 3.27 (bs, 4 H).

The following compounds were prepared following the experimental procedure for the preparation of Example 15, starting from (E)-3-{6-[(E)-2-(tetrahydro-pyran-2-yloxycarbamoyl)-vinyl]-pyridin-2-yl}-acrylic acid (obtained as described in preparation 5) and the corresponding amine.

### Example 16: (E)-3-{6-((E)-3-(4-Benzoyl-piperazin-1-yl)-3-oxo-propenyl]-pyridin-2-yl}-N-hydroxy-acrylamide hydrochloride

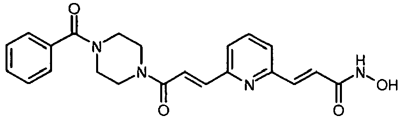

LC-MS: Method C, column Acquity UPLC-BEH C18 50x2.1mmx1.7µm, rt=1.27;
(ES+) MH⁺: 407.19
¹H NMR (DMSO-d₆ +TFA) δ (ppm): 7.90 (t, 1 H), 7.75 (d, 1 H), 7.35-7.68 (m, 9 H), 7.00 (d, 1 H), 3.21-4.11 (m, 8 H).

### Example 17: (E)-3-{6-[(E)-3-(4-Benzyl-piperidin-1-yl)-3-oxo-propenyl]-pyridin-2-yl}-N-hydroxy-acrylamide hydrochloride

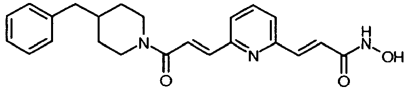

LC-MS: Method C, column Acquity UPLC-BEH C18 50x2.1mmx1.7µm, rt=1.92;
(ES+) MH⁺: 392.24
¹H NMR (DMSO-d₆) δ (ppm): 7.94 (t, 1 H), 7.80 (d, 1 H), 7.57-7.67 (m, 1 H), 7.62 (d, 1 H), 7.52 (d, 1 H), 7.49 (d, 1 H), 7.13-7.36 (m, 5 H), 7.03 (d, 1 H), 4.01-4.62 (m, 2 H), 2.58-3.21 (m, 2 H), 2.54 (d, 2 H), 1.74-1.98 (m, 1 H), 1.50-1.74 (m, 2 H), 0.86-1.30 (m, 2 H).

### Example 18: (E)-3-(6-{(E)-3-[4-(2-Chloro-phenyl)-piperazin-1-yl]-3-oxo-propenyl}-pyridin-2-yl)-N-hydroxy-acrylamide hydrochloride

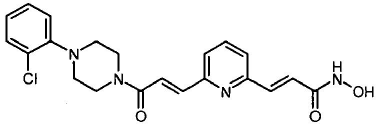

LC-MS: Method C, column Acquity UPLC-BEH C18 50x2.1mmx1.7µm, rt=1.89;
(ES+) MH⁺: 413.25
¹H NMR (DMSO-d₆) δ (ppm): 7.93 (t, 1 H), 7.80 (d, 1 H), 7.66 (d, 1 H), 7.60 (d, 1 H), 7.55 (d, 1 H), 7.52 (d, 1 H), 7.44 (dd, 1 H), 7.28-7.37 (m, 1 H), 7.18 (dd, 1 H), 7.04-7.12 (m, 1 H), 7.05 (d, 1 H), 3.62-4.09 (m, 4 H), 3.03 (bs, 4 H).

### Example 19: (E)-N-Hydroxy-3-{6-[(E)-3-oxo-3-(4-phenyl-piperidin-1-yl)-propenyl]-pyridin-2-yl}-acrylamide hydrochloride

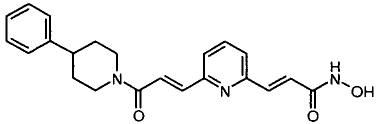

LC-MS: Method C, column Acquity UPLC-BEH C18 50x2.1mmx1.7µm, rt=1.81;
(ES+) MH⁺: 378.30
¹H NMR (DMSO-d₆) δ (ppm): 7.90 (t, 1 H), 7.77 (d, 1 H), 7.63 (d, 1 H), 7.57 (d, 1 H), 7.51 (d, 1 H), 7.50 (d, 1 H), 7.14-7.38 (m, 5 H), 7.01 (d, 1 H), 4.54-4.83 (m, 1 H), 4.21-4.46 (m, 1 H), 3.05-3.40 (m, 1 H), 2.67-2.96 (m, 2 H), 1.77-2.05 (m, 2 H), 1.41-1.74 (m, 2 H).

### Example 20: (E)-N-Hydroxy-3-{6-[(E)-3-oxo-3-(4-pyrimidin-2-yl-piperazin-1-yl)-propenyl]-pyridin-2-yl}-acrylamide hydrochloride

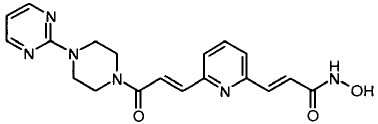

LC-MS: Method C, column Acquity UPLC-BEH C18 50x2.1mmx1.7µm, rt=1.08;
(ES+) MH⁺: 381.16
¹H NMR (DMSO-d₆) δ (ppm): 10.35 (bs, 1 H), 8.44 (d, 2 H), 7.95 (t, 1 H), 7.82 (d, 1 H), 7.67 (d, 1 H), 7.62 (d, 1 H), 7.56 (d, 1 H), 7.53 (d, 1 H), 7.07 (d, 1 H), 6.73 (t, 1 H), 3.56-4.07 (m, 8 H).

### Example 21: (E)-3-(6-{(E)-3-[4-(4-Chloro-phenyl)-piperazin-1-yl]-3-oxo-propenyl}-pyridin-2-yl)-N-hydroxy-acrylamide hydrochloride

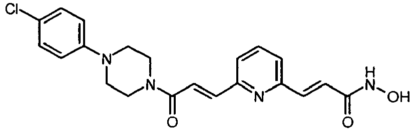

LC-MS: Method C, column Acquity UPLC-BEH C18 50x2.1mmx1.7µm, rt=1.85;
(ES+) MH⁺: 413.32
¹H NMR (DMSO-d₆) δ (ppm): 7.94 (t, 1 H), 7.82 (d, 1 H), 7.66 (d, 1 H), 7.61 (d, 1 H), 7.55 (d, 1 H), 7.52 (d, 1 H), 7.29 (m, 2 H), 7.06 (m, 2 H), 7.05 (d, 1 H), 3.54-4.13 (m, 4 H), 3.24 (bs, 4 H).

### Example 22: (E)-3-{6-[(E)-3-(4-Benzyl-piperazin-1-yl)-3-oxo-propenyl]-pyridin-2-yl}-N-hydroxy-acrylamide hydrochloride

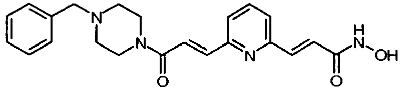

LC-MS: Method C, column Acquity UPLC-BEH C18 50x2.1mmx1.7µm, rt=1.06;
(ES+) MH⁺: 393.38
¹H NMR (300 MHz, DMSO-d₆) ppm 11.47 (bs, 1 H), 7.91 (t, 1 H), 7.76 (d, 1 H), 7.54 (d, 1 H), 7.40-7.67 (m, 8 H), 7.06 (d, 1 H), 4.41-4.67 (m, 2 H), 4.35 (bs, 2 H), 3.55-3.85 (m, 1 H), 2.84-3.50 (m, 5 H)

### Example 23: (E)-N-Hydroxy-3-{6-[(E)-3-oxo-3-(4-phenethyl-piperazin-1-yl)-propenyl]-pyridin-2-yl}-acrylamide hydrochloride

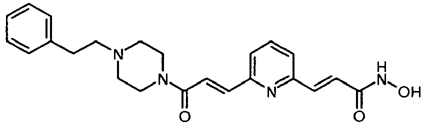

LC-MS: Method C, column Acquity UPLC-BEH C18 50x2.1mmx1.7µm, rt=1.20;
(ES+) MH⁺: 407.32
¹H NMR (DMSO-d₆ +TFA) δ (ppm): 11.39 (bs, 1 H), 7.92 (t, 1 H), 7.80 (d, 1 H), 7.63 (d, 1 H), 7.55-7.64 (m, 2 H), 7.51 (d, 1 H), 7.22-7.40 (m, 5 H), 7.07 (d, 1 H), 4.27-4.75 (m, 2 H), 3.49-3.88 (m, 3 H), 3.22-3.50 (m, 3 H), 2.94-3.22 (m, 4 H).

### Example 24: (E)-3-{6-[(E)-3-(4-Benzoyl-piperidin-1-yl)-3-oxo-propenyl]-pyridin-2-yl}-N-hydroxy-acrylamide hydrochloride

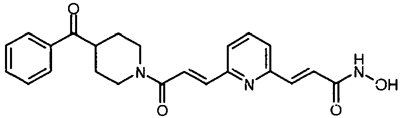

LC-MS: Method C, column Acquity UPLC-BEH C18 50x2.1mmx1.7µm, rt=1.63;
(ES+) MH⁺: 406.31
¹H NMR (DMSO-d₆) δ (ppm): 7.98-8.08 (m, 2 H), 7.93 (t, 1 H), 7.79 (d, 1 H), 7.51-7.70 (m, 6 H), 7.48 (d, 1 H), 7.03 (d, 1 H), 4.49 (d, 1 H), 4.26 (d, 1 H), 3.80 (tt, 1 H), 3.37 (t, 1 H), 2.96 (t, 1 H), 1.79-2.01 (m, 2 H), 1.35-1.68 (m, 2 H).

### Example 25: (±)-(E)-N-Hydroxy-3-{6-[(E)-3-oxo-3-(3-phenyl-piperidin-1-yl)-propenyl]-pyridin-2-yl}-acrylamide hydrochloride

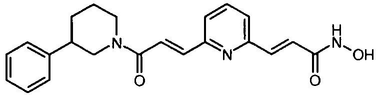

LC-MS: Method C, column Acquity UPLC-BEH C18 50x2.1mmx1.7µm, rt=1.83;
(ES+) MH⁺: 378.30
¹H NMR (DMSO-d₆ 353K+TFA) δ (ppm): 7.86 (t, 1 H), 7.70 (d, 1 H), 7.55 (d, 1 H), 7.48-7.54 (m, 1 H), 7.48 (d, 1 H), 7.46 (d, 1 H), 7.27-7.38 (m, 4 H), 7.16-7.28 (m, 1 H), 7.01 (d, 1 H), 4.12-4.45 (m, 2 H), 3.04 (bs, 1 H), 2.62-2.82 (m, 2 H), 1.92-2.13 (m, 1 H), 1.69-1.92 (m, 2 H), 1.40-1.69 (m, 1 H).

### Example 26: (E)-N-Hydroxy-3-{6-[(E)-3-oxo-3-(4-phenyl-3,6-dihydro-2H-pyridin-1-yl)-propenyl]-pyridin-2-yl}-acrylamide hydrochloride

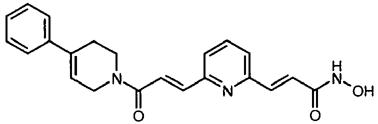

LC-MS: Method C, column Acquity UPLC-BEH C18 50x2.1mmx1.7µm, rt=1.82;
(ES+) MH⁺: 376.35
¹H NMR (DMSO-d₆ +TFA) δ (ppm): 7.92 (t, 1 H), 7.74-7.87 (m, 1 H), 7.69 (d, 1 H), 7.54 (d, 1 H), 7.49 (d, 1 H), 7.42-7.65 (m, 3 H), 7.32-7.41 (m, 2 H), 7.21-7.32 (m, 1 H), 7.04 (d, 1 H), 6.16-6.29 (m, 1 H), 4.34-4.53 (m, 1 H), 4.15-4.34 (m, 1 H), 3.88-4.01 (m, 1 H), 3.69-3.87 (m, 1 H), 2.43-2.69 (m, 2 H).

### Example 27: (E)-3-(6-{(E)-3-[4-(2,6-Dimethyl-phenyl)-piperidin-1-yl]-3-oxo-propenyl}-pyridin-2-yl)-N-hydroxy-acrylamide hydrochloride

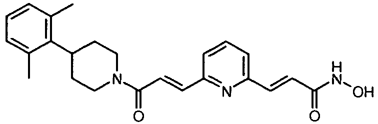

LC-MS: Method C, column Acquity UPLC-BEH C18 50x2.1mmx1.7µm, rt=2.02;
(ES+) MH⁺: 406.31
¹H NMR (DMSO-d₆) δ (ppm): 7.92 (t, 1 H), 7.76-7.85 (m, 1 H), 7.66 (d, 1 H), 7.44-7.62 (m, 3 H), 7.02 (d, 1 H), 6.96 (s, 3 H), 4.47-4.72 (m, 1 H), 4.32-4.42 (m, 1 H), 3.17-3.40 (m, 2 H), 2.71-2.89 (m, 1 H), 2.35 (s, 6 H), 1.89-2.14 (m, 2 H), 1.55-1.78 (m, 2 H).

### Example 28: (E)-3-{6-[(E)-3-(4-Cyano-4-phenyl-piperidin-1-yl)-3-oxo-propenyl]-pyridin-2-yl}-N-hydroxy-acrylamide hydrochloride

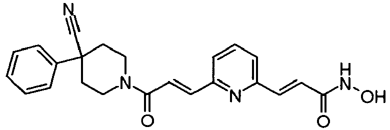

LC-MS: Method C, column Acquity UPLC-BEH C18 50x2.1mmx1.7µm, rt=1.72;
(ES+) MH⁺: 403.28 ¹H NMR (DMSO-d₆ +TFA) δ (ppm): 7.91 (t, 1 H), 7.78 (d, 1 H), 7.64 (d, 1 H), 7.56-7.61 (m, 2 H), 7.56 (d, 1 H), 7.53 (d, 1 H), 7.47-7.61 (m, 1 H), 7.42-7.49 (m, 2 H), 7.34-7.41 (m, 1 H), 7.01 (d, 1 H), 4.58-4.89 (m, 1 H), 4.28-4.58 (m, 1 H), 3.32-3.56 (m, 1 H), 2.84-3.13 (m, 1 H), 1.81-2.36 (m, 4 H).

### Example 29: (E)-N-Hydroxy-3-{6-[(E)-3-oxo-3-(4-pyridin-2-yl-piperazin-1-yl)-propenyl]-pyridin-2-yl}-acrylamide hydrochloride

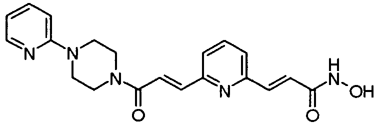

LC-MS: Method C, column Acquity UPLC-BEH C18 50x2.1mmx1.7µm, rt=0.85;
(ES+) MH⁺: 380.33
¹H NMR (DMSO-d₆ +TFA) δ (ppm): 7.99-8.10 (m, 2 H), 7.92 (t, 1 H), 7.74-7.85 (m, 1 H), 7.63 (d, 1 H), 7.52-7.64 (m, 1 H), 7.56 (d, 1 H), 7.51 (d, 1 H), 7.40 (d, 1 H), 7.04 (d, 1 H), 6.93-7.03 (m, 1 H), 3.65-4.27 (m, 8 H).

### Example 30: (E)-N-Hydroxy-3-(6-{(E)-3-oxo-3-[4-(2-oxo-2,3-dihydro-benzoimidazol-1-yl)-piperidin-1-yl]-propenyl}-pyridin-2-yl)-acrylamide hydrochloride

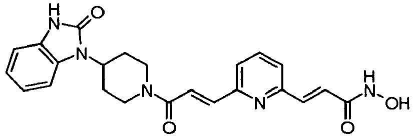

LC-MS: Method C, column Acquity UPLC-BEH C18 50x2.1mmx1.7µm, rt=1.34;
(ES+) MH⁺: 434.31
¹H NMR (DMSO-d₆ +TFA) δ (ppm): 10.84 (s, 1 H), 7.91 (t, 1 H), 7.80 (d, 1 H), 7.66 (d, 1 H), 7.58 (d, 1 H), 7.54 (d, 1 H), 7.51 (d, 1 H), 7.20-7.30 (m, 1 H), 7.02 (d, 1 H), 6.94-7.01 (m, 3 H), 4.59-4.83 (m, 1 H), 4.23-4.57 (m, 2 H), 3.16-3.45 (m, 1 H), 2.76-2.96 (m, 1 H), 2.03-2.43 (m, 2 H), 1.53-1.97 (m, 2 H).

### Example 31: (E)-3-(6-{(E)-3-[4-(2,6-Dimethyl-phenyl)-piperazin-1-yl]-3-oxo-propenyl}-pyridin-2-yl)-N-hydroxy-acrylamide hydrochloride

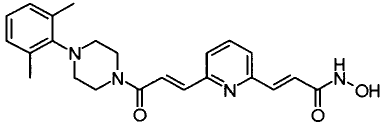

LC-MS: Method C, column Acquity UPLC-BEH C18 50x2.1mmx1.7µm, rt=2.04;
(ES+) MH⁺: 407.31
¹H NMR (DMSO-d₆) δ (ppm): 7.93 (t, 1 H), 7.80 (d, 1 H), 7.66 (d, 1 H), 7.60 (d, 1 H), 7.55 (d, 1 H), 7.51 (d, 1 H), 7.03 (d, 1 H), 6.89-7.00 (m, 3 H), 3.56-3.96 (m, 4 H), 3.06 (bs, 4 H), 2.29 (s, 6 H).

### Example 32: (E)-N-Hydroxy-3-{6-[(E)-3-oxo-3-(4-oxo-1-phenyl-1,3,8-triaza-spiro[4.5]dec-8-yl)-propenyl]-pyridin-2-yl}-acrylamide hydrochloride

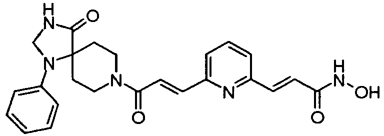

LC-MS: Method C, column Acquity UPLC-BEH C18 50x2.1mmx1.7µm, rt=1.43;
(ES+) MH⁺: 448.32
¹H NMR (DMSO-d₆) δ (ppm): 8.82 (s, 1 H), 7.93 (t, 1 H), 7.82 (d, 1 H), 7.67 (d, 1 H), 7.60 (d, 1 H), 7.57 (d, 1 H), 7.51 (d, 1 H), 7.14-7.31 (m, 2 H), 7.03 (d, 1 H), 6.62-6.84 (m, 3 H), 4.62 (s, 2 H), 4.34-4.52 (m, 1 H), 4.04-4.30 (m, 1 H), 3.74-4.00 (m, 1 H), 3.32-3.61 (m, 1 H), 2.22-2.48 (m, 2 H), 1.47-1.96 (m, 2 H).

### Example 33: (±)-(E)-N-Hydroxy-3-{6-[(E)-3-oxo-3-(3-phenyl-piperazin-1-yl)-propenyl]-pyridin-2-yl}-acrylamide hydrochloride

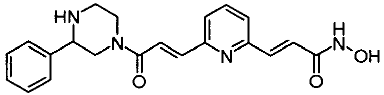

LC-MS: Method C, column Acquity UPLC-BEH C18 50x2.1mmx1.7µm, rt=1.01;
(ES+) MH⁺: 379.30
¹H NMR (DMSO-d₆ 353K +Na₂CO₃) δ (ppm): 10.76 (bs, 1 H), 8.82 (bs, 1 H), 7.86 (t, 1 H), 7.67-7.76 (m, 1 H), 7.56-7.64 (m, 2 H), 7.29-7.57 (m, 7 H), 7.05 (d, 1 H), 4.29-4.64 (m, 2 H), 4.05 (dd, 1 H), 3.39 (dd, 1 H), 3.21-3.32 (m, 1 H), 2.92-3.03 (m, 2 H).

### Example 34: (E)-N-Hydroxy-3-(6-{(E)-3-[4-(4-methoxy-phenyl)-piperazin-1-yl]-3-oxo-propenyl}-pyridin-2-yl)-acrylamide hydrochloride

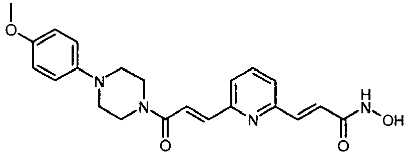

LC-MS: Method C, column Acquity UPLC-BEH C18 50x2.1mmx1.7µm, rt=1.21;
(ES+) MH⁺: 409.13
¹H NMR (DMSO-d₆ +Na₂CO₃) δ (ppm): 9.94 (bs, 1 H), 7.86 (t, 1 H), 7.70 (d, 1 H), 7.62 (d, 1 H), 7.53 (d, 1 H), 7.52 (d, 1 H), 7.41 (d, 1 H), 7.01 (d, 1 H), 6.94 (m, 2 H), 6.85 (m, 2 H), 3.67-3.71 (m, 3 H), 3.53-4.05 (m, 4 H), 2.88-3.19 (m, 4 H).

### Example 35: (E)-N-Hydroxy-3-(6-{(E)-3-oxo-3-[4-(4-trifluoromethyl-phenyl)-iperazin-1-yl]-propenyl}-pyridin-2-yl)-acrylamide hydrochloride

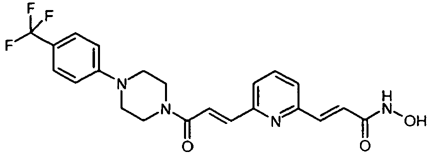

LC-MS: Method C, column Acquity UPLC-BEH C18 50x2.1mmx1.7µm, rt=1.99;
(ES+) MH⁺: 447.04
¹H NMR (DMSO-d₆+Na₂CO₃) δ (ppm): 9.84 (bs, 1 H), 7.84 (t, 1 H), 7.67 (d, 1 H), 7.62 (d, 1 H), 7.46-7.58 (m, 4 H), 7.33 (d, 1 H), 7.10 (m, 2 H), 6.98 (d, 1 H), 3.59-4.15 (m, 4 H), 3.28-3.59 (m, 4 H).

### Example 36: (E)-3-(6-{(E)-3-[4-(4-Cyano-phenyl)-piperazin-1-yl]-3-oxo-propenyl}-pyridin-2-yl)-N-hydroxy-acrylamide hydrochloride

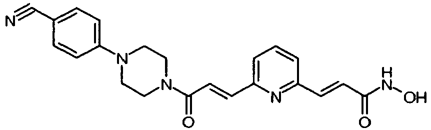

LC-MS: Method C, column Acquity UPLC-BEH C18 50x2.1mmx1.7pm, rt=1.52;
(ES+) MH⁺: 404.08
¹H NMR (DMSO-d₆) δ (ppm): 7.91 (dd, 1 H), 7.79 (d, 1 H), 7.45-7.72 (m, 6 H), 6.94-7.13 (m, 3 H), 3.63-3.95 (m, 4 H), 3.45 (bs, 4 H).

### Example 37: (E)-3-(6-{(E)-3-[4-(4-Fluoro-phenyl)-piperazin-1-yl]-3-oxo-propenyl}-pyridin-2-yl)-N-hydroxy-acrylamide hydrochloride

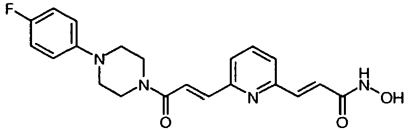

LC-MS: Method C, column Acquity UPLC-BEH C18 50x2.1mmx1.7µm, rt=1.49;
(ES+) MH⁺: 397.06
¹H NMR (DMSO-d₆) δ (ppm): 7.92 (t, 1 H), 7.72-7.86 (m, 1 H), 7.64 (d, 1 H), 7.59 (d, 1 H), 7.54 (d, 1 H), 7.51 (d, 1 H), 7.04 (d, 1 H), 6.83-7.34 (m, 4 H), 3.51-4.28 (m, 4 H), 2.93-3.46 (m, 4 H).

### Example 38: (E)-3-(6-{(E)-3-[4-(4-Bromo-phenyl)-piperazin-1-yl]-3-oxo-propenyl}-pyridin-2-yl)-N-hydroxy-acrylamide hydrochloride

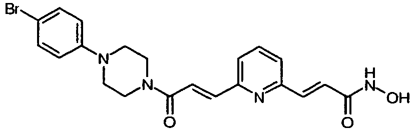

LC-MS: Method C, column Acquity UPLC-BEH C18 50x2.1mmx1.7µm, rt=1.87;
(ES+) MH⁺: 456.94
¹H NMR (DMSO-d₆) δ (ppm): 7.92 (t, 1 H), 7.79 (d, 1 H), 7.64 (d, 1 H), 7.59 (d, 1 H), 7.54 (d, 1 H), 7.51 (d, 1 H), 7.39 (m, 2 H), 7.03 (d, 1 H), 6.97 (m, 2 H), 3.51-4.22 (m, 4 H), 3.03-3.40 (m, 4 H).

### Example 39: (E)-3-(6-{(E)-3-[4-(4-Benzyloxy-phenyl)-piperazin-1-yl]-3-oxo-propenyl}-pyridin-2-yl)-N-hydroxy-acrylamide hydrochloride

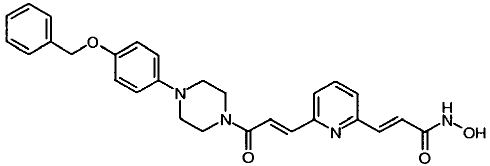

LC-MS: Method C, column Acquity UPLC-BEH C18 50x2.1mmx1.7µm, rt=1.76;
(ES+) MH⁺: 485.08
¹H NMR (DMSO-d₆) δ (ppm): 7.94 (t, 1 H), 7.81 (d, 1 H), 7.67 (d, 1 H), 7.61 (d, 1 H), 7.58 (d, 1 H), 7.52 (d, 1 H), 7.26-7.77 (m, 7 H), 7.13 (m, 2 H), 7.08 (d, 1 H), 5.14 (s, 2 H), 3.81-4.54 (m, 4 H), 3.15-3.81 (m, 4 H).

### Example 40: (E)-N-Hydroxy-3-{6-[(E)-3-oxo-3-(4-pyridin-4-yl-piperazin-1-yl)-propenyl]-pyridin-2-yl}-acrylamide hydrochloride

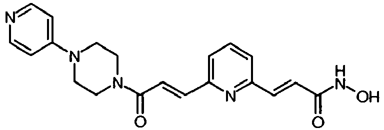

LC-MS: Method C, column Acquity UPLC-BEH C18 50x2.1mmx1.7µm, rt=0.88;
(ES+) MH⁺: 380.11
¹H NMR (DMSO-d₆) δ (ppm): 13.73 (bs, 1 H), 8.16-8.41 (m, 2 H), 7.91 (t, 1 H), 7.78 (d, 1 H), 7.63 (d, 1 H), 7.50 (d, 1 H), 7.58 (s, 2 H), 7.13-7.29 (m, 2 H), 7.05 (d, 1 H), 3.56-4.33 (m, 8 H).

### Example 41: (E)-3-(6-{(E)-3-[5-(4-Chloro-phenyl)-(1S,4S)-2,5-diaza-bicyclo[2.2.1]hept-2-yl]-3-oxo-propenyl}-pyridin-2-yl)-N-hydroxy-acrylamide trifluoroacetate

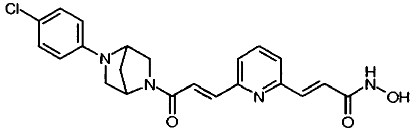

LC-MS: Method F, column Acquity UPLC-BEH C18 50x2.1mmx1.7µm, rt=2.69;
(ES+) MH⁺: 425.01
¹H NMR (DMSO-d₆) δ (ppm): 10.90 (bs, 1 H), 7.62-7.81 (m, 1 H), 7.62-7.98 (m, 2 H), 7.33-7.62 (m, 3 H), 7.17 (m, 2 H), 6.85-7.15 (m, 1 H), 6.65 (m, 2 H), 4.39-5.23 (m, 2 H), 3.29-3.83 (m, 3 H), 3.04 (dd, 1 H), 1.80-2.18 (m, 2 H).

### Example 42: (±)-(E)-N-Hydroxy-3-{6-[(E)-3-oxo-3-(3-o-tolyl-piperidin-1-yl)-propenyl]-pyridin-2-yl}-acrylamide hydrochloride

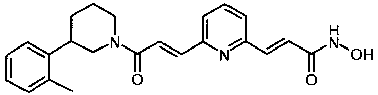

LC-MS: Method C, column Acquity UPLC-BEH C18 50x2.1mmx1.7µm, rt=1.90;
(ES+) MH⁺: 392.16
¹H NMR (DMSO-d₆) δ (ppm): 6.56-8.27 (m, 11 H), 4.41-4.74 (m, 1 H), 3.96-4.41 (m, 1 H), 3.01-3.36 (m, 1 H), 2.56-3.03 (m, 2 H), 2.32 (s, 3 H), 1.69-2.06 (m, 3 H), 1.34-1.69 (m, 1 H).

### Example 43: (±)-(E)-N-Hydroxy-3-{6-[(E)-3-oxo-3-(3-m-tolyl-piperidin-1-yl)-propenyl]-pyridin-2-yl}-acrylamide hydrochloride

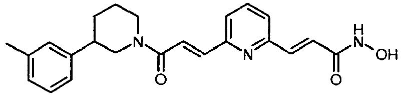

LC-MS: Method C, column Acquity UPLC-BEH C18 50x2.1mmx1.7µm, rt=1.94;
(ES+) MH⁺: 392.16
¹H NMR (DMSO-d₆ +Na₂CO₃) δ (ppm): 9.83 (bs, 1 H), 6.53-8.20 (m, 11 H), 4.39-4.80 (m, 1 H), 4.02-4.40 (m, 1 H), 2.92-3.26 (m, 1 H), 2.53-2.90 (m, 2 H), 2.30 (s, 3 H), 1.65-2.08 (m, 3 H), 1.28-1.66 (m, 1 H).

### Example 44: (±)-(E)-N-Hydroxy-3-{6-[(E)-3-(3-naphthalen-1-yl-piperidin-1-yl)-3-oxo-propenyl]-pyridin-2-yl}-acrylamide hydrochloride

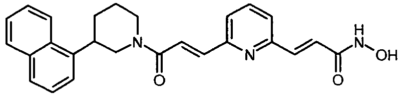

LC-MS: Method F, column Acquity UPLC-BEH C18 50x2.1mmx1.7µm, rt=3.11;
(ES+) MH⁺: 428.02
¹H NMR (DMSO-d₆ 353K) δ (ppm): 8.04-8.41 (m, 1 H), 7.28-8.03 (m, 12 H), 6.78-7.23 (m, 1 H), 4.05-4.94 (m, 2 H), 3.25-3.77 (m, 1 H), 2.79-3.39 (m, 2 H), 1.52-2.27 (m, 4 H).

### Example 45: (E)-N-Hydroxy-3-{6-[(E)-3-oxo-3-(1,3,4,5-tetrahydro-pyrido[4,3-b]indol-2-yl)-propenyl]-pyridin-2-yl}-acrylamide hydrochloride

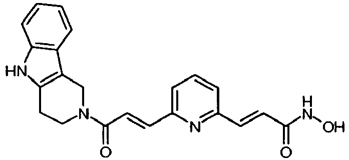

LC-MS: Method C, column Acquity UPLC-BEH C18 50x2.1mmx1.7µm, rt=1.58;
(ES+) MH⁺: 389.11
¹H NMR (DMSO-d₆) δ (ppm): 10.94 (s, 1 H), 7.93 (t, 1 H), 7.84 (d, 1 H), 7.72 (d, 1 H), 7.38-7.66 (m, 4 H), 7.22-7.35 (m, 1 H), 6.79-7.21 (m, 3 H), 4.92 (bs, 1 H), 4.79 (bs, 1 H), 3.72-4.28 (m, 2 H), 2.72-3.16 (m, 2 H).

### Example 46: (±)-(E)-N-Hydroxy-3-{6-[(E)-3-oxo-3-(3-p-tolyl-piperidin-1-yl)-propenyl]-pyridin-2-yl}-acrylamide hydrochloride

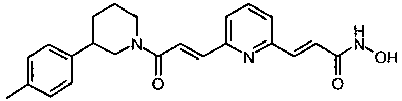

LC-MS: Method C, column Acquity UPLC-BEH C18 50x2.1mmx1.7µm, rt=1.94;
(ES+) MH⁺: 392.09
¹H NMR (DMSO-d₆) δ (ppm): 7.71-8.15 (m, 2 H), 7.34-7.74 (m, 4 H), 6.71-7.34 (m, 5 H), 4.35-4.79 (m, 1 H), 3.88-4.38 (m, 1 H), 3.01-3.42 (m, 1 H), 2.55-2.99 (m, 2 H), 2.28 (s, 3 H), 1.24-2.10 (m, 4 H).

### Example 47: (±)-(E)-N-Hydroxy-3-{6-[(E)-3-(3-naphthalen-2-yl-piperidin-1-yl)-3-oxo-propenyl]-pyridin-2-yl}-acrylamide hydrochloride

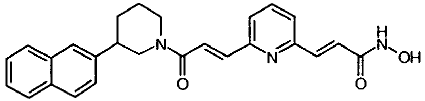

LC-MS: Method F, column Acquity UPLC-BEH C18 50x2.1mmx1.7µm, rt=3.16;
(ES+) MH⁺: 428.08
¹H NMR (DMSO-d₆ 353K) δ (ppm): 7.75-7.98 (m, 5 H), 7.32-7.77 (m, 8 H), 7.02 (d, 1 H), 4.02-4.79 (m, 2 H), 2.99-3.46 (m, 2 H), 2.76-3.00 (m, 1 H), 2.03-2.23 (m, 1 H), 1.79-2.04 (m, 2 H), 1.44-1.82 (m, 1 H).

### Example 48: (±)-(E)-3-(6-{(E)-3-[3-(4-Fluoro-phenyl)-piperidin-1-yl]-3-oxo-propenyl}-pyridin-2-yl)-N-hydroxy-acrylamide hydrochloride

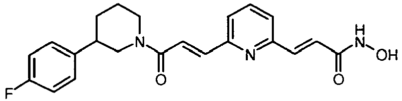

LC-MS: Method C, column Acquity UPLC-BEH C18 50x2.1mmx1.7µm, rt=1.82;
(ES+) MH⁺: 396.07
¹H NMR (DMSO-d₆) δ (ppm): 7.75-8.09 (m, 2 H), 7.27-7.75 (m, 6 H), 6.66-7.26 (m, 3 H), 4.38-4.77 (m, 1 H), 3.87-4.39 (m, 1 H), 3.02-3.47 (m, 1 H), 2.53-3.01 (m, 2 H), 1.20-2.18 (m, 4 H).

### Example 49: (E)-N-Hydroxy-3-(6-{(E)-3-[4-(4-isopropyl-phenyl)-piperazin-1-yl]-3-oxo-propenyl}-pyridin-2-yl)-acrylamide hydrochloride

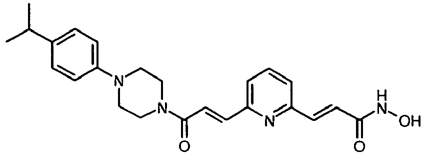

LC-MS: Method C, column Acquity UPLC-BEH C18 50x2.1mmx1.7µm, rt=1.75;
(ES+) MH⁺: 421.1
¹H NMR (DMSO-d₆) δ (ppm): 7.93 (t, 1 H), 7.80 (d, 1 H), 7.66 (d, 1 H), 7.51-7.73 (m, 2 H), 7.51 (d, 1 H), 7.32 (m, 4 H), 7.05 (d, 1 H), 3.69-4.33 (m, 4 H), 3.12-3.66 (m, 4 H), 2.88 (spt, 1 H), 1.19 (d, 6 H).

### Example 50: (E)-3-(6-{(E)-3-[4-(4-tert-Butyl-phenyl)-piperazin-1-yl]-3-oxo-propenyl}-pyridin-2-yl)-N-hydroxy-acrylamide hydrochloride

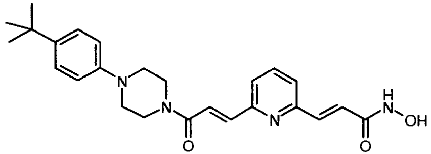

LC-MS: Method C, column Acquity UPLC-BEH C18 50x2.1mmx1.7µm, rt=1.88;
(ES+) MH⁺: 435.1
¹H NMR (DMSO-d₆) δ (ppm): 7.92 (t, 1 H), 7.80 (d, 1 H), 7.66 (d, 1 H), 7.45-7.73 (m, 2 H), 7.51 (d, 1 H), 7.35 (m, 4 H), 7.05 (d, 1 H), 3.59-4.47 (m, 4 H), 3.04-3.56 (m, 4 H), 1.27 (s, 9 H).

### Example 51: (E)-N-Hydroxy-3-(6-{(E)-3-[4-(4-methanesulfonyl-phenyl)-piperazin-1-yl]-3-oxo-propenyl}-pyridin-2-yl)-acrylamide hydrochloride

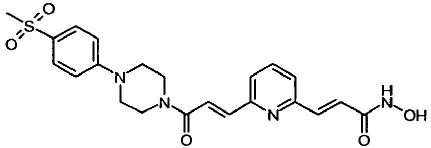

LC-MS: Method C, column Acquity UPLC-BEH C18 50x2.1mmx1.7µm, rt= 1.29;
(ES+) MH⁺: 457
¹H NMR (DMSO-d₆) δ (ppm): 7.91 (t, 1 H), 7.78 (d, 1 H), 7.71 (m, 2 H), 7.63 (d, 1 H), 7.58 (s, 1 H), 7.54 (d, 1 H), 7.51 (d, 1 H), 7.11 (m, 2 H), 7.02 (d, 1 H), 3.60-3.85 (m, 4 H), 3.33-3.60 (m, 4 H), 3.09 (s, 3 H).

### Example 52: (±)-(E)-N-Hydroxy-3-{6-[(E)-3-oxo-3-(3-phenyl-pyrrolidin-1-yl)-propenyl]-pyridin-2-yl}-acrylamide hydrochloride

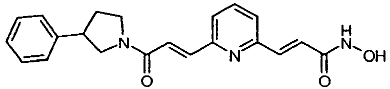

LC-MS: Method C, column Acquity UPLC-BEH C18 50x2.1mmx1.7µm, rt=1.64;
(ES+) MH⁺: 364.12
¹H NMR (DMSO-d₆) δ (ppm): 7.90 (t, 1 H), 7.76 (d, 1 H), 7.13-7.65 (m, 9 H), 7.00 (d, 1 H), 3.88-4.08 (m, 1 H), 3.07-4.50 (m, 4 H), 2.20-2.47 (m, 1 H), 1.80-2.20 (m, 1 H).

### Example 53: (±)-(E)-N-Hydroxy-3-{6-[(E)-3-oxo-3-(2-phenyl-piperidin-1-yl)-propenyl]-pyridin-2-yl}-acrylamide hydrochloride

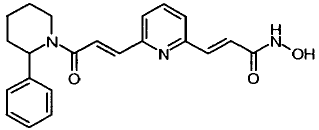

LC-MS: Method C, column Acquity UPLC-BEH C18 50x2.1mmx1.7µm, rt=1.79;
(ES+) MH⁺: 378.09 ¹H NMR (DMSO-d₆ 353K) δ (ppm): 7.84 (t, 1 H), 7.64 (d, 1 H), 7.52-7.60 (m, 1 H), 7.48-7.56 (m, 1 H), 7.49 (d, 1 H), 7.47 (d, 1 H), 7.33-7.43 (m, 2 H), 7.20-7.34 (m, 3 H), 7.00 (d, 1 H), 5.44-5.95 (m, 1 H), 3.90-4.48 (m, 1 H), 2.79-3.13 (m, 1 H), 2.29-2.47 (m, 1 H), 1.81-2.08 (m, 1 H), 1.35-1.81 (m, 4 H).

### Example 54: (±)-(E)-N-Hydroxy-3-{6-[(E)-3-oxo-3-(4-phenyl-azepan-1-yl)-propenyl]-pyridin-2-yl}-acrylamide hydrochloride

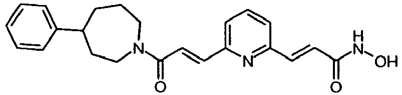

LC-MS: Method C, column Acquity UPLC-BEH C18 50x2.1mmx1.7µm, rt=1.84;
(ES+) MH⁺: 392.08
¹H NMR (DMSO-d₆) δ (ppm): 7.82-8.00 (m, 1 H), 7.77 (d, 1 H), 7.40-7.69 (m, 4 H), 7.08-7.40 (m, 5 H), 6.99 (d, 1 H), 3.25-4.12 (m, 4 H), 2.59-2.82 (m, 1 H), 1.90-2.10 (m, 2 H), 1.62-1.88 (m, 4 H).

### Example 55: (E)-3-{6-[(E)-3-(3,4-Dihydro-2H-quinolin-1-yl)-3-oxo-propenyl]-pyridin-2-yl}-N-hydroxy-acrylamide trifluoroacetate

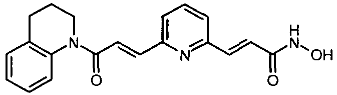

LC-MS: Method C, column Acquity UPLC-BEH C18 50x2.1mmx1.7µm, rt=1.63;
(ES+) MH⁺: 350.15
¹H NMR (DMSO-d₆) δ (ppm): 10.87 (bs, 1 H), 7.86 (t, 1 H), 7.55-7.65 (m, 3 H), 7.43 (d, 1 H), 7.34 (d, 1 H), 7.06-7.29 (m, 4 H), 6.84 (d, 1 H), 3.83 (t, 2 H), 2.76 (t, 2 H), 1.94 (quint, 2 H).

### Example 56: (E)-N-Hydroxy-3-{6-[(E)-3-oxo-3-(1,3,4,9-tetrahydro-beta-carbolin-2-yl)-propenyl]-pyridin-2-yl}-acrylamide hydrochloride

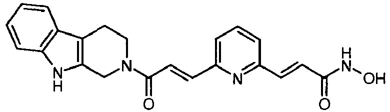

LC-MS: Method C, column Acquity UPLC-BEH C18 50x2.1 mmx1.7µm, rt=1.69;
(ES+) MH⁺: 389.13
¹H NMR (DMSO-d₆ 353K) δ (ppm): 10.62 (bs, 1 H), 7.88 (t, 1 H), 7.68 (d, 1 H), 7.64 (d, 1 H), 7.51-7.58 (m, 1 H), 7.50 (d, 1 H), 7.51 (d, 1 H), 7.42 (d, 1 H), 7.33 (d, 1 H), 6.87-7.20 (m, 3 H), 4.87 (s, 2 H), 4.01 (t, 2 H), 2.84 (t, 2 H).

### Example 57: (E)-3-{6-[(E)-3-(4-Benzooxazol-2-yl-piperidin-1-yl)-3-oxo-propenyl]-pyridin-2-yl}-N-hydroxy-acrylamide hydrochloride

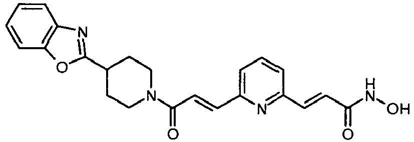

LC-MS: Method C, column Acquity UPLC-BEH C18 50x2.1mmx1.7µm, rt=1.60;
(ES+) MH⁺: 419.03
¹H NMR (DMSO-d₆) δ (ppm): 7.93 (t, 1 H), 7.80 (d, 1 H), 7.65-7.76 (m, 2 H), 7.64 (d, 1 H), 7.60 (d, 1 H), 7.52 (d, 2 H), 7.21-7.43 (m, 2 H), 7.03 (d, 1 H), 3.78-4.81 (m, 2 H), 3.24-3.75 (m, 2 H), 2.83-3.27 (m, 1 H), 2.00-2.41 (m, 2 H), 1.45-2.01 (m, 2 H).

### Example 58: (E)-3-{6-[(E)-3-(3,4-Dihydro-1H-isoquinolin-2-yl)-3-oxo-propenyl]-pyridin-2-yl}-N-hydroxy-acrylamide hydrochloride

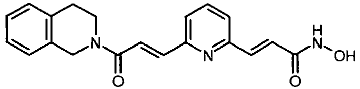

LC-MS: Method C, column Acquity UPLC-BEH C18 50x2.1mmx1.7µm, rt=1.52;
(ES+) MH⁺: 350.08
¹H NMR (DMSO-d₆) δ (ppm): 7.92 (t, 1 H), 7.80 (d, 1 H), 7.67 (d, 1 H), 7.59 (d, 1 H), 7.55 (d, 1 H), 7.52 (d, 1 H), 7.14-7.37 (m, 4 H), 7.03 (d, 1 H), 4.43-5.08 (m, 2 H), 3.51-4.15 (m, 2 H), 2.75-3.15 (m, 2 H).

### Example 59: (E)-3-(6-{(E)-3-[4-(1H-Benzoimidazol-2-yl)-piperidin-1-yl]-3-oxo-propenyl}-pyridin-2-yl)-N-hydroxy-acrylamide hydrochloride

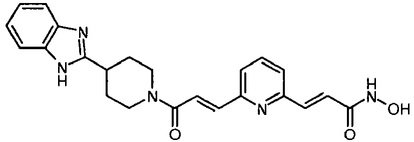

LC-MS: Method F, column Acquity UPLC-BEH C18 50x2.1mmx1.7µm, rt=1.44;
(ES+) MH⁺: 418.03
¹H NMR (DMSO-d₆) δ (ppm): 10.95 (bs, 1 H), 7.90 (t, 1 H), 7.71-7.85 (m, 3 H), 7.65 (d, 1 H), 7.51 (d, 1 H), 7.37-7.70 (m, 4 H), 7.02 (d, 1 H), 4.54-4.86 (m, 1 H), 4.28-4.54 (m, 1 H), 3.49-3.75 (m, 1 H), 3.22-3.49 (m, 1 H), 2.77-3.18 (m, 1 H), 2.02-2.40 (m, 2 H), 1.39-2.06 (m, 2 H).

### Example 60: (E)-3-(6-{(E)-3-[Spiro[indene-1,4'-piperidine-1'-yl]-3-oxo-propenyl}-pyridin-2-yl)-N-hydroxy-acrylamide hydrochloride

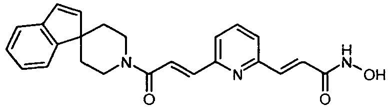

LC-MS: Method C, column Acquity UPLC-BEH C18 50x2.1mmx1.7µm, rt=1.89;
(ES+) MH⁺:402.13
¹H NMR (DMSO-d₆) δ (ppm): 7.98 (t, 1 H), 7.87 (d, 1 H), 7.74 (d, 1 H), 7.65 (d, 1 H), 7.59 (d, 1 H), 7.55 (d, 1 H), 7.45 (d, 1 H), 7.31-7.40 (m, 1 H), 7.12-7.28 (m, 3 H), 7.06 (d, 1 H), 6.86 (d, 1 H), 3.92-4.86 (m, 2 H), 3.45-3.86 (m, 1 H), 3.18 (t, 1 H), 1.77-2.22 (m, 2 H), 1.30 (t, 2 H).

### Example 61: (E)-3-(6-{(E)-3-(6-{(E)-3-[Spiro[2-benzofuran-1,4'-piperidine-1'-yl]-3-oxo-propenyl}-pyridin-2-yl)-N-hydroxy-acrylamide hydrochloride

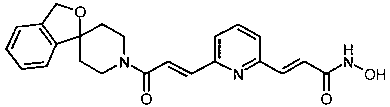

LC-MS: Method F, column Acquity UPLC-BEH C18 50x2.1mmx1.7µm, rt=2.42;
(ES+) MH⁺: 405.98
¹H NMR (DMSO-d₆) δ (ppm): 7.92 (t, 1 H), 7.80 (d, 1 H), 7.67 (d, 1 H), 7.59 (d, 1 H), 7.54 (d, 1 H), 7.51 (d, 1 H), 7.16-7.41 (m, 4 H), 7.02 (d, 1 H), 5.05 (s, 2 H), 4.41-4.77 (m, 1 H), 3.91-4.39 (m, 1 H), 3.49 (t, 1 H), 2.77-3.27 (m, 1 H), 1.79-2.20 (m, 2 H), 1.46-1.80 (m, 2 H).

### Example 62: (E)-N-Hydroxy-3-(6-{(E)-3-oxo-3-[4-(2-phenyl-benzoimidazol-1-yl)-piperidin-1-yl]-propenyl}-pyridin-2-yl)-acrylamide hydrochloride

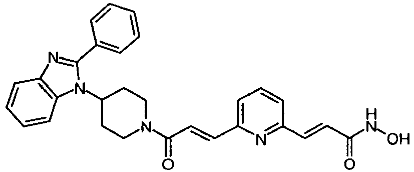

LC-MS: Method F, column Acquity UPLC-BEH C18 50x2.1mmx1.7µm, rt=1.26;
(ES+) MH⁺: 494.05
¹H NMR (DMSO-d₆ 353K) δ (ppm): 8.00 (dd, 1 H), 7.77-7.94 (m, 4 H), 7.64-7.77 (m, 4 H), 7.33-7.60 (m, 6 H), 7.04 (d, 1 H), 4.64-4.87 (m, 1 H), 4.53 (d, 2 H), 2.78-3.31 (m, 2 H), 2.29-2.46 (m, 2 H), 1.76-2.31 (m, 2 H).

### Example 63: (±)-(E)-N-Hydroxy-3-{6-[(E)-3-(4-methyl-3-phenyl-piperazin-1-yl)-3-oxo-propenyl]-pyridin-2-yl}-acrylamide hydrochloride

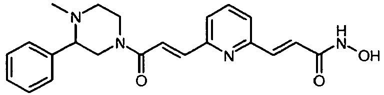

LC-MS: Method F, column Acquity UPLC-BEH C18 50x2.1mmx1.7µm, rt=1.48;
(ES+) MH⁺:393.1
¹H NMR (DMSO-d₆ 353K) δ (ppm): 7.87 (t, 1H), 7.72 (d, 1H), 7.67-7.83 (m, 2H), 7.38-7.62 (m, 7H), 7.04 (d, 1H), 4.16-4.72 (m, 3H), 3.80-4.10 (m, 1H), 3.46-3.81 (m, 2H), 3.27 (td, 1H), 2.52 (s, 3H).

### Example 64: (±)-(E)-3-{6-[(E)-3-(4-Ethyl-3-phenyl-piperazin-1-yl)-3-oxo-propenyl]-pyridin-2-yl}-N-hydroxy-acrylamide hydrochloride

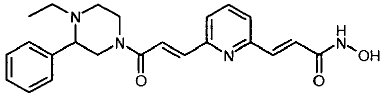

LC-MS: Method C, column Acquity UPLC-BEH C18 50x2.1mmx1.7µm, rt= 1,13;
(ES+) MH⁺: 407.1
¹H NMR (DMSO-d₆ 353K) δ (ppm): 12.10 (bs, 1H), 7.78-8.00 (m, 3H), 7.27-7.78 (m, 8 H), 7.05 (d, 1H), 4.53-4.85 (m, 1H), 4.21-4.53 (m, 2H), 3.86-4.14 (m, 1H), 3.47-3.86 (m, 2H), 3.10-3.36 (m, 1H), 2.97 (dq, 1H), 2.85 (dq, 1H), 1.18 (t, 3H).

### Example 65: (±)-(E)-3-{6-[(E)-3-(4-Benzyl-3-phenyl-piperazin-1-yl)-3-oxo-propenyl]-pyridin-2-yl}-N-hydroxy-acrylamide hydrochloride

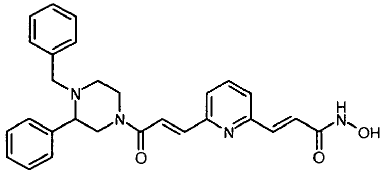

LC-MS: Method C, column Acquity UPLC-BEH C18 50x2.1mmx1.7µm, rt=1.44;
(ES+) MH⁺: 469.12
¹H NMR (DMSO-d₆ 353K) δ (ppm): 7.86 (t, 2H), 7.28-7.75 (m, 14H), 7.02 (d, 1H), 3.98 (d, 1H), 3.44-4.76 (m, 6H), 3.10-3.38 (m, 1H), 2.75-3.09 (m, 1H).

### Example 66: (±)-(E)-3-{6-[(E)-3-(4-Acetyl-3-phenyl-piperazin-1-yl)-3-oxo-propenyl]-pyridin-2-yl}-N-hydroxy-acrylamide hydrochloride

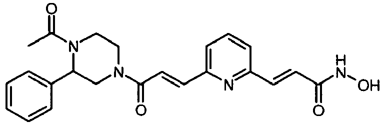

LC-MS: Method C, column Acquity UPLC-BEH C18 50x2.1mmx1.7µm, rt=1.82;
(ES+) MH⁺: 421.02
¹H NMR (DMSO-d₆ 353K) δ (ppm): 7.85 (t, 1H), 7.63 (dd, 1H), 7.53 (d, 1H), 7.48 (d, 1H), 7.13-7.44 (m, 7H), 7.03 (d, 1H), 5.50 (bs, 1H), 4.60 (d, 1H), 3.86-4.27 (m, 2H), 3.53-3.85 (m, 1H), 3.14-3.53 (m, 2H), 2.09 (s, 3H).

### Example 67: (±)-(E)-N-Hydroxy-3-(6-{(E)-3-[3-(2-methoxy-phenyl)-piperidin-1-yl]-3-oxo-propenyl}-pyridin-2-yl)-acrylamide hydrochloride

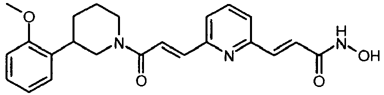

LC-MS: Method C, column Acquity UPLC-BEH C18 50x2.1mmx1.7µm, rt=1.86;
(ES+) MH⁺: 408.6
¹H NMR (DMSO-d₆ 353K) δ (ppm): 7.74-7.94 (m, 1H), 7.35-7.75 (m, 5H), 7.10-7.35 (m, 2H), 6.89-7.12 (m, 2H), 6.88 (d, 1H), 3.99-4.84 (m, 2H), 3.54-3.99 (m, 3H), 2.71-3.38 (m, 3H), 1.72-2.23 (m, 3H), 1.30-1.72 (m, 1H).

### Example 68: (±)-(E)-N-Hydroxy-3-(6-{(E)-3-[3-(3-methoxy-phenyl)-piperidin-1-yl]-3-oxo-propenyl}-pyridin-2-yl)-acrylamide hydrochloride

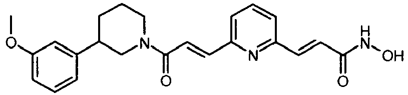

LC-MS: Method C, column Acquity UPLC-BEH C18 50x2.1mmx1.7µm, rt=1.78;
(ES+) MH⁺: 408.12
¹H NMR (MeOD 333K) δ (ppm): 8.38 (dd, 1H), 7.99-8.23 (m, 2H), 7.59-7.86 (m, 3H), 7.25 (dd, 1H), 7.10 (d, 1H), 6.74-6.96 (m, 3H), 4.64 (bs, 1H), 4.29 (bs, 1H), 3.80 (s, 3H), 3.21-3.48 (m, 1H), 2.71-3.05 (m, 2H), 2.05-2.15 (m, 1H), 1.80-2.02 (m, 2H), 1.62-1.77 (m, 1H).

### Example 69: (±)-(E)-N-Hydroxy-3-(6-{(E)-3-[3-(4-methoxy-phenyl)-piperidin-1-yl]-3-oxo-propenyl}-pyridin-2-yl)-acrylamide hydrochloride

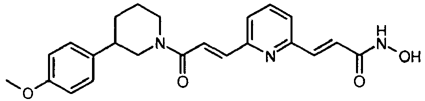

LC-MS: Method C, column Acquity UPLC-BEH C18 50x2.1mmx1.7µm, rt=1.76;
(ES+) MH⁺: 408.12
¹H NMR (DMSO-d₆ 353K) δ (ppm): 7.85 (dd, 1H), 7.60-7.73 (m, 1H), 7.36-7.60 (m, 4H), 7.22 (m, 2H), 7.02 (d, 1H), 6.90 (m, 2H), 4.34 (bs, 2H), 3.75 (s, 3H), 3.00 (bs, 2H), 2.59-2.80 (m, 1H), 1.92-2.07 (m, 1H), 1.78-1.92 (m, 1H), 1.66-1.78 (m, 1H), 1.48-1.66 (m, 1H).

### Example 70: (±)-(E)-N-Hydroxy-3-(6-{(E)-3-oxo-3-[3-(2-trifluoromethyl-phenyl)-piperidin-1-yl]-propenyl}-pyridin-2-yl)-acrylamide hydrochloride

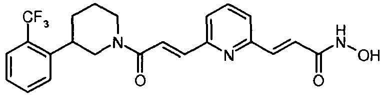

LC-MS: Method C, column Acquity UPLC-BEH C18 50x2.1mmx1.7µm, rt=1.97;
(ES+) MH⁺: 446.03
¹H NMR (DMSO-d₆ 353K) δ (ppm): 7.78-8.02 (m, 1H), 7.54-7.79 (m, 5H), 7.27-7.58 (m, 4H), 7.01 (d, 1H), 3.90-4.84 (m, 2H), 2.77-3.59 (m, 3H), 1.75-2.19 (m, 3H), 1.20-1.74 (m, 1H).

### Example 71: (±)-(E)-N-Hydroxy-3-(6-{(E)-3-oxo-3-[3-(3-trifluoromethyl-phenyl)-piperidin-1-yl]-propenyl}-pyridin-2-yl)-acrylamide hydrochloride

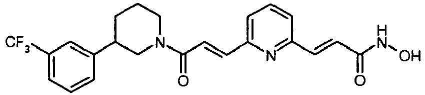

LC-MS: Method C, column Acquity UPLC-BEH C18 50x2.1mmx1.7µm, rt=2.05;
(ES+) MH⁺: 446.09
¹H NMR (MeOD 333K) δ (ppm): 8.32 (dd, 1H), 8.10 (d, 1H), 8.02 (d, 1H), 7.50-7.83 (m, 7H), 7.08 (bs, 1H), 4.67 (bs, 1H), 4.31 (bs, 1H), 3.32 (bs, 1H), 2.76-3.07 (m, 2H), 2.08-2.18 (m, 1H), 1.84-2.03 (m, 2H), 1.65-1.80 (m, 1H).

### Example 72: (±)-(E)-N-Hydroxy-3-(6-{(E)-3-oxo-3-[3-(4-trifluoromethyl-phenyl)-piperidin-1-yl]-propenyl}-pyridin-2-yl)-acrylamide hydrochloride

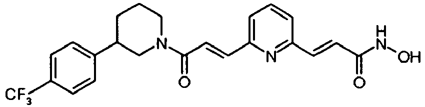

LC-MS: Method C, column Acquity UPLC-BEH C18 50x2.1 mmx1.7µm, rt=2.07;
(ES+) MH⁺: 446.10
¹H NMR (MeOD 333K) δ (ppm): 8.34 (dd, 1H), 8.12 (d, 1H), 8.05 (d, 1H), 7.70-7.86 (m, 2H), 7.59-7.70 (m, 3H), 7.52 (m, 2H), 7.09 (bs, 1H), 4.65 (bs, 1H), 4.35 (bs, 1H), 3.32 (bs, 1H), 2.93 (bs, 2H), 2.05-2.21 (m, 1H), 1.82-2.05 (m, 2H), 1.61-1.82 (m, 1H).

### Example 73: (E)-N-Hydroxy-3-{6-[(E)-3-oxo-3-((S)-3-phenyl-piperidin-1-yl)-propenyl]-pyridin-2-yl}-acrylamide hydrochloride

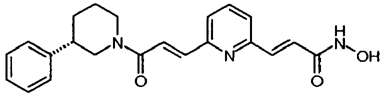

LC-MS: Method C, column Acquity UPLC-BEH C18 50x2.1mmx1.7µm, rt=1.78;
(ES+) MH⁺: 378.15
¹H NMR (DMSO-d₆ 353K) δ (ppm): 7.85 (t, 1H), 7.68 (d, 1H), 7.39-7.64 (m, 4H), 7.16-7.39 (m, 5H), 7.02 (d, 1H), 3.63-4.83 (m, 1H), 2.85-3.32 (m, 2H), 2.56-2.85 (m, 2H), 1.94-2.11 (m, 1H), 1.68-1.94 (m, 2H), 1.39-1.70 (m, 1H).
[α]_{D}= -112.9 (C=0.5, MeOH)

### Example 74: (E)-N-Hydroxy-3-{6-[(E)-3-oxo-3-((R)-3-phenyl-piperidin-1-yl)-propenyl]-pyridin-2-yl}-acrylamide hydrochloride

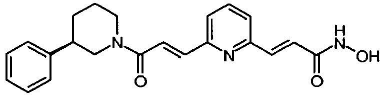

LC-MS: Method C, column Acquity UPLC-BEH C18 50x2.1 mmx1.7µm, rt=1.78;
(ES+) MH⁺: 378.15
¹H NMR (MeOD 333K) δ (ppm): 8.25 (t, 1H), 8.01 (d, 1H), 7.94 (d, 1H), 7.52-7.82 (m, 3H), 7.18-7.39 (m, 5H), 7.07 (bs, 1H), 4.66 (bs, 1H), 4.30 (bs, 1H), 3.33-3.43 (m, 1H), 2.74-2.98 (m, 2H), 2.06-2.14 (m, 1H), 1.84-2.02 (m, 2H), 1.63-1.78 (m, 1H).
[α]_{D}=+137.5 (C=0.5, MeOH)

The following compounds were prepared according to the synthetic procedure of Example 15 starting from (E)-3-{3-[(E)-2-(tetrahydro-pyran-2-yloxycarbamoyl)-vinyl]-phenyl}-acrylic acid (prepared as described in Preparation 2) and the corresponding amine.

### Example 75: (E)-N-Hydroxy-3-{3-[(E)-3-oxo-3-(4-phenyl-3,6-dihydro-2H-pyridin-1-yl)-propenyl]-phenyl}-acrylamide

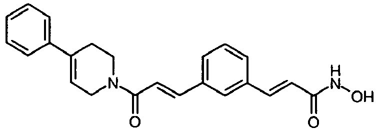

LC-MS: Method C, column Acquity UPLC-BEH C18 50x2.1mmx1.7µm, rt=2.03;
(ES+) MH⁺: 375.11
¹H NMR (DMSO-d₆ 353K) δ (ppm): 7.87 (t, 1H), 7.67 (dt, 1H), 7.40-7.59 (m, 6H), 7.32-7.40 (m, 2H), 7.20-7.32 (m, 2H), 6.61 (d, 1H), 6.14-6.23 (m, 1H), 4.32 (ddd, 2H), 3.88 (t, 2H), 2.54-2.66 (m, 2H).

### Example 76: (E)-3-(3-{(E)-3-[4-(4-Chloro-phenyl)-piperazin-1-yl]-3-oxo-propenyl}-phenyl)-N-hydroxy-acrylamide hydrochloride

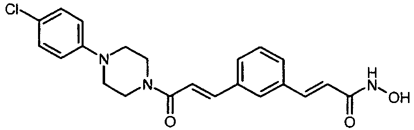

LC-MS: Method C, column Acquity UPLC-BEH C18 50x2.1mmx1.7µm, rt=2.01;
(ES+) MH⁺: 412.10
¹H NMR (DMSO-d₆ 373 K) δ (ppm): 7.79-7.86 (m, 1H), 7.60-7.68 (m, 1H), 7.51-7.57 (m, 2H), 7.47-7.50 (m, 1H), 7.44 (dd, 1H), 7.24 (m, 2H), 7.20 (d, 1H), 6.96 (m, 2H), 6.62 (d, 1H), 3.74-3.86 (m, 4H), 3.21-3.28 (m, 4H).

### Example 77: (E)-3-{3-[(E)-3-(4-Benzoyl-piperidin-1-yl)-3-oxo-propenyl]-phenyl}-N-hydroxy-acrylamide

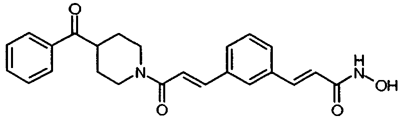

LC-MS: Method C, column Acquity UPLC-BEH C18 50x2.1 mmx1.7µm, rt=1.82;
(ES+) MH⁺: 405.15
¹H NMR (DMSO-d₆ 373 K) δ (ppm): 7.92-8.02 (m, 2H), 7.82 (t, 1H), 7.57-7.68 (m, 2H), 7.48-7.57 (m, 4H), 7.36-7.48 (m, 2H), 7.18 (d, 1H), 6.61 (d, 1H), 4.22-4.40 (m, 2H), 3.64-3.79 (m, 1H), 3.13-3.28 (m, 2H), 1.84-2.00 (m, 2H), 1.51-1.72 (m, 2H).

### Example 78: (±)-(E)-N-Hydroxy-3-{3-[(E)-3-oxo-3-(3-m-tolyl-piperidin-1-yl)-propenyl]-phenyl}-acrylamide

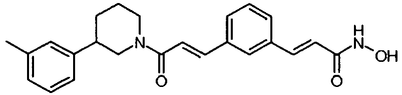

LC-MS: Method C, column Acquity UPLC-BEH C18 50x2.1mmx1.7µm, rt=2.17;
(ES+) MH⁺: 391.15
¹H NMR (DMSO-d₆ 373K) δ (ppm): 7.80 (s, 1H), 7.56-7.69 (m, 2H), 7.36-7.56 (m, 3H), 7.15-7.28 (m, 2H), 6.97-7.15 (m, 3H), 6.60 (d, 1H), 4.31-4.45 (m, 2H), 2.89-3.14 (m, 2H), 2.60-2.80 (m, 1H), 2.32 (s, 3H), 1.92-2.07 (m, 1H), 1.67-1.92 (m, 2H), 1.46-1.67 (m, 1H).

### Example 79: (±)-(E)-N-Hydroxy-3-{3-[(E)-3-oxo-3-(3-o-tolyl-piperidin-1-yl)-propenyl]-phenyl}-acrylamide

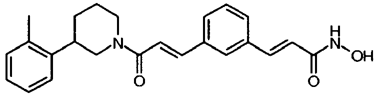

LC-MS: Method C, column Acquity UPLC-BEH C18 50x2.1mmx1.7µm, rt=2.13;
(ES+) MH⁺: 391.15
¹H NMR (DMSO-d₆) δ (ppm): 7.76-7.84 (m, 1H), 7.62 (d, 1H), 7.45-7.57 (m, 3H), 7.42 (dd, 1H), 7.05-7.32 (m, 5H), 6.60 (d, 1H), 4.33-4.47 (m, 2H), 2.84-3.07 (m, 3H), 2.34 (s, 3H), 1.71-2.02 (m, 3H), 1.55-1.71 (m, 1H).

### Example 80: (±)-(E)-N-Hydroxy-3-{3-[(E)-3-oxo-3-(3-p-tolyl-piperidin-1-yl)-propenyl]-phenyl}-acrylamide

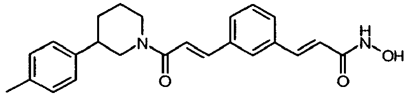

LC-MS: Method C, column Acquity UPLC-BEH C18 50x2.1mmx1.7µm, rt=2.20;
(ES+) MH⁺: 391.20
¹H NMR (DMSO-d₆ 373K) δ (ppm): 7.74-7.85 (m, 1H), 7.59-7.66 (m, 1H), 7.30-7.57 (m, 4H), 6.94-7.30 (m, 5H), 6.61 (d, 1H), 4.37 (bs, 2 H), 2.81-3.25 (m, 2H), 2.57-2.83 (m, 1H), 2.30 (s, 3H), 1.93-2.14 (m, 1H), 1.34-1.93 (m, 3H).

### Example 81: (E)-N-Hydroxy-3-{3-[(E)-3-oxo-3-((R)-3-phenyl-piperidin-1-yl)-propenyl]-phenyl}-acrylamide

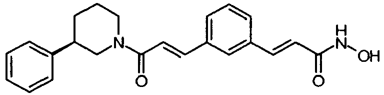

LC-MS: Method C, column Acquity UPLC-BEH C18 50x2.1mmx1.7µm, rt=2.03;
(ES+) MH⁺: 377.16
¹H NMR (DMSO-d₆ 353K) δ (ppm): 7.79-7.86 (m, 1H), 7.60-7.69 (m, 1H), 7.45-7.57 (m, 3H), 7.42 (dd, 1H), 7.28-7.37 (m, 4H), 7.19-7.28 (m, 2H), 6.60 (d, 1H), 4.40 (bs, 2H), 3.49 (bs, 1H), 3.01 (bs, 1H), 2.61-2.85 (m, 1H), 1.93-2.06 (m, 1H), 1.68-1.90 (m, 2H), 1.47-1.67 (m, 1H).
[α]_{D}=+112.1 (C=0.5, MeOH)

### Example 82: (E)-N-Hydroxy-3-{3-[(E)-3-oxo-3-((S)-3-phenyl-piperidin-1-yl)-propenyl]-phenyl}-acrylamide

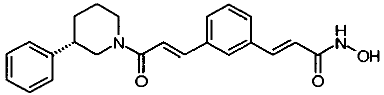

LC-MS: Method C, column Acquity UPLC-BEH C18 50x2.1mmx1.7µm, rt=2.04;
(ES+) MH⁺: 377.21
¹H NMR (DMSO-d₆ 373K) δ (ppm): 7.81 (s, 1H), 7.63 (d, 1H), 7.19-7.58 (m, 9H), 7.20 (d, 1H), 6.61 (d, 1H), 4.19-4.55 (m, 2H), 2.88-3.20 (m, 2 H), 2.62-2.87 (m, 1H), 1.93-2.15 (m, 1H), 1.39-1.93 (m, 3H).
[α]_{D}= -104.8 (C=0.5, MeOH)

### Example 83: (±)-(E)-N-Hydroxy-3-{3-[(E)-3-oxo-3-(3-phenyl-piperidin-1-yl)-propenyl]-phenyl}-acrylamide

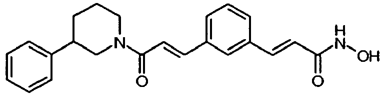

LC-MS: Method C, column Acquity UPLC-BEH C18 50x2.1mmx1.7µm, rt=2.04;
(ES+) MH⁺: 377.21
¹H NMR (DMSO-d₆ 373K) δ (ppm): 7.72-7.89 (m, 1H), 7.63 (dt, 1 H), 7.38-7.57 (m, 4H), 7.19-7.38 (m, 5H), 7.20 (d, 1H), 6.60 (d, 1H), 4.18-4.60 (m, 2H), 3.03 (t, 2H), 2.65-2.86 (m, 1H), 1.94-2.15 (m, 1H), 1.69-1.95 (m, 2H), 1.50-1.69 (m, 1H).

### Example 84: (±)-(E)-3-(3-{(E)-3-[3-(4-Fluoro-phenyl)-piperidin-1-yl]-3-oxo-propenyl}-phenyl)-N-hydroxy-acrylamide

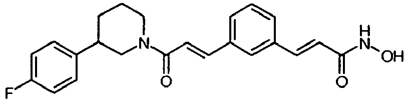

LC-MS: Method C, column Acquity UPLC-BEH C18 50x2.1mmx1.7µm, rt=2.07;
(ES+) MH⁺: 395.19
¹H NMR (DMSO-d₆ 373K) δ (ppm): 7.75-7.85 (m, 1H), 7.60-7.67 (m, 1H), 7.29-7.57 (m, 6H), 7.20 (d, 1H), 6.99-7.15 (m, 2H), 6.61 (d, 1H), 3.80-4.73 (m, 2H), 2.88-3.26 (m, 2H), 2.64-2.88 (m, 1H), 1.91-2.16 (m, 1H), 1.10-1.92 (m, 3H).

### Example 85: (E)-3-(3-{(E)-3-[4-(3-Chloro-phenyl)-piperazin-1-yl]-3-oxo-propenyl}-phenyl)-N-hydroxy-acrylamide hydrochloride

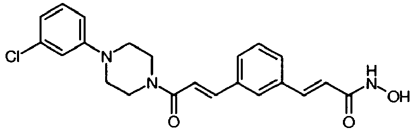

LC-MS: Method C, column Acquity UPLC-BEH C18 50x2.1mmx1.7µm, rt=2.06;
(ES+) MH⁺: 412.10
¹H NMR (DMSO-d₆ 373K) δ (ppm): 7.84 (s, 1H), 7.61-7.70 (m, 1H), 7.35-7.58 (m, 4H), 7.08-7.34 (m, 2H), 6.95 (t, 1H), 6.91 (dd, 1H), 6.80 (dd, 1H), 6.62 (d, 1H), 3.63-4.00 (m, 4 H), 2.89-3.46 (m, 4H).

### Example 86: (E)-3-{3-[(E)-3-(4-Benzyl-piperidin-1-yl)-3-oxo-propenyl]-phenyl}-N-hydroxy-acrylamide

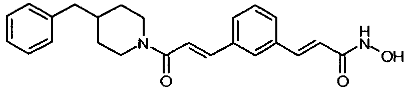

LC-MS: Method C, column Acquity UPLC-BEH C18 50x2.1mmx1.7µm, rt=2.15;
(ES+) MH⁺: 391.15
¹H NMR (DMSO-d₆ 373K) δ (ppm): 7.70-7.86 (m, 1H), 7.36-7.69 (m, 5H), 7.24-7.35 (m, 2H), 7.05-7.24 (m, 4H), 6.43-6.75 (m, 1H), 3.79-4.54 (m, 2H), 2.76-3.14 (m, 2H), 2.59 (d, 2H), 1.79-2.06 (m, 1H), 1.40-1.78 (m, 2H), 0.80-1.40 (m, 2H).

### Example 87: (E)-3-(3-{(E)-3-[4-(2-Chloro-phenyl)-piperazin-1-yl]-3-oxo-propenyl}-phenyl)-N-hydroxy-acrylamide hydrochloride

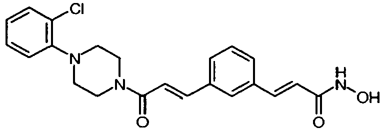

LC-MS: Method C, column Acquity UPLC-BEH C18 50x2.1mmx1.7µm, rt=2.08;
(ES+) MH⁺: 412.15
¹H NMR (DMSO-d₆ 373K) δ (ppm): 7.84 (t, 1H), 7.65 (dt, 1H), 7.37-7.58 (m, 5H), 7.26-7.35 (m, 1H), 7.23 (d, 1H), 7.18 (dd, 1H), 7.06 (td, 1H), 6.62 (d, 1H), 3.53-4.07 (m, 4H), 2.84-3.28 (m, 4H).

### Example 88: (E)-3-(3-{(E)-3-[4-(4-Cyano-phenyl)-piperazin-1-yl]-3-oxo-propenyl}-phenyl)-N-hydroxy-acrylamide hydrochloride

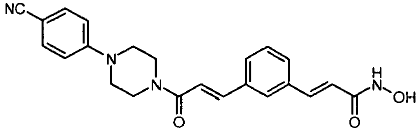

LC-MS: Method C, column Acquity UPLC-BEH C18 50x2.1mmx1.7µm, rt=1.74;
(ES+) MH⁺: 403.17
¹H NMR (DMSO-d₆ 373K) δ (ppm): 7.74-7.90 (m, 1H), 7.31-7.76 (m, 7H), 7.21 (d, 1H), 7.02 (m, 2H), 6.63 (d, 1H), 3.81 (d, 4H), 3.19-3.60 (m, 4H).

### Example 89: (E)-N-Hydroxy-3-{3-[(E)-3-oxo-3-(5-phenyl-3,6-dihydro-2H-pyridin-1-yl)-propenyl]-phenyl}-acrylamide

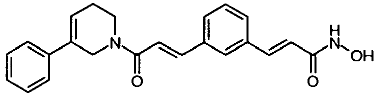

LC-MS: Method C, column Acquity UPLC-BEH C18 50x2.1mmx1.7µm, rt=2.06;
(ES+) MH⁺: 375.05
¹H NMR (DMSO-d₆) δ (ppm): 7.00-8.37 (m, 12H), 6.56 (d, J=15.26 Hz, 1H), 6.33 (bs, 1H), 4.32-4.74 (m, 2H), 3.43-4.07 (m, 2H), 1.99-2.45 (m, 2H).

### Example 90: (E)-N-Hydroxy-3-{3-[(E)-3-oxo-3-(3-phenyl-piperazin-1-yl)-propenyl]-phenyl}-acrylamide hydrochloride

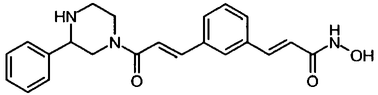

LC-MS: Method C, column Acquity UPLC-BEH C18 50x2.1mmx1.7µm, rt=1.12;
(ES+) MH⁺: 378.37
¹H NMR (DMSO-d₆ 353K) δ (ppm): 9.83 (bs, 2H), 7.80-8.03 (m, 1H), 7.64-7.75 (m, 2H), 7.38-7.63 (m, 8H), 7.28 (d, J=15.55 Hz, 1H), 6.62 (d, J=16.43 Hz, 1H), 4.49-4.70 (m, 2H), 4.42 (dd, J=11.44, 2.93 Hz, 1H), 3.50-3.70 (m, 2H), 3.33- 3.50 (m, 1H), 3.22 (td, J=12.47, 3.23 Hz, 1H).

### Example 91: (E)-N-Hydroxy-3-(3-{(E)-3-oxo-3-[4-(4-trifluoromethyl-phenyl)-piperazin-1-yl]-propenyl}-phenyl)-acrylamide hydrochloride

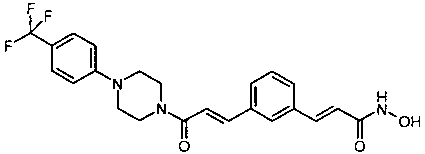

LC-MS: Method C, column Acquity UPLC-BEH C18 50x2.1mmx1.7µm, rt=2.19;
(ES+) MH⁺: 446.16
¹H NMR (DMSO-d₆ 353K) δ (ppm) 7.86 (bs, 1H), 7.58-7.78 (m, 1H), 7.50 (bs, 6H), 7.25 (d, J=15.55 Hz, 1H), 7.02-7.18 (m, 2H), 6.62 (d, J=16.43 Hz, 1H), 3.82 (bs, 4H), 3.32-3.56 (m, 4H)

The following compounds were prepared following the experimental procedure for the preparation of Example 15, starting from (E)-3-{6-[(E)-2-(tetrahydro-pyran-2-yloxycarbamoyl)-vinyl]-pyridin-2-yl}-acrylic acid (obtained as described in Preparation 5) and the corresponding amine.

### Example 92: (E)-3-(6-{(E)-3-[3-(2-Fluoro-phenyl)-piperidm-1-yl]-3-oxo-propenyl}-pyridin-2-yl)-N-hydroxy-acrylamide hydrochloride

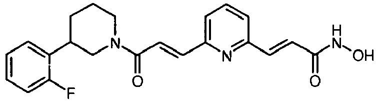

LC-MS: Method C, column Acquity UPLC-BEH C18 50x2.1mmx1.7µm, rt=1.82;
(ES+) MH⁺: 396.11
¹H NMR (DMSO-d₆ 353K) δ (ppm): 7.85 (t, J=7.78 Hz, 1H), 7.66 (d, J=7.63 Hz, 1H), 7.37-7.57 (m, 5H), 7.11-7.34 (m, 3H), 7.02 (d, J=15.85 Hz, 1H), 4.16-4.49 (m, 2H), 2.95-3.20 (m, 3H), 1.79-2.03 (m, 3H), 1.54-1.69 (m, 1H).

### Example 93: (E)-3-(6-{(E)-3-[3-(3-Fluoro-phenyl)-piperidin-1-yl]-3-oxo-propenyl}-pyridin-2-yl)-N-hydroxy-acrylamide hydrochloride

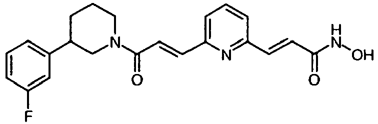

LC-MS: Method C, column Acquity UPLC-BEH C18 50x2.1mmx1.7µm, rt=1.84;
(ES+) MH⁺: 396.08
'1H NMR (DMSO-d₆ 353K) δ (ppm): 7.85 (t, J=7.78 Hz, 1H), 7.68 (dd, J=7.92, 0.88 Hz, 1H), 7.29-7.59 (m, 5H), 7.10-7.19 (m, 2H), 6.97-7.07 (m, 2H), 4.23-4.55 (m, 2H), 2.98-3.17 (m, 2H), 2.67-2.90 (m, 1H), 1.93-2.14 (m, 1H), 1.71-1.90 (m, 2H), 1.52-1.66 (m, 1H).

### Example 94: (E)-3-[6-((E)-3-{3-[3-(4-Fluoro-phenyl)-[1,2,4]oxadiazol-5-yl]-piperidin-1-yl}-3-oxo-propenyl)-pyridin-2-yl]-N-hydroxy-acrylamide hydrochloride

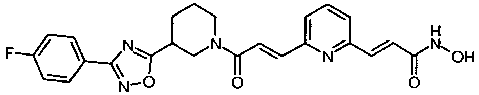

LC-MS: Method C, column Acquity UPLC-BEH C18 50x2.1mmx1.7µm, rt=1.85;
(ES+) MH⁺: 464.09
¹H NMR (DMSO-d₆) δ (ppm): 7.99-8.18 (m, 2H), 7.90 (t, J=7.78 Hz, 1H), 7.59 (d, J=8.22 Hz, 1H), 7.51 (d, J=15.26 Hz, 1H), 7.13-7.84 (m, 5H), 7.00 (d, J=16.14 Hz, 1H), 3.89-4.78 (m, 2H), 3.53-3.83 (m, 1H), 3.24-3.53 (m, 2H), 1.43-2.37 (m, 4H).

### Example 95: (E)-N-Hydroxy-3-{6-[(E)-3-oxo-3-(5-phenyl-3,6-dihydro-2H-pyridin-1-yl)-propenyl]-pyridin-2-yl}-acrylamide hydrochloride

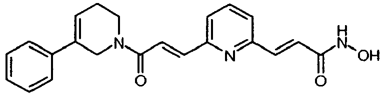

LC-MS: Method C, column Acquity UPLC-BEH C18 50x2.1mmx1.7µm, rt=1.80;
(ES+) MH⁺: 376.04
¹H NMR (DMSO-d₆) δ (ppm): 7.68 (d, J=15.26 Hz, 1H), 7.43-8.03 (m, 7H), 7.34-7.42 (m, 2H), 7.21-7.35 (m, 1H), 7.03 (d, J=15.25 Hz, 1H), 6.34 (bs, 1H), 4.45-4.67 (m, 2H), 3.39-4.03 (m, 2H), 2.14-2.46 (m, 2H).

### Example 96: (E)-N-Hydroxy-3-{6-[(E)-3-oxo-3-(6-phenyl-3,4-dihydro-1H-isoquinolin-2-yl)-propenyl]-pyridin-2-yl}-acrylamide hydrochloride

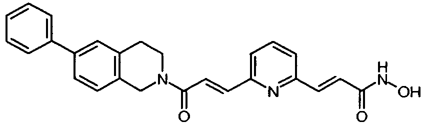

LC-MS: Method C, column Acquity UPLC-BEH C18 50x2.1mmx1.7µm, rt=1.96;
(ES+) MH⁺: 426.13
¹H NMR (DMSO-d₆) δ (ppm): 7.90 (t, J=7.63 Hz, 1H), 7.79 (d, J=7.63 Hz, 1H), 7.21-7.74 (m, 12H), 7.02 (d, J=15.26 Hz, 1H), 4.55-5.09 (m, 2H), 3.77-4.16 (m, 2H), 2.78-3.22 (m, 2H).

### Example 97: (E)-N-Hydroxy-3-{6-[(E)-3-oxo-3-(2-phenyl-6,7-dihydro-4H-thiazolo[5,4-c]pyridin-5-yl)-propenyl]-pyridin-2-yl}-acrylamide hydrochloride

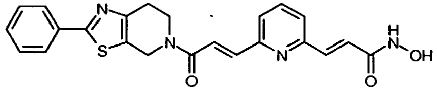

LC-MS: Method C, column Acquity UPLC-BEH C18 50x2.1mmx1.7µm, rt=1.73;
(ES+) MH⁺: 433.08
¹H NMR (DMSO-d₆ +Na₂CO₃) δ (ppm): 7.87-7.99 (m, 2H), 7.84 (t, J=7.63 Hz, 1H), 7.39-7.76 (m, 7H), 7.30 (d, J=15.26 Hz, 1H), 6.96 (d, J=15.55 Hz, 1H), 4.73-5.34 (m, 2H), 3.74-4.26 (m, 2H), 2.74-3.13 (m, 2H).

### Example 98: (E)-N-Hydroxy-3-{6-[(E)-3-oxo-3-(2-phenyl-6,7-dihydro-5H-thiazolo[5,4-b]pyridin-4-yl)-propenyl]-pyridin-2-yl}-acrylamide trifluoroacetate

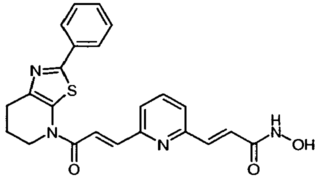

LC-MS: Method C, column Acquity UPLC-BEH C18 50x2.1mmx1.7µm, rt=1.78;
(ES+) MH⁺: 432.98
¹H NMR (DMSO-d₆) δ (ppm): 7.67-7.97 (m, 6H), 7.39-7.66 (m, 5H), 7.02 (d, J=15.55 Hz, 1H), 4.08-4.34 (m, 2H), 2.94 (t, J=6.31 Hz, 2H), 2.05-2.25 (m, 2H).

### 2. BIOLOGICAL TESTING

### Methods and results

### 2.1 Histone acetylation assay

In order to assess the ability of the compounds to modify histone acetylation levels, a dose-response study was carried out using the cell line K562 (derived from human lymphoma). The cells were incubated with the compound for 3 h, then fixed with 1% formaldehyde in PBS and permeabilized with a solution containing 0.1 % Triton X-100 in PBS. After washing, the cells were pre-incubated with 10% goat serum in PBS for 30 min at 4°C, exposed for 1 h at RT to a monoclonal antibody against acetylated histones and then incubated for 1 h with a secondary antibody conjugated with FITC. Histone acetylation levels were measured by cytofluorometry (FACS) (Ronzoni, S. et al. Cytometry A. 2005, 66, 52-61).

### 2.2 Assay of enzyme inhibition of HDAC

The in-vitro activity of HDAC inhibitors was assayed using a BIOMOL Kit, according to the instructions from the manufacturer (Biomolecular Research Laborator). 15 µl of 30 x diluted nuclear fraction of Hela cells, was diluted to 50 µl with the assay buffer containing the HDAC inhibitor and the substrate (lysine with acetylated amino group on the side chain) at a concentration of 200 µM. The samples were incubated for 15 min at RT and then exposed to a developer (10 min at RT). In this last step a fluorophore was produced, whose fluorescence was measured using an excitation wavelength of 355 nm and an emission at 460 nm. The IC₅₀ was calculated using GraphPad Software.

The compounds of examples 2-4, 8, 10-12, 14, 16, 19-20, 22-23, 27-34, 37, 40-41, 44-45, 47, 51-54, 57-64, 66, 70-71, 76-77, 83, and 88 exhibited an IC₅₀ value between 0.1 and 0.5 µM. The compounds of examples 13, 15, 17-18, 21, 24-26, 35-36, 42-43, 46, 48, 55, 67-69, 73 and 74 exhibited an IC₅₀ value below 0.1 µM.

### 2.3 Cell growth

CellTiter-Glo^{®} Luminescent Cell Viability Assay (Promega) is a homogeneous method of determining the number of viable cells in culture based on quantitation of the present ATP, which indicates the presence of metabolically active cells. The homogeneous assay procedure involves addition of a single reagent (CellTiter-Glo^{®} Reagent) directly to the cells, which leads to cell lysis and generation of a luminescent signal proportional to the amount of the ATP and the number of cells present in culture. The assay relies on the properties of a proprietary thermostable luciferase (Ultra-Glo^{®} recombinant luciferase), which generates a luminescent signal. K562, A549 and HCT-116 cells, in exponential growth, were incubated for 72 h with different concentrations of the inhibitors. After 72 h, a volume of CellTiter-Glo^{®} Reagent equal to the volume of cell culture medium was added. The content was mixed for 2 min to induce cell lysis. The luminescence was recorded after further 10 min at RT in order to obtain a stabilized luminescent signal.

The IC₅₀ was calculated using GraphPad Software.

The obtained results are illustrated in the following table 2. IC₅₀ results were allocated to one of 3 ranges as follows: Range A: IC₅₀≤1.0 µM; Range B: from 1.0 to 3.0 µM; Range C: IC₅₀≥3.0 µM.

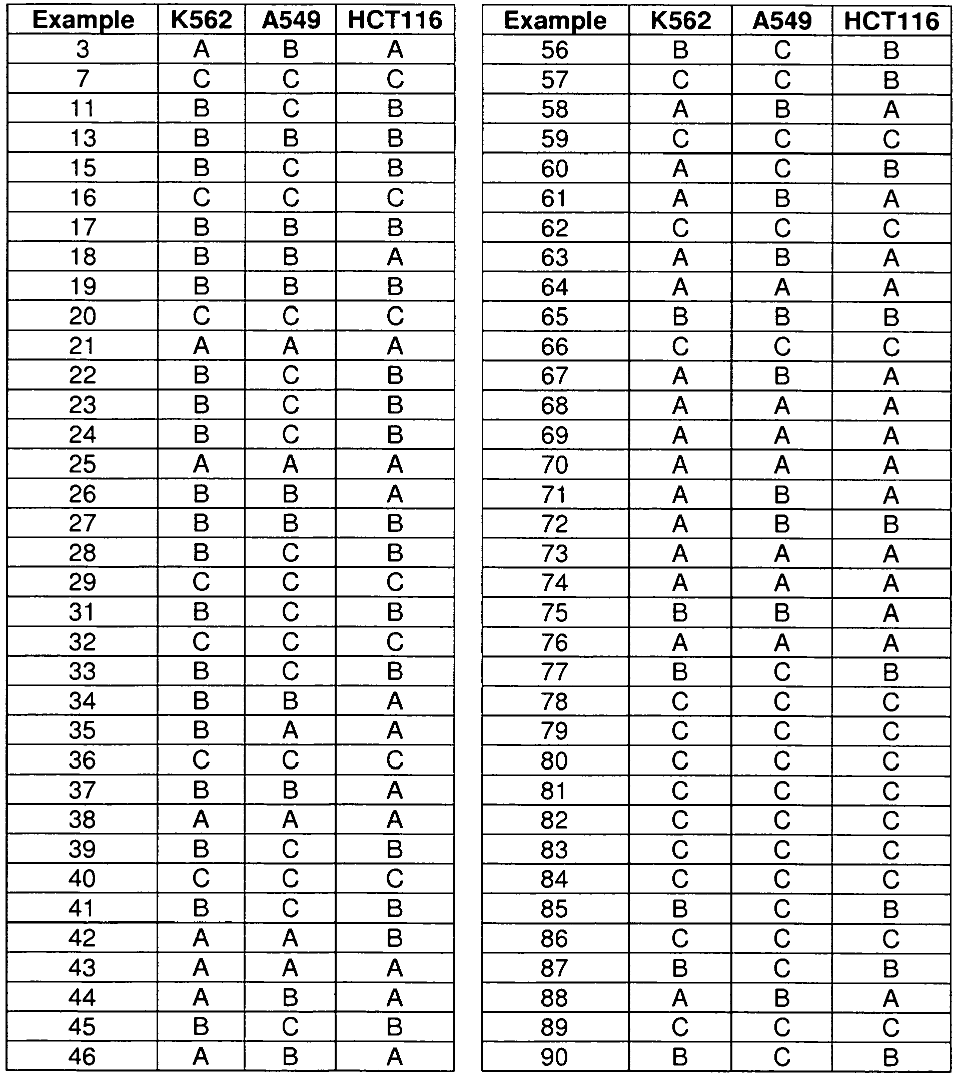

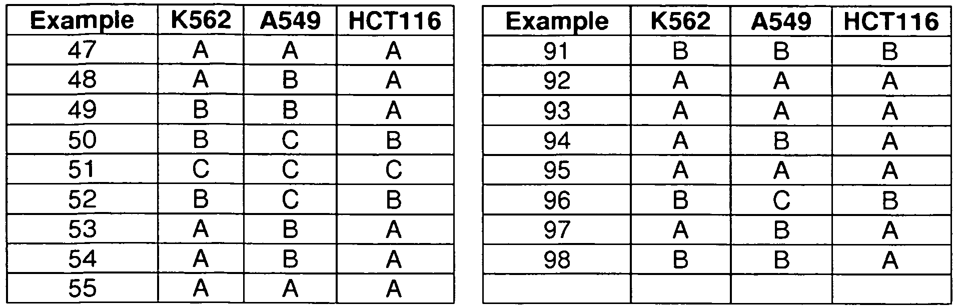

## Claims

1. A compound of formula (I) 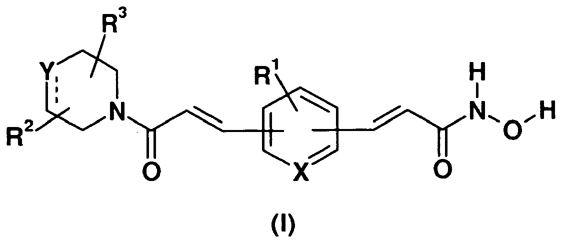 wherein:
the dotted line is an optional additional bond;
**R¹** is hydrogen;
**R², R³** are, independently, hydrogen; C₁-C₆ alkyl; aryl, optionally substituted by halogen, C₁-C₆ alkyl, C₁-C₆ alkoxy, or C₁-C₆ haloalkyl; heteroaryl, optionally substituted by aryl, which may be optionally substituted by halogen; or taken together with the carbon atoms to which they are bound form a bridged bicyclic ring or a fused heterocycle;
**X** is CH or nitrogen;
**Y** is a bond, oxygen, (CH₂)ₘC**R⁴R⁵**(CH₂)ₙ, or N**R⁶**;
**m, n** are, independently, zero or 1;
**R⁴**, **R⁵** are, independently, hydrogen; CN; C₁-C₆ alkyl, optionally substituted by aryl; (CO)-aryl; aryl, optionally substituted by one or more substituents selected from C₁-C₆ alkyl; heterocyclyl or heteroaryl; or taken together with the carbon atom to which they are bound form a spirocycle; or **R⁴** taken together with the carbon atom to which it is bound and **R²** together with the carbon atom to which it is bound can form a fused heterocycle;
**R⁶** is hydrogen; C₁-C₆ alkyl, optionally substituted by aryl; aryl, optionally substituted by one or more substituents selected from halogen, CN, C₁-C₆ alkyl, C₁-C₆ alkoxy, C₁-C₆ haloalkyl, methanesulfonyl, or benzyloxy; heterocyclyl or heteroaryl; (CO)**R⁷**;
**R⁷** is hydrogen; aryl; C₁-C₆ alkyl;
and the pharmaceutically acceptable salts thereof, provided that, when the dotted line is an additional bond, then **Y** is C**R⁴R⁵,** wherein **R⁴** is as defined above and **R⁵ is** absent.

2. A compounds according to claim 1, wherein:
the dotted line is an optional additional bond;
**R¹** is hydrogen;
**R², R³** are, independently, hydrogen; C₁-C₃ alkyl, phenyl, naphthyl, or taken together with the carbon atoms to which they are bound form a bridged bicyclic ring or a fused heterocycle;
**X** is CH or nitrogen;
**Y** is a bond, (CH₂)ₘC**R⁴R⁵**(CH₂)ₙ, or N**R⁶;**
**m, n** are, independently, zero or 1;
**R⁴, R⁵** are, independently, hydrogen; CN; C₁-C₃ alkyl, optionally substituted by phenyl; (CO)-phenyl; phenyl or naphthyl; 6-membered heterocyclyl or heteroaryl, containing one or two heteroatoms selected from nitrogen or oxygen, optionally fused with one or more phenyl rings; or taken together with the carbon atom to which they are bound form a spirocycle; or **R⁴** taken together with the carbon atom to which it is bound and **R²** together with the carbon atom to which it is bound can form a fused heterocycle;
**R⁶** is hydrogen; C₁-C₃ alkyl, optionally substituted by phenyl; phenyl; 6-membered heterocyclyl or heteroaryl, containing one or two nitrogen heteroatoms, optionally fused with one or more phenyl rings; (CO)-C₁-C₃ alkyl; (CO)-phenyl;
and the pharmaceutically acceptable salts thereof, provided that, when the dotted line is an additional bond, then **Y** is C**R⁴R⁵,** wherein **R⁴** is as defined above and **R⁵** is absent.

3. A compounds according to claims 1-2, selected from:
(E)-N-hydroxy-3-{4-[(E)-3-(4-methyl-piperazin-1-yl)-3-oxo-propenyl]-phenyl}-acrylamide;
(E)-N-hydroxy-3-{3-[(E)-3-(4-methyl-piperazin-1-yl)-3-oxo-propenyl]-phenyl}-acrylamide;
(E)-N-hydroxy-3-{3-[(E)-3-oxo-3-(4-phenyl-piperazin-1-yl)-propenyl]-phenyl}-acrylamide;
(E)-3-[3-((E)-3-[1,4']bipiperidinyl-1'-yl-3-oxo-propenyl)-phenyl]-N-hydroxy-acrylamide;
(E)-N-hydroxy-3-{3-[(E)-3-oxo-3-(cis-3,4,5-trimethyl-piperazin-1-yl)-propenyl]-phenyl}-acrylamide;
(E)-3-{3-[(E)-3-((1S,4S)-5-methyl-2,5-diaza-bicyclo[2.2.1]hept-2-yl)-3-oxo-propenyl]-phenyl}-N-hydroxy-acrylamide;
(E)-N-hydroxy-3-{4-[(E)-3-oxo-3-(4-phenyl-piperazin-1-yl)-propenyl]-phenyl}-acrylamide;
(E)-3-[4-((E)-3-[1,4']bipiperidinyl-1'-yl-3-oxo-propenyl)-phenyl]-N-hydroxy-acrylamide;
(E)-N-hydroxy-3-{4-[(E)-3-oxo-3-(*cis*-3,4,5-trimethyl-piperazin-1-yl)-propenyl]-phenyl}-acrylamide;
(E)-N-hydroxy-3-{4-((E)-3-oxo-3-((1S,4S)-5-methyl-2,5-diaza-bicyclo[2.2.1]hept-2-yl)-propenyl]-phenyl}-acrylamide;
(E)-N-hydroxy-3-{5-[(E)-3-oxo-3-(4-phenyl-piperazin-1-yl)-propenyl]-pyridin-2-yl}-acrylamide;
(E)-N-hydroxy-3-{5-[(E)-3-(4-methyl-piperazin-1-yl)-3-oxo-propenyl]-pyridin-2-yl}-acrylamide;
(E)-N-hydroxy-3-{6-[(E)-3-oxo-3-(4-phenyl-piperazin-1-yl)-propenyl]-pyridin-2-yl}-acrylamide;
(E)-N-hydroxy-3-{6-[(E)-3-(4-methyl-piperazin-1-yl)-3-oxo-propenyl]-pyridin-2-yl}-acrylamide;
(E)-3-(6-{(E)-3-[4-(3-chloro-phenyl)-piperazin-1-yl]-3-oxo-propenyl}-pyridin-2-yl)-N-hydroxy-acrylamide;
(E)-3-{6-[(E)-3-(4-benzoyl-piperazin-1-yl)-3-oxo-propenyl]-pyridin-2-yl}-N-hydroxy-acrylamide hydrochloride;
(E)-3-(6-{(E)-3-[4-(2-chloro-phenyl)-piperazin-1-yl]-3-oxo-propenyl}-pyridin-2-yl)-N-hydroxy-acrylamide hydrochloride;
(E)-N-hydroxy-3-{6-[(E)-3-oxo-3-(4-phenyl-piperidin-1-yl)-propenyl]-pyridin-2-yl}-acrylamide hydrochloride;
(E)-N-hydroxy-3-{6-[(E)-3-oxo-3-(4-pyrimidin-2-yl-piperazin-1-yl)-propenyl]-pyridin-2-yl}-acrylamide hydrochloride;
(E)-3-(6-{(E)-3-[4-(4-chloro-phenyl)-piperazin-1-yl]-3-oxo-propenyl}-pyridin-2-yl)-N-hydroxy-acrylamide hydrochloride;
(E)-3-{6-[(E)-3-(4-benzyl-piperazin-1-yl)-3-oxo-propenyl]-pyridin-2-yl}-N-hydroxy-acrylamide hydrochloride;
(E)-N-hydroxy-3-{6-[(E)-3-oxo-3-(4-phenethyl-piperazin-1-yl)-propenyl]-pyridin-2-yl}-acrylamide hydrochloride;
(E)-3-{6-[(E)-3-(4-benzoyl-piperidin-1-yl)-3-oxo-propenyl]-pyridin-2-yl}-N-hydroxy-acrylamide hydrochloride;
(±)-(E)-N-hydroxy-3-{6-[(E)-3-oxo-3-(3-phenyl-piperidin-1-yl)-propenyl]-pyridin-2-yl}-acrylamide hydrochloride;
(E)-N-hydroxy-3-{6-[(E)-3-oxo-3-(4-phenyl-3,6-dihydro-2H-pyridin-1-yl)-propenyl]-pyridin-2-yl}-acrylamide hydrochloride;
(E)-3-(6-{(E)-3-[4-(2,6-dimethyl-phenyl)-piperidin-1-yl]-3-oxo-propenyl}-pyridin-2-yl)-N-hydroxy-acrylamide hydrochloride;
(E)-3-{6-[(E)-3-(4-cyano-4-phenyl-piperidin-1-yl)-3-oxo-propenyl]-pyridin-2-yl}-N-hydroxy-acrylamide hydrochloride;
(E)-N-hydroxy-3-{6-[(E)-3-oxo-3-(4-pyridin-2-yl-piperazin-1-yl)-propenyl]-pyridin-2-yll-acrylamide hydrochloride;
(E)-N-hydroxy-3-(6-{(E)-3-oxo-3-[4-(2-oxo-2,3-dihydro-benzoimidazol-1-yl)-piperidin-1-yl]-propenyl}-pyridin-2-yl)-acrylamide hydrochloride;
(E)-3-(6-{(E)-3-[4-(2,6-dimethyl-phenyl)-piperazin-1-yl]-3-oxo-propenyl}-pyridin-2-yl)-N-hydroxy-acrylamide hydrochloride;
(E)-N-hydroxy-3-{6-((E)-3-oxo-3-(4-oxo-1-phenyl-1,3,8-triaza-spiro[4.5]dec-8-yl)-propenyl]-pyridin-2-yl}-acrylamide hydrochloride;
(±)-(E)-N-hydroxy-3-{6-[(E)-3-oxo-3-(3-phenyl-piperazin-1-yl)-propenyl]-pyridin-2-yl}-acrylamide hydrochloride;
(E)-N-hydroxy-3-(6-{(E)-3-[4-(4-methoxy-phenyl)-piperazin-1-yl]-3-oxo-propenyl}-pyridin-2-yl)-acrylamide hydrochloride;
(E)-N-hydroxy-3-(6-{(E)-3-oxo-3-[4-(4-trifluoromethyl-phenyl)-piperazin-1-yl]-propenyl}-pyridin-2-yl)-acrylamide hydrochloride;
(E)-3-(6-{(E)-3-[4-(4-cyano-phenyl)-piperazin-1-yl]-3-oxo-propenyl}-pyridin-2-yl)-N-hydroxy-acrylamide hydrochloride;
(E)-3-(6-{(E)-3-[4-(4-fluoro-phenyl)-piperazin-1-yl]-3-oxo-propenyl}-pyridin-2-yl)-N-hydroxy-acrylamide hydrochloride;
(E)-3-(6-{(E)-3-[4-(4-bromo-phenyl)-piperazin-1-yl]-3-oxo-propenyl}-pyridin-2-yl)-N-hydroxy-acrylamide hydrochloride;
(E)-3-(6-{(E)-3-[4-(4-benzyloxy-phenyl)-piperazin-1-yl]-3-oxo-propenyl}-pyridin-2-yl)-N-hydroxy-acrylamide hydrochloride;
(E)-N-hydroxy-3-{6-[(E)-3-oxo-3-(4-pyridin-4-yl-piperazin-1-yl)-propenyl]-pyridin-2-yl}-acrylamide hydrochloride;
(E)-3-(6-{(E)-3-[5-(4-chloro-phenyl)-(1S,4S)-2,5-diaza-bicyclo[2.2.1]hept-2-yl]-3-oxo-propenyl}-pyridin-2-yl)-N-hydroxy-acrylamide trifluoroacetate;
(±)-(E)-N-hydroxy-3-{6-[(E)-3-oxo-3-(3-o-tolyl-piperidin-1-yl)-propenyl]-pyridin-2-yl}-acrylamide hydrochloride;
(±)-(E)-N-hydroxy-3-{6-[(E)-3-oxo-3-(3-m-tolyl-piperidin-1-yl)-propenyl]-pyridin-2-yl}-acrylamide hydrochloride;
(±)-(E)-N-hydroxy-3-{6-[(E)-3-(3-naphthalen-1-yl-piperidin-1-yl)-3-oxo-propenyl]-pyridin-2-yl}-acrylamide hydrochloride;
(E)-N-hydroxy-3-{6-[(E)-3-oxo-3-(1,3,4,5-tetrahydro-pyrido[4,3-b]indol-2-yl)-propenyl]-pyridin-2-yl}-acrylamide hydrochloride;
(±)-(E)-N-hydroxy-3-{6-[(E)-3-oxo-3-(3-p-tolyl-piperidin-1-yl)-propenyl]-pyridin-2-yl}-acrylamide hydrochloride;
(±)-(E)-N-hydroxy-3-{6-[(E)-3-(3-naphthalen-2-yl-piperidin-1-yl)-3-oxo-propenyl]-pyridin-2-yl}-acrylamide hydrochloride;
(±)-(E)-3-(6-{(E)-3-[3-(4-fluoro-phenyl)-piperidin-1-yl]-3-oxo-propenyl}-pyridin-2-yl)-N-hydroxy-acrylamide hydrochloride;
(E)-N-hydroxy-3-(6-{(E)-3-[4-(4-isopropyl-phenyl)-piperazin-1-yl]-3-oxo-propenyl}-pyridin-2-yl)-acrylamide hydrochloride;
(E)-3-(6-{(E)-3-[4-(4-tert-butyl-phenyl)-piperazin-1-yl]-3-oxo-propenyl}-pyridin-2-yl)-N-hydroxy-acrylamide hydrochloride;
(E)-N-hydroxy-3-(6-{(E)-3-[4-(4-methanesulfonyl-phenyl)-piperazin-1-yl]-3-oxo-propenyl}-pyridin-2-yl)-acrylamide hydrochloride;
(±)-(E)-N-hydroxy-3-{6-[(E)-3-oxo-3-(3-phenyl-pyrrolidin-1-yl)-propenyl]-pyridin-2-yl}-acrylamide hydrochloride;
(±)-(E)-N-hydroxy-3-{6-[(E)-3-oxo-3-(2-phenyl-piperidin-1-yl)-propenyl]-pyridin-2-yl}-acrylamide hydrochloride;
(±)-(E)-N-hydroxy-3-{6-[(E)-3-oxo-3-(4-phenyl-azepan-1-yl)-propenyl]-pyridin-2-yl}-acrylamide hydrochloride
(E)-3-{6-[(E)-3-(3,4-dihydro-2H-quinolin-1-yl)-3-oxo-propenyl]-pyridin-2-yl}-N-hydroxy-acrylamide trifluoroacetate;
(E)-N-hydroxy-3-{6-[(E)-3-oxo-3-(1,3,4,9-tetrahydro-beta-carbolin-2-yl)-propenyl]-pyridin-2-yl}-acrylamide hydrochloride;
(E)-3-{6-[(E)-3-(4-benzooxazol-2-yl-piperidin-1-yl)-3-oxo-propenyl]-pyridin-2-yl}-N-hydroxy-acrylamide hydrochloride;
(E)-3-{6-[(E)-3-(3,4-dihydro-1H-isoquinolin-2-yl)-3-oxo-propenyl]-pyridin-2-yl}-N-hydroxy-acrylamide hydrochloride;
(E)-3-(6-{(E)-3-[4-(1H-benzoimidazol-2-yl)-piperidin-1-yl]-3-oxo-propenyl}-pyridin-2-yl)-N-hydroxy-acrylamide hydrochloride;
(E)-3-(6-{(E)-3-[spiro[indene-1,4'-piperidine-1'-yl]-3-oxo-propenyl}-pyridin-2-yl)-N-hydroxy-acrylamide hydrochloride;
(E)-3-(6-{(E)-3-[spiro[2-benzofuran-1,4'-piperidine-1'-yl]-3-oxo-propenyl}-pyridin-2-yl)-N-hydroxy-acrylamide hydrochloride;
(E)-N-hydroxy-3-(6-{(E)-3-oxo-3-[4-(2-phenyl-benzoimidazol-1-yl)-piperidin-1-yl]-propenyl}-pyridin-2-yl)-acrylamide hydrochloride;
(±)-(E)-N-hydroxy-3-{6-[(E)-3-(4-methyl-3-phenyl-piperazin-1-yl)-3-oxo-propenyl]-pyridin-2-yl}-acrylamide hydrochloride
(±)-(E)-3-{6-[(E)-3-(4-ethyl-3-phenyl-piperazin-1-yl)-3-oxo-propenyl]-pyridin-2-yl}-N-hydroxy-acrylamide hydrochloride;
(±)-(E)-3-{6-[(E)-3-(4-benzyl-3-phenyl-piperazin-1-yl)-3-oxo-propenyl]-pyridin-2-yl}-N-hydroxy-acrylamide hydrochloride;
(±)-(E)-3-{6-[(E)-3-(4-acetyl-3-phenyl-piperazin-1-yl)-3-oxo-propenyl]-pyridin-2-yl}-N-hydroxy-acrylamide hydrochloride;
(±)-(E)-N-hydroxy-3-(6-{(E)-3-[3-(2-methoxy-phenyl)-piperidin-1-yl]-3-oxo-propenyl}-pyridin-2-yl)-acrylamide hydrochloride;
(±)-(E)-N-hydroxy-3-(6-{(E)-3-[3-(3-methoxy-phenyl)-piperidin-1-yl]-3-oxo-propenyl}-pyridin-2-yl)-acrylamide hydrochloride;
(±)-(E)-N-hydroxy-3-(6-{(E)-3-[3-(4-methoxy-phenyl)-piperidin-1-yl]-3-oxo-propenyl}-pyridin-2-yl)-acrylamide hydrochloride;
(±)-(E)-N-hydroxy-3-(6-{(E)-3-oxo-3-[3-(2-trifluoromethyl-phenyl)-piperidin-1-yl]-propenyl}-pyridin-2-yl)-acrylamide hydrochloride;
(±)-(E)-N-hydroxy-3-(6-{(E)-3-oxo-3-[3-(3-trifluoromethyl-phenyl)-piperidin-1-yl]-propenyl}-pyridin-2-yl)-acrylamide hydrochloride;
(±)-(E)-N-hydroxy-3-(6-{(E)-3-oxo-3-[3-(4-trifluoromethyl-phenyl)-piperidin-1-yl]-propenyl}-pyridin-2-yl)-acrylamide hydrochloride;
(E)-N-hydroxy-3-{6-[(E)-3-oxo-3-((S)-3-phenyl-piperidin-1-yl)-propenyl]-pyridin-2-yl}-acrylamide hydrochloride;
(E)-N-hydroxy-3-{6-[(E)-3-oxo-3-((R)-3-phenyl-piperidin-1-yl)-propenyl]-pyridin-2-yl}-acrylamide hydrochloride;
(E)-N-hydroxy-3-{3-[(E)-3-oxo-3-(4-phenyl-3,6-dihydro-2H-pyridin-1-yl)-propenyl]-phenyl}-acrylamide hydrochloride;
(E)-3-(3-{(E)-3-[4-(4-chloro-phenyl)-piperazin-1-yl]-3-oxo-propenyl}-phenyl)-N-hydroxy-acrylamide hydrochloride;
(E)-3-{3-[(E)-3-(4-benzoyl-piperidin-1-yl)-3-oxo-propenyl]-phenyl}-N-hydroxy-acrylamide;
(±)-(E)-N-hydroxy-3-{3-[(E)-3-oxo-3-(3-m-tolyl-piperidin-1-yl)-propenyl]-phenyl}-acrylamide;
(±)-(E)-N-hydroxy-3-{3-[(E)-3-oxo-3-(3-o-tolyl-piperidin-1-yl)-propenyl]-phenyl}-acrylamide;
(±)-(E)-N-hydroxy-3-{3-[(E)-3-oxo-3-(3-p-tolyl-piperidin-1-yl)-propenyl]-phenyl}-acrylamide;
(E)-N-hydroxy-3-{3-[(E)-3-oxo-3-((R)-3-phenyl-piperidin-1-yl)-propenyl]-phenyl}-acrylamide;
(E)-N-hydroxy-3-{3-[(E)-3-oxo-3-((S)-3-phenyl-piperidin-1-yl)-propenyl]-phenyl}-acrylamide;
(±)-(E)-N-hydroxy-3-{3-[(E)-3-oxo-3-(3-phenyl-piperidin-1-yl)-propenyl]-phenyl}-acrylamide;
(±)-(E)-3-(3-{(E)-3-[3-(4-fluoro-phenyl)-piperidin-1-yl]-3-oxo-propenyl}-phenyl)-N-hydroxy-acrylamide;
(E)-3-(3-{(E)-3-[4-(3-chloro-phenyl)-piperazin-1-yl]-3-oxo-propenyl}-phenyl)-N-hydroxy-acrylamide hydrochloride;
(E)-3-{3-[(E)-3-(4-benzyl-piperidin-1-yl)-3-oxo-propenyl]-phenyl}-N-hydroxy-acrylamide;
(E)-3-(3-{(E)-3-[4-(2-chloro-phenyl)-piperazin-1-yl]-3-oxo-propenyl}-phenyl)-N-hydroxy-acrylamide hydrochloride;
(E)-3-(3-{(E)-3-[4-(4-cyano-phenyl)-piperazin-1-yl]-3-oxo-propenyl}-phenyl)-N-hydroxy-acrylamide;
(E)-N-hydroxy-3-{3-[(E)-3-oxo-3-(5-phenyl-3,6-dihydro-2H-pyridin-1-yl)-propenyl]-phenyl}-acrylamide;
(E)-N-hydroxy-3-(3-((E)-3-oxo-3-(3-phenylpiperazin-1-yl)prop-1-enyl)phenyl)acrylamide;
(E)-N-hydroxy-3-(3-{(E)-3-oxo-3-[4-(4-trifluoromethyl-phenyl)-piperazin-1-yl]-propenyl}-phenyl)-acrylamide;
(E)-3-(6-{(E)-3-[3-(2-fluoro-phenyl)-piperidin-1-yl]-3-oxo-propenyl}-pyridin-2-yl)-N-hydroxy-acrylamide hydrochloride;
(E)-3-(6-{(E)-3-[3-(3-fluoro-phenyl)-piperidin-1-yl]-3-oxo-propenyl}-pyridin-2-yl)-N-hydroxy-acrylamide hydrochloride;
(E)-3-[6-((E)-3-{3-[3-(4-fluoro-phenyl)-1,2,4-oxadiazol-5-yl]-piperidin-1-yl}-3-oxo-propenyl)-pyridin-2-yl]-N-hydroxy-acrylamide hydrochloride;
(E)-N-hydroxy-3-{6-[(E)-3-oxo-3-(5-phenyl-3,6-dihydro-2H-pyridin-1-yl)-propenyl]-pyridin-2-yl}-acrylamide hydrochloride;
(E)-N-hydroxy-3-{6-[(E)-3-oxo-3-(6-phenyl-3,4-dihydro-1H-isoquinolin-2-yl)-propenyl]-pyridin-2-yl}-acrylamide trifluoro-acetate;
(E)-N-hydroxy-3-{6-[(E)-3-oxo-3-(2-phenyl-6,7-dihydro-4H-thiazolo[5,4-c]pyridin-5-yl)-propenyl]-pyridin-2-yl}-acrylamide hydrochloride;
(E)-N-hydroxy-3-{6-[(E)-3-oxo-3-(2-phenyl-6,7-dihydro-5H-thiazolo[5,4-b]pyridin-4-yl)-propenyl]-pyridin-2-yl}-acrylamide trifluoro-acetate.

4. A compound as defined in any of claims 1 to 3, for use as HDAC inhibitors.

5. A compound according to claim 4, for use in the treatment or prevention of diseases linked to the disregulation of histone deacetylase activity.

6. Pharmaceutical composition comprising one or more compounds of formula **(I)** as defined in any of claims 1 to 3.

7. Pharmaceutical composition according to claim 6, for use in the treatment or prevention of diseases linked to the disregulation of histone deacetylase activity.

8. Pharmaceutical composition according to claim 7, further containing additional active principles useful for treating said diseases.

9. Pharmaceutical composition according to any of claims 6 to 7, in the form of tablets, capsules, oral preparations, powders, granules, pills, injectable or infusible liquid solutions, suspensions, emulsions, suppositories, ointments, creams, lotions, gels, pastes, transdermal delivery devices.

10. Process for preparing a compound according to any of claims 1 to 3, comprising
a: subjecting a compound of formula **A1** 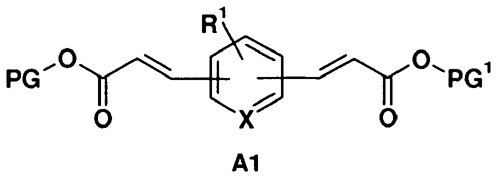 wherein **R¹, X** are as defined in claim 1 and **PG, PG¹** are protecting groups, to deprotection reactions;
b: treating the deprotected sites with suitable precursors of the moieties: 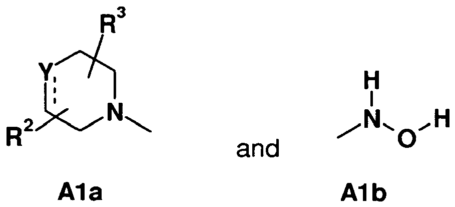 wherein the dotted line, **R², R³** and **Y** are as defined in claim1.

11. Process according to claim 10, wherein two independent deprotection reactions are performed, which may take place in any order, said process being performed as follows:
i) first deprotection (removing **PG** or **PG¹)**
ii) reaction with the first precursor (of moiety **A1a** or **A1b)**
iii) second deprotection (removing **PG¹** or **PG**)
iv) reaction with the second precursor (of moiety **A1b** or **A1a).**

12. Process according to any of claims 10 or 11, wherein **PG** and **PG¹** are independently a methyl or *tert*-butyl group

## Patentansprüche

1. Verbindung gemäß der Formel (I) 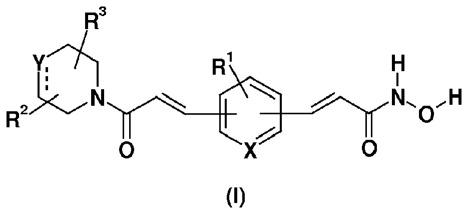 wobei:
**R¹** die gestrichelte Linie eine optionale zusätzliche Bindung ist, Wasserstoff ist,
**R², R³** unabhängig voneinander sind: Wasserstoff; C₁-C₆-Alkyl; Aryl, welches optional mit Halogen, C₁-C₆-Alkyl, C₁-C₆-Alkoxy oder C₁-C₆-Haloalkyl substituiert ist; Heteroaryl, welches optional mit Aryl substituiert ist, welches optional mit Halogen substituiert sein kann; oder zusammengenommen mit den Kohlenstoffatomen, an welche diese gebunden sind, einen überbrückten bicyclischen Ring oder einen fusionierten Heterocyclus ausbilden,
**X** CH oder Stickstoff ist,
**Y** eine Bindung, Sauerstoff, (CH₂)ₘC**R⁴R⁵**(CH₂)ₙ oder N**R⁶** ist,
**m,n** unabhängig voneinander null oder 1 sind,
**R⁴, R⁵** unabhängig voneinander sind: Wasserstoff; CN; C₁-C₆-Alkyl, welches optional mit Aryl substituiert ist; (CO)-Aryl; Aryl, welches optional mit einem oder mehreren Substituenten ausgewählt aus C₁-C₆-Alkyl substituiert ist; Heterocyclyl oder Heteroaryl; oder zusammengenommen mit dem Kohlenstoffatom, an welches diese gebunden sind, einen Spirocyclus bildet; oder **R⁴** zusammen mit dem Kohlenstoffatom, an welches dieser gebunden ist, und **R²** zusammen mit dem Kohlenstoffatom, an welches dieser gebunden ist, einen fusionierten Heterocyclus bildet,
**R⁶** Wasserstoff; C₁-C₆-Alkyl, welches optional mit Aryl substituiert ist; Aryl, welches optional mit einem oder mehreren Substituenten ausgewählt aus Halogen, CN, C₁-C₆-Alkyl, C₁-C₆-Alkoxy, C₁-C₆-Haloalkyl, Methansulfonyl oder Benzyloxy substituiert ist; Heterocyclyl oder Heteroaryl; (CO)**R⁷** ist,
**R⁷** Wasserstoff; Aryl; C₁-C₆-Alkyl ist,
und die pharmazeutisch akzeptablen Salze hiervon, mit der Maßgabe, dass, wenn die gestrichelte Linie eine zusätzliche Bindung ist, dann **Y CR⁴R⁵** ist, worin **R⁴** wie zuvor beschrieben definiert ist und **R⁵** abwesend ist.

2. Verbindung nach Anspruch 1, wobei:
die gestrichelte Linie eine optionale zusätzliche Bindung ist,
**R¹** Wasserstoff ist,
**R², R³** unabhängig voneinander sind: Wasserstoff; C₁-C₃-Alkyl; Phenyl; Naphthyl oder zusammen mit den Kohlenstoffatomen, an welche diese gebunden sind, einen überbrückten bicyclischen Ring oder einen fusionierten Heterocyclus bilden,
**X** CH oder Stickstoff ist,
**Y** eine Bindung, (CH₂) ₘC**R⁴R⁵**(CH₂)ₙ oder **NR⁶** ist,
**m,n** unabhängig voneinander null oder 1 sind,
**R⁴, R⁵** unabhängig voneinander sind: Wasserstoff; CN; C₁-C₃-Alkyl, welches optional mit Phenyl substituiert ist; (CO)-Phenyl; Phenyl oder Naphthyl; sechsgliedriger Heterocyclyl oder Heteroaryl, welcher ein oder zwei Heteroatome ausgewählt aus Stickstoff oder Sauerstoff enthält und optional mit einem oder mehreren Phenylringen fusioniert ist; oder zusammen mit dem Kohlenstoffatom, an welche diese gebunden sind, einen Spirocyclus ausbilden, oder **R⁴** zusammen mit dem Kohlenstoffatom, an welches dieser gebunden ist, und **R²** zusammen mit dem Kohlenstoffatom, an welches dieser gebunden ist, einen fusionierten Heterocyclus ausbilden kann,
**R⁶** Wasserstoff; C₁-C₃-Alkyl, welches optional mit Phenyl substituiert ist; Phenyl; 6-gliedriger Heterocyclyl oder Heteroaryl, welcher einen oder mehrere Stickstoffheteroatome enthält und optional mit einem oder mehreren Phenylringen fusioniert ist; (CO)- C₁-C₃-Alkyl; (CO)-Phenyl ist,
und die pharmazeutisch akzeptablen Salze hiervon, mit der Maßgabe, dass wenn die gestrichelte Linie eine zusätzliche Bindung ist, dann **Y CR⁴R⁵** ist, worin **R⁴** wie zuvor definiert ist und **R⁵** abwesend ist.

3. Verbindung nach Anspruch 1 oder 2 ausgewählt aus:
(E)-N-Hydroxy-3-{4-[(E)-3-(4-methyl-piperazin-1-yl)-3-oxo-propenyl]-phenyl}-acrylamid;
(E)-N-Hydroxy-3-{3-[(E)-3-(4-methyl-piperazin-1-yl)-3-oxo-propenyl]-phenyl}-acrylamid;
(E)-N-Hydroxy-3-{3-[(E)-3-oxo-3-(4-phenyl- piperazin-1-yl)propenyl]-phenyl}-acrylamid;
(E)-3[3-((E)-3-[1,4']Bipiperidinyl-1'-yl-3-oxo-propenyl)-phenyl]-N-hydroxy-acrylamid;
(E)-N-Hydroxy-3-{3-[(E)-3-oxo-3-(*cis*-3,4,5-trimethyl-piperazin-1-yl)-propenyl]-phenyl}-acrylamid;
(E)-3-{3-[(E)-3-((1S,4S)-5-Methyl-2,5-diaza-bicyclo[2.2.1]hept-2-yl)-3-oxo-propenyl]-phenyl}-N-hydroxy-acrylamid;
(E)-N-Hydroxy-3-{4-[(E)-3-oxo-3-(4-phenyl-piperazin-1-yl)-propenyl]-phenyl}-acrylamid;
(E)-3-[4-((E)-3-[1,4']Bipiperidinyl-1'-yl-3-oxo-propenyl)-phenyl]-N-hydroxy-acrylamid;
(E)-N-Hydroxy-3-{4-[(E)-3-oxo-3-(*cis*-3,4,5-trimethyl-piperazin-1-yl)-propenyl]-phenyl}- acrylamid;
(E)-N-Hydroxy-3-{4-[(E)-3-oxo-3-(1S,4S)-5-methyl-2,5-diaza-bicyclo[2.2.1]hept-2-yl)-propenyl]-phenyl}-acrylamid;
(E)-N-Hydroxy-3-{-[(E)-3-oxo-3-(4-phenyl-piperazin-1-yl)-propenyl]-pyridin-2-yl}-acrylamid;
(E)-N-Hydroxy-3-{5-[(E)-3-(4-methyl-piperazin-1-yl)-3-oxo-propenyl]-pyridin-2-yl}-acrylamid;
(E)-N-Hydroxy-3-{6-[(E)-3-oxo-3-(4-phenyl-piperazin-1-yl)-propenyl]-pyridin-2-yl}-acrylamid;
(E)-N-Hydroxy-3-{6-[(E)-3-(4-methyl-piperazin-1-yl)-3-oxo-propenyl]-pyridin-2-yl}-acrylamid;
(E)-3-(6-{(E)-3-[4-(3-Chloro-phenyl)-piperazin-1-yl]-3-oxo-propenyl}-pyridin-2-yl)-N-hydroxy-acrylamid;
(E)-3-{6-[(E)-3-(4-Benzoyl-piperazin-1-yl)-3-oxo-propenyl]-pyridin-2-yl}-N-hydroxy-acrylamidhydrochlorid;
(E)-3-(6-{(E)-3-[4-(2-Chloro-phenyl)-piperazin-1-yl]-3-oxo-propenyl}-pyridin-2-yl)-N-hydroxy-acrylamidhydrochlorid
(E)-N-Hydroxy-3-{6-[(E)-3-oxo-3-(4-phenyl-piperidin-1-yl)-propenyl]-pyridin-2-yl}-acrylamidhydrochlorid;
(E)-N-Hydroxy-3-{6-[(E)-3-oxo-3-(4-pyrimidin-2-yl-piperazin-1-yl)-propenyl]-pyridin-2-yl}-acrylamidhydrochlorid;
(E)-3-(6-{(E)-3-[4-(4-Chloro-phenyl)-piperazin-1-yl)-3-oxo-propenyl}-pyridin-2-yl)-N-hydroxy-acrylamidhydrochlorid;
(E)-3-{6-[(E)-3-(4-Benzyl-piperazin-1-yl)-3-oxo-propenyl]-pyridin-2-yl}-N-hydroxy-acrylamidhydrochlorid;
(E)-N-Hydroxy-3-{6-[(E)-3-oxo-3-(4-phenethyl-piperazin-1-yl)-propenyl]-pyridin-2-yl}-acrylamidhydrochlorid;
(E)-3-{6-[(E)-3-(4-Benzoyl-piperidin-1-yl)-3-oxo-propenyl]-pyridin-2-yl}-N-hydroxy-acrylamidhydrochlorid;
(±)-(E)-N-Hydroxy-3-{6-(E)-3-oxo-3-(3-phenyl-piperidin-1-yl)-propenyl]-pyridin-2-yl}-acrylamidhydrochlorid;
(E)-N-Hydroxy-3-{6-[(E)-3-oxo-3-(4-phenyl-3,6-dihydro-2H-pyridin-1-yl)-propenyl]-pyridin-2-yl}-acrylamidhydrochlorid:
(E)-3-(6-{(E)-3-[4-(2,6-Dimethyl-phenyl)-piperidin-1-yl]-3-oxo-propenyl}-pyridin-2-yl)-N-hydroxy-acrylamidhydrochlorid;
(E)-3-{6[(E)-3-(4-Cyano-4-phenyl-piperidin-1-yl)-3-oxo-propenyl]-pyridin-2-yl}-N-hydroxy-acrylamidhydrochlorid;
(E)-N-Hydroxy-3-{6-[(E)-3-oxo-3-(4-pyridin-2-yl-piperazin-1-yl)-propenyl]-pyridin-2-yl}-acrylamidhydrochlorid;
(E)-N-Hydroxy-3-(6-{(E)-3-oxo-3-[4-(2-oxo-2,3-dihydro-benzoimidazol-1-yl)-piperidin-1-yl]-propenyl}-pyridin-2-yl)-acrylamidhydrochlorid;
(E)-3-(6-{(E)-3-[4-(2,6-Dimethyl-phenyl)-piperazin-1-yl]-3-oxo-propenyl}-pyridin-2-yl)-N-hydroxy-acrylamidhydrochlorid;
(E)-N-Hydroxy-3-{6-[(E)-3-oxo-3-(4-oxo-1-phenyl-1,3,8-triaza-spiro[4, 5]dec-8-yl)-propenyl]-pyridin-2-yl}-acrylamidhydrochlorid;
(±)-(E)-N-Hydroxy-3-{6-[(E)-3-oxo-3-(3-phenyl-piperazin-1-yl)-propenyl]-pyridin-2-yl}-acrylamidhydrochlorid;
(E)-N-Hydroxy-3-(6-{(E)-3-[4-(4-methoxy-phenyl)-piperazin-1-yl]-3-oxo-propenyl}-pyridin2-yl)-acrylamidhydrochlorid;
(E)-N-Hydroxy-3-(6-{(E)-3-oxo-3-[4-(4-trifluoromethyl-phenyl)-piperazin-1-yl]-propenyl}-pyridin-2-yl)-acrylamidhydrochlorid;
(E)-3-(6-{(E)-3-[4-(4-Cyano-phenyl)-piperazin-1-yl]-3-oxo-propenyl}-pyridin-2-yl)-N-hydroxy-acrylamidhydrochlorid;
(E)-3-(6-{(E)-3-[4-(4-Fluoro-phenyl)-piperazin-1-yl]-3-oxo-propenyl}-pyridin-2-yl)-N-hydroxy-acrylamidhydrochlorid;
(E)-3-(6-{(E)-3-[4-(4-Bromo-phenyl)-piperazin-1-yl]-3-oxo-propenyl}-pyridin-2-yl)-N-hydroxy-acrylamidhydrochlorid;
(E)-3-(6-{(E)-3-(4-(4-Benzyloxy-phenyl)-piperazin-1-yl]-3-oxo-propenyl}-pyridin-2-yl)-N-hydroxy-acrylamidhydrochlorid;
(E)-N-Hydroxy-3-{6-[(E)-3-oxo-3-[4-pyridin-4-yl-piperazin-1-yl)-propenyl]-pyridirin-2-yl)-acrylamidhydrochlorid;
(E)-3-(6-{(E)-3-[5-(4-Chloro-phenyl)-(1S,4S)-2,5-diaza-bicyclo[2.2.1]hept-2-yl]-3-oxo-propenyl}-pyridin-2-yl)-N-hydroxy-acrylamidtrifluoroacetat;
(±)-(E)-N-Hydroxy-3-{6-[(E)-3-oxo-3-(3-o-tolyl-piperidin-1-yl)-propenyl]-pyridin-2-yl}-acrylamidhydrochlorid;
(±)-(E)-N-Hydroxy-3-{6-[(E)-3-oxo-3-(3-m-tolyl-piperidin-1-yl)-propenyl]-pyridin-2-yl}-acrylamidhydrochlorid;
(±)-(E)-N-Hydroxy-3-{6-[(E)-3-(3-naphthalen-1-yl-piperidin-1-yl)-3-oxo-propenyl]-pyridin-2-yl}-acrylamidhydrochlorid;
(E)-N-Hydroxy-3-{6-[(E)-3-oxo-3-(1,3,4,5-tetrahydro-pyrido[4,3-b]indol-2-yl)-propenyl]-pyridin-2-yl}-acrylamidhydrochlorid;
(±)-(E)-N-Hydroxy-3-{6-[(E)-3-oxo-3-(3-p-tolyl-piperidin-1-yl)-propenyl]-pyridin-2-yl}-acrylamidhydrochlorid;
(±)-(E)-N-Hydroxy-3-{6-[(E)-3-(3-naphthalen-2-yl-piperidin-1-yl)-3-oxo-propenyl]-pyridin-2-yl}-acrylamidhydrochlorid;
(±)-(E)-3-(6-{(E)-3-[3-(4-fluoro-phenyl)-piperidin-1-yl]-3-oxo-propenyl)-pyridin-2-yl)-N-hydroxy-acrylamidhydrochlorid;
(E)-N-Hydroxy-3-(6-{(E)-3-[4-(4-isopropyl-phenyl)-piperazin-1-yl]-3-oxo-propenyl}-pyridin-2-yl)-acrylamidhydrochlorid;
(E)-3-(6-{(E)-3-[4-(4-Tert-butyl-phenyl)-piperazin-1-yl]-3-oxo-propenyl}-pyridin-2-yl)-N-hydroxy-acrylamidhydrochlorid;
(E)-N-Hydroxy-3-(6-{(E)-3-[4-(4-methansulfonyl-phenyl)-piperazin-1-yl]-3-oxo-propenyl}-pyridin-2-yl)-acrylamidhydrochlorid;
(±)-(E)-N-Hydroxy-3-{6-[(E)-3-oxo-3-(3-phenyl-pyrrolidin-1-yl)-propenyl]-pyridin-2-yl}-acrylamidhydrochlorid;
(±)-(E)-N-Hydroxy-3-{6-[(E)-3-oxo-3-(2-phenyl-piperidin-1-yl)-propenyl]-pyridin-2-yl}-acrylamidhydrochlorid;
(±)-(E)-N-Hydroxy-3-{6-[(E)-3-oxo-3-(4-phenyl-azepan-1-yl)-propenyl]-pyridin-2-yl}-acrylamidhydrochlorid
(E)-3-{6-[(E)-3-(3,4-Dihydro-2H-chinolin-1-yl)-3-oxo-propenyl-pyridin-2-yl}-N-hydroxy-acrylamidtrifluoroacetat;
(E)-N-Hydroxy-3-{6-[(E)-3-oxo-3-(1,3,4,9-tetrahydro-beta-carbolin-2-yl)-propenyl-pyridin-2-yl)-acrylamidhydrochlorid;
(E)-3-{6-[(E)-3-(4-Benzooxazol-2-yl-piperidin-1-yl)-3-oxo-propenyl]-pyridin-2-yl}-N-hydroxy-acrylamidhydrochlorid;
(E)-3-{6-[(E)-3-(3,4-Dihydro-1H-isochinolin-2-yl)-3-oxo-propenyl]-pyridin-2-yl}-N-hydroxy-acrylamidhydrochlorid;
(E)-3-(6-{(E)-3-[4-(1H-Benzoimidazol-2-yl)-piperidin-1-yl]-3-oxo-propenyl} pyridin-2-yl)-N-hydroxy-acrylamidhydrochlorid;
(E)-3-(6-{(E)-3-[Spiro[inden-1,4'-piperidin-1'-yl]-3-oxo-propenyl}pyridin-2-yl)-N-hydroxy-acrylamidhydrochlorid;
(E)-3-(6-{(E)-3-[Spiro[2-benzofuran-1,4'-piperidin-1'-yl]-3-oxo-propenyl}-pyridin-2-yl)-N-hydroxy-acrylamidhydrochlorid;
(E)-N-Hydroxy-3-(6-{(E)-3-oxo-3-(4-(2-phenyl-benzoimidazol-1-yl]-piperidin-1-yl]-propenyl}-pyridin-2-yl)-acrylamidhydrochlorid;
(±)-(E)-N-Hydroxy-3-{6-[(E)-3-(4-methyl-3-phenyl-piperazin-1-yl)-3-oxo-propenyl]-pyridin-2-yl}-acrylamidhydrochlorid
(±)-(E)-3-{6-[(E)-3-(4-Ethyl-3-phenyl-piperazin-1-yl)-3-oxo-propenyl]-pyridin-2-yl-N-hydroxy-acrylamidhydrochlorid;
(±)-(E)-3-{6-[(E)-3-(4-Benzyl-3-phenyl-piperazin-1-yl)-3-oxo-propenyl]-pyridin-2-yl}-N-hydroxy-acrylamidhydrochlorid;
(±)-(E)-3-{6-[(E)-3-(4-Acetyl-3-phenyl-piperazin-1-yl)-3-oxo-propenyl]-pyridin-2-yl}-N-hydroxy-acrylamidhydrochlorid;
(±)-(E)-N-Hydroxy-3-(6-{(E)-3-[3-(2-methoxy-phenyl)-piperidin-1-yl]-3-oxo-propenyl}-pyridin-2-yl)-acrylamidhydrochlorid;
(±)-(E)-N-Hydroxy-3-(6-{(E)-3-[3-(3-methoxy-phenyl)-piperidin-1-yl]-3-oxo-propenyl}-pyridin-2-yl)-acryamidhydrochlorid;
(±)-(E)-N-Hydroxy-3-(6-{(E)-3-[3-(4-methoxy-phenyl)-piperidin-1-yl]-3-oxo-propenyl}-pyridin-2-yl)-acrylamidhydrochlorid;
(±)-(E)-N-Hydroxy-3-(6-{(E)-3-oxo-3-[3-(2-trifluoromethyl-phenyl)-piperidin-1-yl]-propenyl}-pyridin-2-yl)-acrylamidhydrochlorid;
(±)-(E)-N-Hydroxy-3-(6-{(E)-3-oxo-3-[3-(3-trifluoromethyl-phenyl)-piperidin-1-yl]-propenyl}-pyridin-2-yl)-acrylamidhydrochlorid;
(±)-(E)-N-Hydroxy-3-(6-{(E)-3-oxo-3-[3-(4-trifluoromethyl-phenyl)-piperidin-1-yl]-propenyl}-pyridin-2-yl)-acrylamidhydrochlorid;
(E)-N-Hydroxy-3-{6-[(E)-3-oxo-3-((S)-3-phenyl-piperidin-1-yl)-propenyl]-pyridin-2-yl}-acrylamidhydrochlorid;
(E)-N-Hydroxy-3-{6-[(E)-3-oxo-3-((R)-3-phenyl-piperidin-1-yl)-propenyl]-pyridin-2-yl}-acrylamidhydrochlorid;
(E)-N-Hydroxy-3-{3-[(E)-3-oxo-3-(4-phenyl-3,6-dihydro-2H-pyridin-1-yl)-propenyl]-phenyl)-acrylamidhydrochlorid;
(E)-3-(3-{(E)-3-[4-(4-Chloro-phenyl)-piperazin-1-yl]-3-oxo-propenyl}-phenyl)-N-hydroxy-acrylamidhydrochlorid;
(E)-3-{3-[(E)-3-(4-Benzoyl-piperidin-1-yl)-3-oxo-propenyl]-phenyl)-N-hydroxy-acrylamid;
(±)-(E)-N-Hydroxy-3-{3-[(E)-3-oxo-3-(3-m-tolyl-piperidin-1-yl)-propenyl]phenyl}-acrylamid;
(±)-(E)-N-Hydroxy-3-{3-[(E)-3-oxo-3-(3-o-tolyl-piperidin-1-yl)-propenyl]-phenyl}-acrylamid;
(±)-(E)-N-Hydroxy-3-{3-[(E)-3-oxo-3-(3-p-tolyl-piperidin-1-yl)-propenyl]-phenyl}-acrylamid;
(E)-N-Hydroxy-3-{3-[(E)-3-oxo-3-((R)-3-phenyl-piperidin-1-yl)-propenyl]-phenyl}-acrylamid;
(E)-N-Hydroxy-3-{3-[(E)-3-oxo-3-((S)-3-phenyl-piperidin-1-yl)-propenyl]-phenyl}-acrylamid;
(±)-(E)-N-Hydroxy-3-{3-[(E)-3-oxo-3-(3-phenyl-piperidin-1-yl)-propenyl]-phenyl}-acrylamid;
(±)-(E)-3-(3-{(E)-3-[3-(4-Fluoro-phenyl)-piperidin-1-yl]-3-oxo-propenyl}-phenyl)-N-hydroxy-acrylamid;
(E)-3-(3-{(E)-3-[4-(3-Chloro-phenyl)-piperazin-1yl]-3-oxo-propenyl}-phenyl)-N-hydroxy-acrylamidhydrochlorid;
(E)-3-(3-{(E)-3-(4-Benzyl-piperidin-1-yl)-3-oxo-propenyl]-phenyl}-N-hydroxy-acrylamid;
(E)-3-(3-{(E)-3-[4-(2-Chloro-phenyl)-piperazin-1-yl]-3-oxo-propenyl}-phenyl)-N-hydroxy-acrylamidhydrochlorid;
(E)-3-(3-{(E)-3-[4-(4-Cyano-phenyl)-piperazin-1-yl]-3oxo-propenyl}-phenyl)-N-hydroxy-acrylamid;
(E)-N-Hydroxy-3-{3-[(E)-3-oxo-3-(5-phenyl-3,6-dihydro-2H-pyridin-1-yl)-propenyl]-phenyl}-acrylamid;
(E)-N-Hydroxy-3-(3-((E)-3-oxo-3-(3-phenylpiperazin-1yl)-prop-1-enyl)phenyl)acrylamid;
(E)-N-Hydroxy-3-(3-{(E)-3-oxo-3-[4-(4-trifluoromethyl-phenyl)-piperazin1-1yl]-propenyl}-phenyl)-acrylamid;
(E)-3-(6-{(E)-3-[3-(2-Fluoro-phenyl)-piperidin-1-yl]-3-oxo-propenyl}-pyridin-2-yl)-N-hydroxy-acrylamidhydrochlorid;
(E)-3-(6-{(E)-3-[3-(3-Fluoro-phenyl)-piperidin-1-yl]-3-oxo-propenyl}-pyridin-2-yl)-N-hydroxy-acrylamidhydrochlorid;
(E)-3-[6-((E)-3-{3-[3-(4-Fluoro-phenyl)-1,2,4-oxadiazol-5-yl]-piperidin1-1-yl)-3-oxo-propenyl)-pyridin-2-yl]-N-hydroxy-acrylamidhydrochlorid;
(E)-N-Hydroxy-3-{6-[(E)-3-oxo-3-(5-phenyl-3,6-dihydro-2H-pyridin-1-yl)-propenyl]-pyridin-2-yl}-acrylamidhydrochlorid;
(E)-N-Hydroxy-3-{6-[(E)-3-oxo-3-(6-phenyl-3,4-dihydro-1H-isochinolin-2-yl)-propenyl]-pyridin-2-yl}-acrylamidtrifluoroacetat;
(E)-N-Hydroxy-3-{6-[(E)-3-oxo-3-(2-phenyl-6,7-dihydro-4H-thiazolo-[5,4-c]pyridin-5-yl)-propenyl]-pyridin-2-yl)-acrylamidhydrochlorid;
(E)-N-Hydroxy-3-{6-[(E)-3-oxo-3-(2-phenyl-6,7-dihydro-5H-thiazolo-[5,4-b]pyridin-4-yl)-propenyl]-pyridin-2-yl)-acrylamidhydrochlorid;

4. Verbindung nach einem der Ansprüche 1 bis 3 zur Verwendung als HDAC-Inhibitoren.

5. Verbindung nach Anspruch 4 zur Verwendung bei der Behandlung oder Prävention von Krankheiten, welche mit der Disregulation von Histon-Deacetylase-Aktivität verbunden ist.

6. Pharmazeutische Zusammensetzung enthaltend eine oder mehre Verbindungen der Formal (I) nach einem der Ansprüche 1 bis 3.

7. Pharmazeutische Zusammensetzung nach Anspruch 6 zur Verwendung bei der Behandlung oder Prävention von Krankheiten, welche mit der Disregulation von Histon-Deacetylase-Aktivität verbunden sind.

8. Pharmazeutische Zusammensetzung nach Anspruch 7, welche des Weiteren aktive Bestandteile enthält, welche für die Behandlung der Krankheiten nützlich sind.

9. Pharmazeutischen Zusammensetzung nach einem der Ansprüche 6 bis 7 in der Form von Tabletten, Kapseln, oralen Präparationen, Pulvern, Granulaten, Pillen, injizierbaren oder unauflösbaren flüssigen Lösungen, Suspensionen, Emulsionen, Suppositorien, Salben, Cremes, Lotionen, Gelen, Pasten, transdermalen Zuführvorrichtungen.

10. Verfahren zum Herstellen einer Verbindung nach einem der Ansprüche 1 bis 3 umfassend:
a: Unterwerfen einer Verbindung der Formel **A1** 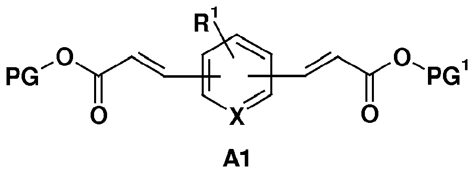 worin **R¹, X** wie in dem Patentanspruch 1 definiert sind und **PG, PG¹** Schutzgruppen für Entschützungsreaktionen sind,
b:Behandeln der entschützten Stellen mit geeigneten Vorläufern der Reste: 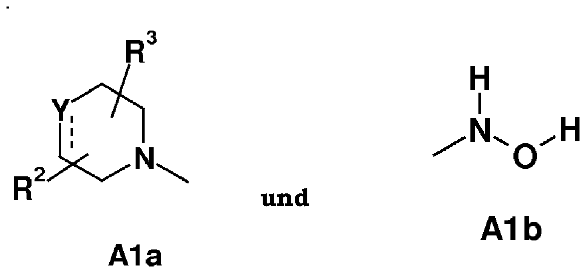 worin die gestrichelte Linie, **R², R³** und **Y** wie in dem Patentanspruch 1 definiert sind.

11. Verfahren nach Anspruch 10, wobei zwei unabhängige Entschützungsreaktionen, welche in jeder Reihenfolge stattfinden können, durchgeführt werden, wobei das Verfahren wie folgt durchgeführt wird:
i) erste Entschützung (Entfernen von **PG** oder **PG¹),**
ii) Reaktion mit dem ersten Vorläufer (von dem Rest **A1a** oder **Alb),**
iii) zweite Entschützung (Entfernen von **PG¹** oder **PG)**
iv) Reaktion mit dem zweiten Vorläufer (von dem Rest **A1b** oder **A1a)**.

12. Verfahren nach einem der Ansprüche 10 oder 11, wobei **PG** oder **PG¹** unabhängig voneinander eine Methyl- oder Tert-butyl-Gruppe sind.

## Revendications

1. Composé de formule (I) 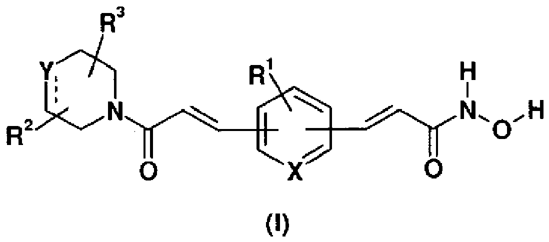 dans laquelle :
la ligne pointillée est une liaison supplémentaire facultative ;
R¹ est un atome d'hydrogène ;
R², R³ sont indépendamment un atome d'hydrogène ; un groupe alkyle en C₁ à C_{6;} un groupe aryle facultativement substitué par un atome d'halogène, un groupe alkyle en C₁ à C₆, un groupe alcoxy en C₁ à C₆, ou un groupe halogénoalkyle en C₁ à C_{6;} un groupe hétéroaryle facultativement substitué par un groupe aryle, qui peut être facultativement substitué par un atome d'halogène ; ou ensemble avec les atomes de carbone auxquels ils sont liés forment un cycle bicyclique ponté ou un hétérocycle condensé ;
X est un groupe CH ou un atome d'azote ;
Y est une liaison, un atome d'oxygène, un groupe (CH₂)ₘCR⁴R⁵(CH₂)ₙ ou un groupe NR^{6 ;}
m, n sont indépendamment 0 ou 1 ;
R⁴, R⁵ sont indépendamment un atome d'hydrogène ; un groupe CN ; un groupe alkyle en C₁ à C₆ facultativement substitué par un groupe aryle ; un groupe (CO)-aryle ; un groupe aryle facultativement substitué par un ou plusieurs substituants sélectionnés parmi un groupe alkyle en C₁ à C₆; un groupe hétérocyclyle ou hétéroaryle ; ou ensemble avec l'atome de carbone auquel ils sont liés forment un spirocycle ; ou R⁴ ensemble avec l'atome de carbone auquel il est lié et R² ensemble avec l'atome de carbone auquel il est lié peuvent former un hétérocycle condensé ;
R⁶ est un atome d'hydrogène ; un groupe alkyle en C₁ à C₆ facultativement substitué par un groupe aryle ; un groupe aryle facultativement substitué par un ou plusieurs substituants sélectionnés parmi un atome d'halogène, un groupe CN, un groupe alkyle en C₁ à C₆, un groupe alcoxy en C₁ à C₆, un groupe halogénoalkyle en C₁ à C₆, un groupe méthanesulfonyle ou un groupe benzyloxy; un groupe hétérocyclyle ou hétéroaryle ; un groupe (CO)R^{7 ;}
R⁷ est un atome d'hydrogène ; un groupe aryle ; un groupe alkyle en C₁ à C₆ ;
et les sels pharmaceutiquement acceptables de celui-ci, à condition que, lorsque la ligne pointillée est une liaison supplémentaire, alors Y est un groupe CR⁴R⁵ où R⁴ est tel que défini ci-dessus et R⁵ est absent.

2. Composé selon la revendication 1, dans lequel :
la ligne pointillée est une liaison supplémentaire facultative ;
R¹est un atome d'hydrogène ;
R², R³ sont indépendamment un atome d'hydrogène ; un groupe alkyle en C₁ à C₃; un groupe phényle, naphtyle ; ou ensemble avec les atomes de carbone auxquels ils sont liés forment un cycle bicyclique ponté ou un hétérocycle condensé ;
X est un groupe CH ou un atome d'azote ;
Y est une liaison, un groupe (CH₂)ₘCR⁴R⁵(CH₂)ₙ ou un groupe NR^{6 ;}
m, n sont indépendamment 0 ou 1 ;
R⁴, R⁵ sont indépendamment un atome d'hydrogène ; un groupe CN ; un groupe alkyle en C₁ à C₃ facultativement substitué par un groupe phényle ; un groupe (CO)-phényle ; un groupe phényle ou naphtyle; un groupe hétérocyclyle ou hétéroaryle de 6 éléments contenant un ou deux hétéroatomes sélectionnés parmi un atome d'azote ou un atome d'oxygène, facultativement condensé avec un ou plusieurs cycles phényle ; ou ensemble avec l'atome de carbone auquel ils sont liés forment un spirocycle ; ou R⁴ ensemble avec l'atome de carbone auquel il est lité et R² ensemble avec l'atome de carbone auquel il est lié peuvent former un hétérocycle condensé ;
R⁶ est un atome d'hydrogène ; un groupe alkyle en C₁ à C₃ facultativement substitué par un groupe phényle ; un groupe phényle ; un groupe hétérocyclyle ou hétéroaryle de 6 éléments contenant un ou deux hétéroatomes sélectionnés parmi un atome d'azote ou un atome d'oxygène, facultativement condensé avec un ou plusieurs cycles phényle ; un groupe (CO)-alkyle en C₁ à C₃; un groupe (CO)-phényle ;
et les sels pharmaceutiquement acceptables de celui-ci, à condition que, lorsque la ligne pointillée est une liaison supplémentaire, alors Y est un groupe CR⁴R⁵ où R⁴ est tel que défini ci-dessus et R⁵ est absent.

3. Composé selon la revendication 1 ou 2, sélectionné parmi :
(E)-N-hydroxy-3-{4-[(E)-3-(4-méthyl-pipérazin-1-yl)-3-oxo-propényl]-phényl}-acrylamide ;
(E)-N-hydroxy-3-{3-[(E)-3-(4-méthyl-pipérazin-1-yl)-3-oxo-propényl]-phényl}-acrylamide ;
(E)-N-hydroxy-3-{3-[(E)-3-oxo-3-(4-phényl-pipérazin-1-yl)-propényl]-phényl}-acrylamide ;
(E)-3-[3-((E)-3-[1,4']bipipéridinyl-1'-yl-3-oxo-propényl)-phényl]-N-hydroxy-acrylamide ;
(E)-N-hydroxy-3-{3-[(E)-3-oxo-3-(*cis*-3,4,5-triméthyl-pipérazin-1-yl)-propényl]-phényl}-acrylamide;
(E)-3-{3-[(E)-3-((1S,4S)-5-méthyl-2,5-diaza-bicyclo[2.2.1]hept-2-yl)-3-oxo-propényl]-phényl}-N-hydroxy-acrylamide ;
(E)-N-hydroxy-3-{4-[(E)-3-oxo-3-(4-phényl-pipérazin-1-yl)-propényl]-phényl}-acrylamide ;
(E)-3-[4-((E)-3-[1,4']bipipéridinyl-1'-yl-3-oxo-propényl)-phényl]-N-hydroxy-acrylamide ;
(E)-N-hydroxy-3-{4-[(E)-3-oxo-3-(*cis*-3,4,5-triméthyl-pipérazin-1-yl)-propényl]-phényl}-acrylamide ;
(E)-N-hydroxy-3-{4-[(E)-3-oxo-3-((1S,4S)-5-méthyl-2,5-diaza-bicyclo[2.2.1]hept-2-yl)-propényl]-phényl}-acrylamide ;
(E)-N-hydroxy-3-{5-[(E)-3-oxo-3-(4-phényl-pipérazin-1-yl)-propényl]-pyridin-2-yl}-acrylamide ;
(E)-N-hydroxy-3-{5-[(E)-3-(4-méthyl-pipérazin-1-yl)-3-oxo-propényl]-pyridin-2-yl}-acrylamide ;
(E)-N-hydroxy-3-{6-[(E)-3-oxo-3-(4-phényl-pipérazin-1-yl)-propényl]-pyridin-2-yl}-acrylamide ;
(E)-N-hydroxy-3-{6-[(E)-3-(4-méthyl-pipérazin-1-yl)-3-oxo-propényl]-pyridin-2-yl}-acrylamide ;
(E)-3-(6-{(E)-3-[4-(3-chloro-phényl)-pipérazin-1-yl]-3-oxo-propényl}-pyridin-2-yl)-N-hydroxy-acrylamide ;
chlorhydrate de (E)-3-{6-[(E)-3-(4-benzoyl-pipérazin-1-yl)-3-oxo-propényl]-pyridin-2-yl}-N-hydroxy-acrylamide ;
chlorhydrate de (E)-3-(6-{(E)-3-[4-(2-chloro-phényl)-pipérazin-1-yl]-3-oxo-propényl}-pyridin-2-yl)-N-hydroxy-acrylamide ;
chlorhydrate de (E)-N-hydroxy-3-{6-[(E)-3-oxo-3-(4-phényl-pipéridin-1-yl)-propényl]-pyridin-2-yl}-acrylamide ;
chlorhydrate de (E)-N-hydroxy-3-{6-[(E)-3-oxo-3-(4-pyrimidin-2-yl-pipérazin-1-yl)-propényl]-pyridin-2-yl}-acrylamide ;
chlorhydrate de (E)-3-(6-{(E)-3-[4-(4-chloro-phényl)-pipérazin-1-yl]-3-oxo-propényl}-pyridin-2-yl)-N-hydroxy-acrylamide ;
chlorhydrate de (E)-3-{6-[(E)-3-(4-benzyl-pipérazin-1-yl)-3-oxo-propényl]-pyridin-2-yl}-N-hydroxy-acrylamide ;
chlorhydrate de (E)-N-hydroxy-3-{6-[(E)-3-oxo-3-(4-phénéthyl-pipérazin-1-yl)-propényl]-pyridin-2-yl}-acrylamide ;
chlorhydrate de (E)-3-{6-[(E)-3-(4-benzoyl-pipéridin-1-yl)-3-oxo-propényl]-pyridin-2-yl}-N-hydroxy-acrylamide ;
chlorhydrate de (±)-(E)-N-hydroxy-3-{6-[(E)-3-oxo-3-(3-phényl-pipéridin-1-yl)-propényl]-pyridin-2-yl}-acrylamide ;
chlorhydrate de (E)-N-hydroxy-3-{6-[(E)-3-oxo-3-(4-phényl-3,6-dihydro-2H-pyridin-1-yl)-propényl]-pyridin-2-yl}-acrylamide ;
chlorhydrate de (E)-3-(6-{(E)-3-[4-(2,6-diméthyl-phényl)-pipéridin-1-yl]-3-oxo-propényl}-pyridin-2-yl)-N-hydroxy-acrylamide ;
chlorhydrate de (E)-3-{6-[(E)-3-(4-cyano-4-phényl-pipéridin-1-yl)-3-oxo-propényl]-pyridin-2-yl}-N-hydroxy-acrylamide ;
chlorhydrate de (E)-N-hydroxy-3-{6-[(E)-3-oxo-3-(4-pyridin-2-yl-pipérazin-1-yl)-propényl]-pyridin-2-yl}-acrylamide ;
chlorhydrate de (E)-N-hydroxy-3-(6-{(E)-3-oxo-3-[4-(2-oxo-2,3-dihydro-benzoimidazol-1-yl)-pipéridin-1-yl]-propényl}-pyridin-2-yl)-acrylamide ;
chlorhydrate de (E)-3-(6-{(E)-3-[4-(2,6-diméthyl-phényl)-pipérazin-1-yl]-3-oxo-propényl}-pyridin-2-yl)-N-hydroxy-acrylamide ;
chlorhydrate de (E)-N-hydroxy-3-{6-[(E)-3-oxo-3-(4-oxo-1-phényl-1,3,8-triaza-spiro[4.5]dec-8-yl)-propényl]-pyridin-2-yl}-acrylamide ;
chlorhydrate de (±)-(E)-N-hydroxy-3-{6-[(E)-3-oxo-3-(3-phényl-pipérazin-1-yl)-propényl]-pyridin-2-yl}-acrylamide ;
chlorhydrate de (E)-N-hydroxy-3-(6-{(E)-3-[4-(4-méthoxy-phényl)-pipérazin-1-yl]-3-oxo-propényl}-pyridin-2-yl)-acrylamide ;
chlorhydrate de (E)-N-hydroxy-3-(6-{(E)-3-oxo-3-[4-(4-trifluorométhyl-phényl)-pipérazin-1-yl]-propényl}-pyridin-2-yl)-acrylamide ;
chlorhydrate de (E)-3-(6-{(E)-3-[4-(4-cyano-phényl)-pipérazin-1-yl]-3-oxo-propényl}-pyridin-2-yl)-N-hydroxy-acrylamide ;
chlorhydrate de (E)-3-(6-{(E)-3-[4-(4-fluoro-phényl)-pipérazin-1-yl]-3-oxo-propényl}-pyridin-2-yl)-N-hydroxy-acrylamide ;
chlorhydrate de (E)-3-(6-{(E)-3-[4-(4-bromo-phényl)-pipérazin-1-yl]-3-oxo-propényl}-pyridin-2-yl)-N-hydroxy-acrylamide ;
chlorhydrate de (E)-3-(6-{(E)-3-[4-(4-benzyloxy-phényl)-pipérazin-1-yl]-3-oxo-propényl}-pyridin-2-yl)-N-hydroxy-acrylamide ;
chlorhydrate de (E)-N-hydroxy-3-{6-[(E)-3-oxo-3-(4-pyridin-4-yl-pipérazin-1-yl)-propényl]-pyridin-2-yl}-acrylamide ;
trifluoroacétate de (E)-3-(6-{(E)-3-[5-(4-chloro-phényl)-(1S,4S)-2,5-diaza-bicyclo[2.2.1]hept-2-yl]-3-oxo-propényl}-pyridin-2-yl)-N-hydroxy-acrylamide ;
chlorhydrate de (±)-(E)-N-hydroxy-3-{6-[(E)-3-oxo-3-(3-o-tolyl-pipéridin-1-yl)-propényl]-pyridin-2-yl}-acrylamide ;
chlorhydrate de (±)-(E)-N-hydroxy-3-{6-[(E)-3-oxo-3-(3-m-tolyl-pipéridin-1-yl)-propényl]-pyridin-2-yl}-acrylamide ;
chlorhydrate de (±)-(E)-N-hydroxy-3-{6-[(E)-3-(3-naphtalén-1-yl-pipéridin-1-yl)-3-oxo-propényl]-pyridin-2-yl}-acrylamide ;
chlorhydrate de (E)-N-hydroxy-3-{6-[(E)-3-oxo-3-(1,3,4,5-tétrahydro-pyrido[4,3-b]indol-2-yl)-propényl]-pyridin-2-yl}-acrylamide ;
chlorhydrate de (±)-(E)-N-hydroxy-3-{6-[(E)-3-oxo-3-(3-p-tolyl-pipéridin-1-yl)-propényl]-pyridin-2-yl}-acrylamide ;
chlorhydrate de (±)-(E)-N-hydroxy-3-{6-[(E)-3-(3-naphtalén-2-yl-pipéridin-1-yl)-3-oxo-propényl]-pyridin-2-yl}-acrylamide ;
chlorhydrate de (±)-(E)-3-(6-{(E)-3-[3-(4-fluoro-phényl)-pipéridin-1-yl]-3-oxo-propényl}-pyridin-2-yl)-N-hydroxy-acrylamide ;
chlorhydrate de (E)-N-hydroxy-3-(6-{(E)-3-[4-(4-isopropyl-phényl)-pipérazin-1-yl]-3-oxo-propényl}-pyridin-2-yl)-acrylamide ;
chlorhydrate de (E)-3-(6-{(E)-3-[4-(4-tert-butyl-phényl)-pipérazin-1-yl]-3-oxo-propényl}-pyridin-2-yl)-N-hydroxy-acrylamide ;
chlorhydrate de (E)-N-hydroxy-3-(6-{(E)-3-[4-(4-méthanesulfonyl-phényl)-pipérazin-1-yl]-3-oxo-propényl}-pyridin-2-yl)-acrylamide ;
chlorhydrate de (±)-(E)-N-hydroxy-3-{6-[(E)-3-oxo-3-(3-phényl-pyrrolidin-1-yl)-propényl]-pyridin-2-yl}-acrylamide ;
chlorhydrate de (±)-(E)-N-hydroxy-3-{6-[(E)-3-oxo-3-(2-phényl-pipéridin-1-yl)-propényl]-pyridin-2-yl}-acrylamide ;
Chlorhydrate de (±)-(E)-N-hydroxy-3-{6-[(E)-3-oxo-3-(4-phényl-azépan-1-yl)-propényl]-pyridin-2-yl}-acrylamide ;
trifluoroacétate de (E)-3-{6-[(E)-3-(3,4-dihydro-2H-quinoléin-1-yl)-3-oxo-propényl]-pyridin-2-yl}-N-hydroxy-acrylamide trifluoroacétate ;
chlorhydrate de (E)-N-hydroxy-3-{6-[(E)-3-oxo-3-(1,3,4,9-tétrahydro-bêta-carbolin-2-yl)-propényl]-pyridin-2-yl}-acrylamide ;
chlorhydrate de (E)-3-{6-[(E)-3-(4-benzooxazol-2-yl-pipéridin-1-yl)-3-oxo-propényl]-pyridin-2-yl}-N-hydroxy-acrylamide ;
chlorhydrate de (E)-3-{6-[(E)-3-(3,4-dihydro-1H-isoquinoléin-2-yl)-3-oxo-propényl]-pyridin-2-yl}-N-hydroxy-acrylamide ;
chlorhydrate de (E)-3-(6-{(E)-3-[4-(1H-benzoimidazol-2-yl)-pipéridin-1-yl]-3-oxo-propényl}-pyridin-2-yl)-N-hydroxy-acrylamide ;
chlorhydrate de (E)-3-(6-{(E)-3-[spiro[indène-1,4'-pipéridine-1'-yl]-3-oxo-propényl}-pyridin-2-yl)-N-hydroxy-acrylamide ;
chlorhydrate de (E)-3-(6-{(E)-3-[spiro[2-benzofuran-1,4'-pipéridine-1'-yl]-3-oxo-propényl}-pyridin-2-yl)-N-hydroxy-acrylamide ;
chlorhydrate de (E)-N-hydroxy-3-(6-{(E)-3-oxo-3-[4-(2-phényl-benzoimidazol-1-yl)-pipéridin-1-yl]-propényl}-pyridin-2-yl)-acrylamide ;
chlorhydrate de (±)-(E)-N-hydroxy-3-{6-[(E)-3-(4-méthyl-3-phényl-pipérazin-1-yl)-3-oxo-propényl]-pyridin-2-yl}-acrylamide ;
chlorhydrate de (±)-(E)-3-{6-[(E)-3-(4-éthyl-3-phényl-pipérazin-1-yl)-3-oxo-propényl]-pyridin-2-yl}-N-hydroxy-acrylamide ;
chlorhydrate de (±)-(E)-3-{6-[(E)-3-(4-benzyl-3-phényl-pipérazin-1-yl)-3-oxo-propényl]-pyridin-2-yl}-N-hydroxy-acrylamide ;
chlorhydrate de (±)-(E)-3-{6-[(E)-3-(4-acétyl-3-phényl-pipérazin-1-yl)-3-oxo-propényl]-pyridin-2-yl}-N-hydroxy-acrylamide ;
chlorhydrate de (±)-(E)-N-hydroxy-3-(6-{(E)-3-[3-(2-méthoxy-phényl)-pipéridin-1-yl]-3-oxo-propényl}-pyridin-2-yl)-acrylamide ;
chlorhydrate de (±)-(E)-N-hydroxy-3-(6-{(E)-3-[3-(3-méthoxy-phényl)-pipéridin-1-yl]-3-oxo-propényl}-pyridin-2-yl)-acrylamide ;
chlorhydrate de (±)-(E)-N-hydroxy-3-(6-{(E)-3-[3-(4-méthoxy-phényl)-pipéridin-1-yl]-3-oxo-propényl}-pyridin-2-yl)-acrylamide ;
chlorhydrate de (±)-(E)-N-hydroxy-3-(6-{(E)-3-oxo-3-[3-(2-trifluorométhyl-phényl)-pipéridin-1-yl]-propényl}-pyridin-2-yl)-acrylamide ;
chlorhydrate de (±)-(E)-N-hydroxy-3-(6-{(E)-3-oxo-3-[3-(3-trifluorométhyl-phényl)-pipéridin-1-yl]-propényl}-pyridin-2-yl)-acrylamide ;
chlorhydrate de (±)-(E)-N-hydroxy-3-(6-{(E)-3-oxo-3-[3-(4-trifluorométhyl-phényl)-pipéridin-1-yl]-propényl}-pyridin-2-yl)-acrylamide ;
chlorhydrate de (E)-N-hydroxy-3-{6-[(E)-3-oxo-3-((S)-3-phényl-pipéridin-1-yl)-propényl]-pyridin-2-yl}-acrylamide ;
chlorhydrate de (E)-N-hydroxy-3-{6-[(E)-3-oxo-3-((R)-3-phényl-pipéridin-1-yl)-propényl]-pyridin-2-yl}-acrylamide ;
chlorhydrate de (E)-N-hydroxy-3-{3-[(E)-3-oxo-3-(4-phényl-3,6-dihydro-2H-pyridin-1-yl)-propényl]-phényl}-acrylamide ;
chlorhydrate de (E)-3-(3-{(E)-3-[4-(4-chloro-phényl)-pipérazin-1-yl]-3-oxo-propényl}-phényl)-N-hydroxy-acrylamide ;
(E)-3-{3-[(E)-3-(4-benzoyl-pipéridin-1-yl)-3-oxo-propényl]-phényl}-N-hydroxy-acrylamide ;
(±)-(E)-N-hydroxy-3-{3-[(E)-3-oxo-3-(3-m-tolyl-pipéridin-1-yl)-propényl]-phényl}-acrylamide ;
(±)-(E)-N-hydroxy-3-{3-[(E)-3-oxo-3-(3-o-tolyl-pipéridin-1-yl)-propényl]-phényl}-acrylamide ;
(±)-(E)-N-hydroxy-3-{3-[(E)-3-oxo-3-(3-p-tolyl-pipéridin-1-yl)-propényl]-phényl}-acrylamide ;
(E)-N-hydroxy-3-{3-[(E)-3-oxo-3-((R)-3-phényl-pipéridin-1-yl)-propényl]-phény1}-acrylamide ;
(E)-N-hydroxy-3-{3-[(E)-3-oxo-3-((S)-3-phényl-pipéridin-1-yl)-propényl]-phényl}-acrylamide ;
(±)-(E)-N-hydroxy-3-{3-[(E)-3-oxo-3-(3-phényl-pipéridin-1-yl)-propényl]-phényl}-acrylamide ;
(±)-(E)-3-(3-{(E)-3-[3-(4-fluoro-phényl)-pipéridin-1-yl]-3-oxo-propényl}-phényl)-N-hydroxy-acrylamide ;
chlorhydrate de (E)-3-(3-{(E)-3-[4-(3-chloro-phényl)-pipérazin-1-yl]-3-oxo-propényl}-phényl)-N-hydroxy-acrylamide ;
(E)-3-{3-[(E)-3-(4-benzyl-pipéridin-1-yl)-3-oxo-propényl]-phényl}-N-hydroxy-acrylamide ;
chlorhydrate de (E)-3-(3-{(E)-3-[4-(2-chloro-phényl)-pipérazin-1-yl]-3-oxo-propényl}-phényl)-N-hydroxy-acrylamide ;
(E)-3-(3-{(E)-3-[4-(4-cyano-phényl)-pipérazin-1-yl]-3-oxo-propényl}-phényl)-N-hydroxy-acrylamide ;
(E)-N-hydroxy-3-{3-[(E)-3-oxo-3-(5-phényl-3,6-dihydro-2H-pyridin-1-yl)-propény]-phényl}-acrylamide ;
(E)-N-hydroxy-3-(3-((E)-3-oxo-3-(3-phénylpipérazin-1-yl)-prop-1-ényl)phényl)acrylamide ;
(E)-N-hydroxy-3-{3-{(E)-3-oxo-3-[4-(4-trifluorométhyl-phényl)-pipérazin-1-yl]-propényl}-phényl}-acrylamide ;
chlorhydrate de (E)-3-(6-{(E)-3-[3-(2-fluoro-phényl)-pipéridin-1-yl]-3-oxo-propényl}-pyridin-2-yl)-N-hydroxy-acrylamide ;
chlorhydrate de (E)-3-(6-{(E)-3-[3-(3-fluoro-phényl)-pipéridin-1-yl]-3-oxo-propényl}-pyridin-2-yl)-N-hydroxy-acrylamide ;
chlorhydrate de (E)-3-[6-{(E)-3-[3-(4-fluoro-phényl)-1,2,4-oxadiazol-5-yl]-pipéridin-1-yl}-3-oxo-propényl)-pyridin-2-yl]-N-hydroxy-acrylamide ;
chlorhydrate de (E)-N-hydroxy-3-{6-[(E)-3-oxo-3-(5-phényl-3,6-dihydro-2H-pyridin-1-yl)-propényl]-pyridin-2-yl}-acrylamide ;
trifluoroacétate de (E)-N-hydroxy-3-{6-[(E)-3-oxo-3-(6-phényl-3,4-dihydro-1H-isoquinoléin-2-yl)-propényl]-pyridin-2-yl}-acrylamide ;
chlorhydrate de (E)-N-hydroxy-3-{6-[(E)-3-oxo-3-(2-phényl-6,7-dihydro-4H-thiazolo[5,4-c]pyridin-5-yl)-propényl]-pyridin-2-yl}-acrylamide ;
trifluoroacétate de (E)-N-hydroxy-3-{6-[(E)-3-oxo-3-(2-phényl-6,7-dihydro-5H-thiazolo[5,4-c]pyridin-4-yl)-propényl]-pyridin-2-yl}-acrylamide.

4. Composé tel que défini dans l'une quelconque des revendications 1 à 3, pour une utilisation en tant qu'inhibiteurs des HDAC.

5. Composé selon la revendication 4, pour une utilisation dans le traitement ou la prévention de maladies liées au dérèglement de l'activité des histones désacétylases.

6. Composition pharmaceutique comprenant un ou plusieurs composés de formule (I) tel que définis selon l'une quelconque des revendications 1 à 3.

7. Composition pharmaceutique selon la revendication 6, pour une utilisation dans le traitement ou la prévention de maladies liées au dérèglement de l'activité des histones désacétylases.

8. Composition pharmaceutique selon la revendication 7, comprenant en outre des principes actifs additionnels utiles pour le traitement desdites maladies.

9. Composition pharmaceutique selon l'une quelconque des revendications 6 à 7, sous la forme de comprimés, gélules, préparations orales, poudres, granules, pilules, solutions liquides injectables ou perfusables, suspensions, émulsions, suppositoires, pommades, crèmes, lotions, gels, pâtes, dispositifs de délivrance transdermique.

10. Procédé de préparation d'un composé selon l'une quelconque des revendications 1 à 3, comprenant
a. la soumission d'un composé de formule A1 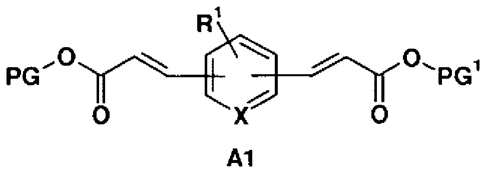 dans laquelle R¹, X sont tels que définis dans la revendication 1, et PG, PG¹ sont des groupes protecteurs, à des réactions de déprotection ;
b. le traitement des sites déprotégés avec des précurseurs des fragments appropriés : 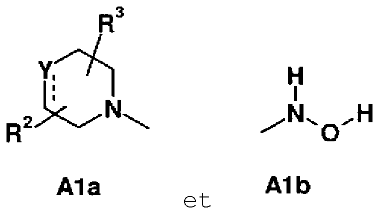 dans lesquels la ligne pointillée, R², R³ et Y sont tels que définis dans la revendication 1.

11. Procédé selon la revendication 10, dans lequel deux réactions de déprotection indépendantes sont réalisées, qui peuvent avoir lieu dans n'importe quel ordre, ledit procédé étant réalisé de la manière suivante :
i) première déprotection (élimination de PG ou PG¹)
ii) réaction avec le premier précurseur (du fragment A1a ou A1b)
iii) seconde déprotection (élimination de PG¹ ou PG)
iv) réaction avec le second précurseur (du fragment A1b ou A1a) .

12. Procédé selon l'une quelconque des revendications 10 à 11, dans lequel PG et PG¹ sont indépendamment un groupe méthyle ou *tert*-butyle.
